(19) Europäisches Patentamt / European Patent Office / Office européen des brevets

(11) EP 3 091 087 A1

(12) EUROPEAN PATENT APPLICATION

(43) Date of publication: 09.11.2016 Bulletin 2016/45

(51) Int Cl.: *C12Q 1/68* (2006.01)

(21) Application number: 15382241.6

(22) Date of filing: 08.05.2015

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
- **Fundació Institut d'Investigació en Ciències de la Salut Germans Trias i Pujol 08916 Badalona, Barcelona (ES)**
- **Universitat Autònoma de Barcelona 08193 Bellaterra (ES)**

(72) Inventors:
- **Beyer, Katrin 08758 Cervelló (ES)**
- **Ariza Fernández, Aurelio 08005 Barcelona (ES)**

(74) Representative: **ZBM Patents - Zea, Barlocci & Markvardsen Plaza Catalunya, 1 08002 Barcelona (ES)**

# (54) METHOD FOR IN VITRO DIAGNOSIS OF SYNUCLEINOPATHIES USING ALPHA-SYNUCLEIN GENE TRANSCRIPTS

(57) The present invention provides a method for the *in vitro* diagnosis of a synucleinopathy in a human patient comprising the step of determining the amount of at least one transcript of the human alpha-synuclein gene (SNCA) selected from the group consisting of SNCAtv3 (SEQ ID NO: 3) and SNCAtv2 (SEQ ID NO: 2) in a biological sample obtained from the patient. The invention further provides means to determine the amount of said transcripts, as well as a method to stablish the response of a patient which has been diagnosed with a synucleinopathy to a medical regime for its treatment by determining the amount of at least one transcript selected from the group consisting of SNCAtv3 and SNCAtv2 in a biological sample from the patient before and after the treatment.

EP 3 091 087 A1

**Description**

BACKGROUND ART

The present invention relates to the field of medicine, particularly to neurodegenerative disorders, specifically to a method of in vitro diagnosis of synucleinopathies using transcripts of the alpha-synuclein gene (SNCA).

Synucleinopathies are diseases characterized by the presence of neuronal proteinaceous inclusions called Lewy bodies (LB). Lewy bodies and Lewy neurites are formed fundamentally by the protein alpha-synuclein. Several synucleinopathies are known, but by far the more relevant disorders included in this group are Parkinson's disease (PD) and Dementia with Lewy bodies (DLB). While PD is most common progressive movement disorder at old age, DLB is the second most frequent cause of dementia after Alzheimer's disease. When it was first described, it was believed that the DLB was a rare disorder; however, in recent years extensive research on this disease has revealed that it is present in 10-15% of autopsied cases. The main symptoms of DLB include fluctuating cognitive impairment, recurrent visual hallucinations and Parkinsonism.

The diagnosis of the various forms of synucleinopathies still relies on an exact medical and family history, an accurate examination of the patient, and on the skills of the examiner. Neuroimaging (DaTscan) has been introduced, but these imaging techniques often require use of radioactive compounds and are not cost-effective, so that their application is limited. Biological diagnosis markers would be of help, but so far none have been found to have clinical significance.

Further, because of clinical overlap, differential diagnosis of PD and DLB is very difficult. Parkinsonism is the predominant symptom of PD, but it can be indistinguishable from the parkinsonism of DLB. DLB could be the same disease as PD but with widespread cortical pathological states, leading to dementia, fluctuating cognition, and the characteristic visual hallucinations. Additionally, many symptoms of Alzheimer's disease (AD) also overlap with DLB, which entails frequent misdiagnosis of DLB. Treatment of PD relies on administration of agents that increase the levels of dopamine (Levodopa). However, patients suffering of DLB benefit from administration of alternative/additional drugs, in particular, acetylcholine esterase inhibitors. It follows that there exists a need to provide cost-effective methods for the differential diagnosis of PD and DLB in order to be able to apply the most effective treatment for each patient.

Patent EP2539461 discloses that specific alterations in Butyrylcholinesterase (BChE) gene are specifically related to DLB. This discovery allows for the differential diagnosis of patients suffering from DLB by determining the genotype of particular alterations in BChE gene in a blood sample. While being a substantial improvement for this field, the disclosed diagnosis may only identify a small proportion of DLB patients. Indeed, recent results have shown that this method identifies only around 10% of DLB patients, so that there is still a substantial proportion of DLB patients that are not diagnosed.

On the other side, several studies have attempted to use the alpha-synuclein protein as a biomarker in human biological fluids for detection of DLB. For example, in WO2011104696 are disclosed monoclonal or polyclonal antibodies that detect protofibrilar or oligomeric soluble forms of alpha-synuclein in biological fluids, e.g. blood, and the use of these antibodies for diagnosing neurodegenerative synucleinopathies. Furthermore, WO201069603 discloses the use of antibodies obtained or isolated from a "pool" of elderly people without PD, which are sequenced and cloned in different vectors. Such antibodies have affinity for monomeric, oligomeric, aggregate forms, fragments and /or posttranslational modified forms of alpha-synuclein and allow detection of DLB.

Finally, WO9950300 discloses methods for differentiation of synucleinopathies from other neurodegenerative diseases by detecting filamentous aggregates of synucleins. In this document, it is mentioned speculatively that the diagnosis could be accomplished by detecting levels of synuclein nucleic acids by performing known techniques, such as polymerase chain reaction (PCR), ligase chain reaction (LCR), isothermal nucleic acid amplification (NASBA) or polymerase chain reaction with reverse transcription (RT-PCR). The above diagnoses are either not reliable or they need of the proliferation of aggregate forms of SNCA (which are found in advanced stages of the synucleinopathies), or both. An early diagnosis is more desirable in terms of management of the disorder. Furthermore, reliable differential diagnosis of DLB patients (as distinguished from PD patients) would allow recommending the most effective treatment regime for these patients.

Therefore, there is still a need to provide means for an early and accurate identification of patients suffering from a synucleinopathy and also for the differential diagnosis of the different synucleinopathies to be used in the common clinical practice.

SUMMARY OF THE INVENTION

The present invention relates to the use of transcripts of the alpha-synuclein gene as biomarkers in human biological fluids for in vitro diagnosis of synucleinopathies.

It is known that the gene encoding the alpha-synuclein protein, called SNCA (SEQ ID NO: 1), presents a complex splicing processing. After conducting extensive studies, the present inventors have surprisingly found that two of said transcripts (but not all) provide information for the *in vitro* diagnosis of synucleinopathies. The present invention is based on using these two transcripts of the SNCA gene, called SNCAtv2 (SEQ ID NO: 2) and SNCAtv3 (SEQ ID NO: 3), as biomarkers in biological fluids for the diagnosis of synucleinopathies. These transcripts SNCAtv2 and SNCAtv3 differ from each other and from the major transcript SNCAtv1 by differential incorporation of one of the four exons located in the 5' untranslated part of the SNCA gene.

Therefore, in a first aspect, the present invention provides a method for the *in vitro* diagnosis of a synucleinopathy in a human patient comprising the step of determining the amount of at least one transcript of the human alpha-synuclein gene (SNCA) selected from the group consisting of SNCAtv2 (SEQ ID NO: 2) and SNCAtv3 (SEQ ID NO: 3) in a biological sample obtained from the patient.

By using this method patients that suffer from a synucleinopathy are identified. Diagnosis is achieved in patients that have recently developed the disease, thus enabling an early diagnosis of the synucleinopathy. This is a big advantage with respect to known methods. Early diagnosis increases the therapeutic margin to reduce or stop the disease progression, thus improving live quality and prognosis for the patient. Further, the present method allows for differential diagnosis of DLB versus Parkinson disease at a very early stage.

In a second aspect, the present invention provides for the use of means for determining the amount of at least one transcript of the human SNCA selected from the group consisting of SNCAtv2 and SNCAtv3 in a biological sample for the diagnosis of a synucleinopathy in the method as defined in the first aspect of the invention.

In a further aspect, the present invention provides for the use of at least one human alpha-synuclein gene transcript selected from SNCAtv2 and SNCAtv3 for the diagnosis of synucleinopathies.

While providing for a reliable and early diagnosis of a synucleinopathy, the present diagnosis method is useful to a clinician, who is able to take appropriate decisions to treat the patient, i.e. to apply a medical regime that is appropriate for treating synucleinopathies. Thus, in another aspect, the invention is directed to a method of deciding or recommending to initiate a medical regime for the treatment of a synucleinopathy in a human patient, which method comprises diagnosing a synucleinopathy or determining whether the patient is suspicious of suffering a synucleinopathy by the method as defined in the first aspect of the invention, wherein (a) if the patient is diagnosed of suffering from a synucleinopathy, or of being suspicious of suffering from a synucleinopathy, then the initiation of the medical regimen is recommended, and (b) if the subject is diagnosed of not suffering from a synucleinopathy, the follow-up is performed optionally in consideration of the result of an examination of the patient by a physician.

The inventors have further found that the amount of SNCAtv2 and SNCAtv3 transcripts may serve to monitor synucleinopathies progression because said amount is inversely correlated with the alpha -synuclein aggregation rate in the brain of the patients (see example 4). As a result, the amount of SNCAtv2 and SNCAtv3 transcripts may also be useful for determining the response to a medical regime that is administered to the patient to treat a synucleinopathy.

Thus another aspect of the invention refers to a method to stablish the response of a human patient which has been diagnosed with a synucleinopathy to a medical regime for the treatment of the synucleinopathy, which method comprises determining the amount of at least one human alpha-synuclein gene transcript selected from the group consisting of SNCAtv3 and SNCAtv2 in a biological sample obtained from the patient being treated and comparing said amount of transcript(s) with the amount of transcript(s) determined for the same patient before the treatment or at an earlier phase of the treatment, wherein a difference in the amount of transcript(s) with respect to before the treatment or earlier phase of the treatment is indicative of a the response to the medical regime.

For a better understanding, the present invention is described below with reference to the accompanying figures, which are presented by way of example, and in no way meant to be limiting of the present invention.

BRIEF DESCRIPTION OF THE FIGURES

Figure 1 shows the change of expression of transcripts SNCAtv1 (SEQ ID NO: 4), SNCAtv2, SNCAtv3, and SNCA112 (SEQ ID NO: 5) in PD and DLB patients regarding healthy controls.

Figure 2 shows the change in expression of transcripts SNCAtv1 (vertical lines), SNCAtv2 (diagonal), SNCAtv3 (squares) and SNCA112 (horizontal lines) at different groups of patients with DLB: who developed the disease before age 65 between 65 to 74 years and after 75 years compared to healthy matched controls age.

Figure 3 shows the change in expression of transcripts SNCAtv1 (vertical lines), SNCAtv2 (horizontal lines), SNCAtv3 (diagonal) and SNCA112 (squares) in different groups of patients diagnosed with DLB: 0 to 1 year, 2 years, 3 to 4 years or over 4 years from diagnosis of the disease, compared to healthy controls.

Figure 4 shows SNCAtv3 expression levels in blood during disease progression, namely, for less than 1 year, for 2 years, for 3-4 years and for more than 4 years since the onset of disease. The Y axis represents SNCAtv3 expression change

DETAILED DESCRPTION OF THE INVENTION

The term "synucleinopathies" as used herein refers to a group of disorders characterised by the abnormal accumulation of aggregates of alpha-synuclein protein in neurons, nerve fibres or glial cells. This group includes Parkinson’s disease and dementia with Lewy bodies. Other rare disorders, such as multiple system atrophy and various neuroaxonal dystrophies also have alpha-synuclein pathologies.

As used herein "alpha-synuclein gene", "SNCA" and "SNCA gene" are indistinctively used to refer to the alpha-synuclein gene, while alpha-synuclein is used to refer to the corresponding protein.

As used herein, the term "transcript" or "transcripts" refers to complete transcripts or specific regions thereof. That refers to complete molecules of ribonucleic acid (RNA) or to specific regions thereof.

As used herein, the terms "transcript SNCAtv1", "transcript SNCAtv2", transcript SNCAtv3", "transcript SNCA112", "SNCAtv1", "SNCAtv2", "SNCAtv3" and"SNCA112" refer to regions or fragments present in the different RNAs derived or produced from the SNCA gene or to complete transcripts or RNA molecules derived or produced from the SNCA gene.

Herein, the terms "biological sample", "biological fluid sample", "biological fluid" and their plurals are used as equivalent and interchangeable for referring to liquids derived from an individual.

As used herein, the terms "DLB" and "Dementia with Lewy bodies" refer to any stage of DLB, regardless of the age of patient or time since diagnosis.

As mentioned above, the present invention provides, in a first aspect, a method for the *in vitro* diagnosis of synucleinopathies in a human patient comprising the step of determining the amount of at least one transcript of the human alpha-synuclein gene (SNCA) selected from the group consisting of SNCAtv2 (SEQ ID NO: 2) and SNCAtv3 (SEQ ID NO: 3) in a biological sample obtained from the patient.

In a particular embodiment of the first aspect of the invention, when the amount of the transcript(s) determined for the patient is reduced with respect to a reference value, this is indicative of a synucleinopathy in the patient.

The term "reference value" referred to in the method of the first aspect is to be understood as a predefined amount of SNCAtv2 or SNCAtv3 transcripts, which are derived from the amounts of said marker in a sample or group of samples. The samples are taken from a subject or group of control subjects that do not suffer from any neurological symptomatology. The skilled person in the art, making use of the general knowledge, is able to choose the subject or group of subjects more adequate for obtaining the reference value.

In one embodiment, the reference value is determined from a population of control subjects that do not show any synucleinopathy. According to the findings of the present inventors, when the amount of SNCAtv2 and SNCAtv3 in the patient being tested was significantly decreased when compared to the reference value obtained from subjects without neurological symptomatology, it would be indicative that the patient suffers from a synucleinopathy. From said data, the clinician is able to take appropriate decisions to treat the patient, i.e. to apply a therapy that is appropriate for synucleinopathies.

In a particular embodiment, the method of the invention comprises determining the amount of at least SNCAtv3 in the sample. In another embodiment, the method of the invention comprises determining both SNCAtv2 and SNCAtv3 transcripts. According to the findings of the present inventors, when the amount of both SNCAtv2 and SNCAtv3 in the subject being tested was significantly decreased compared to the reference value obtained from subjects without neurological symptomatology, it would be indicative that the patient suffers from DLB. Thus Since the method of the invention allows for differential diagnosis of DLB with respect to other synucleinopathies, in particular, with respect to Parkinson disease, by applying the present method the clinician may decide to recommend specific medical regimes that are indicated for treating DLB. In particular, the clinician may recommend administrating acetylcholine esterase inhibitors to the patient.

With the diagnostic method of synucleinopathies of the present invention the disease can be diagnosed at any stage and regardless of patient age. However, it is an advantage of the present invention that an early diagnosis of synucleinopathies is achieved. In particular embodiments, early diagnosis of DLB is achieved by use of the present method when determining the amount of SNCAtv2 and SNCAtv3. In one embodiment the diagnosis is performed in patients that have recently developed the disease, in particular the diagnosis is performed less than 3 years from the onset of the disease, more particularly less than 2 or less than 1 year from onset of disease. In another embodiment, the diagnosis is performed in patients of age below 75, or in patients of age below 65 years.

In a preferred embodiment, the sample of human biological fluid is selected from a group comprising blood, plasma, saliva, urine, cerebrospinal fluid, semen and derivatives thereof. The method of diagnosis of synucleinopathies of the present invention has as additional advantages that it is a non-invasive method, even in its early stages; also, it is a reliable and inexpensive method compared to current techniques.

Determining the amount of SNCAtv2 and/or SNCAtv3 transcripts can be performed by any method known to the skilled person, provided that said method permits the detection and quantification of RNA in a biological sample. Included among the examples of these procedures are PCR, quantitative real-time PCR (QPCR), multiplex PCR, NASBA, LCR, RT-PCR, RNA sequencing, array hybridization or "Northern" transfer, or combinations of these. In a preferred embodiment, the determination of the amount of the SNCA transcripts is performed by quantitative real-time PCR.

In most methods of detection and quantification of RNA mentioned above, before performing this procedure it is necessary to convert the RNA to complementary DNA (cDNA). This conversion is accomplished by known techniques by skilled in the art, such as reverse transcription, among others.

Furthermore, in most procedures that can be used in the method of the present invention, the use of primers is required to detect and/or amplify transcripts of interest. A skilled artisan would get easily and directly the sequence of the primers that can be used from the sequence of transcripts to be analysed. In a preferred embodiment of the present invention, the primer sequences are derived from the sequence of the transcript SNCAtv2 (SEQ ID NO: 2). In another preferred embodiment, the primer sequences are obtained from SNCAtv3 transcript sequence (SEQ ID NO: 3). In a further embodiment the primers used for determining the amount of SNCAtv2 are those with SEQ ID NO: 6 and SEQ ID NO: 8, while the primers used for determining the amount of SNCAtv3 are those with SEQ IDNO: 7 and SEQ ID NO: 8.

The present invention requires comparing the amount of the SNCAtv2 and/or SNCAtv3 transcript in a sample obtained from the patient with a reference value. If the amount of the transcript(s) determined in patient's sample is significantly reduced with respect to the reference value, this is indicative of the presence of a synucleinopathy in the patient. As mentioned above, the reference value is determined from a population of control subjects that do not show neurological symptomatology, in particular subjects that do not suffer from any synucleinopathy. The skilled person may use any available method to establish said comparison. For instance, when qPCR is used for determining the amount of the transcripts, the comparison between the amount of transcripts in the patient's and the amount of transcripts in the control subjects may be established by using the comparative Ct method. The "Ct" or "Ct value" of a qPCT reaction for a given target nucleic acid has the sense generally given in the art, i.e., the cycle threshold value. The Ct value means the number of PCR cycles where the reporter dye signal is sufficiently high to cross an automatically or manually determined threshold value, and it is a relative measure of the concentration of nucleic acid target in the qPCR reaction.

The comparative Ct method is also known as deltadeltaCt ($2^{-\Delta\Delta CT}$) method, where:

$\Delta\Delta Ct = \Delta Ct(\text{patient}) - \Delta Ct(\text{control subjects})$, and
$\Delta Ct = Ct(\text{transcript of interest}) - Ct(\text{transcript of the housekeeping gene})$

The housekeeping gene may be selected according to parameters well known to the skilled person. In particular embodiments, the housekeeping gene is beta-actin and hydroxymethylbilan. In a further particular embodiment, DLB is diagnosed when the reduction of the amount of the transcript(s) determined for the patient with respect to their respective reference values, wherein the reference value is the mean amount of transcript in control subjects, is calculated by the deltadeltaCt method, and said deltadeltaCt method yields a coefficient below 0.5. In one embodiment the tested subject is diagnosed with a synucleinopathy when the comparative Ct method yields a coefficient below 0.5 for SNCAtv3. In a particular embodiment, the patient is diagnosed with DLB when the comparative Ct method yields a coefficient below 0.5 for both SNCAtv2 and SNCAtv3 transcripts.

As mentioned above, a second aspect of the invention provides for use of means for determining the amount of at least one transcript of the human alpha-synuclein gene selected from the group consisting of SNCAtv2 and SNCAtv3 in a biological sample for the diagnosis of synucleinopathies in the method as defined in the first aspect. A particular embodiment refers to use of means for determining the amount of both SNCAtv2 and SNCAtv3 transcripts in a biological sample for the diagnosis of DLB. In certain embodiments said means are primers to determine the amount of the transcripts by qPCR. In particular embodiments, the primers used for determining the amount of SNCAtv2 are those with SEQ ID NO: 6 and SEQ ID NO: 8, while the primers used for determining the amount of SNCAtv3 are those with, SEQ IDNO: 7 and SEQ ID NO: 8. Thus, in a particular embodiment the means comprise at least one pair of primers selected from SEQ ID NO: 6/SEQ ID NO: 8 and SEQ IDNO: 7/SEQ ID NO: 8. In other embodiments the means include both pairs of primers defined above. In further particular embodiments any of the above means are comprised in a kit for the diagnosis of synucleinopathies, particularly for the differential diagnosis of DLB.

As will be evident for the skilled person, the above means, included or not in a kit, may also be employed for determining the amount of SNCAtv2 and/or SNCAtv3 transcripts for determining the progression of the synucleinopathy, deciding or recommending initiating a medical regime for the treatment of the synucleinopathy in the tested subject or stablishing the response of a patient to a medical regime for the treatment of the synucleinopathy. In particular embodiments, the above means are for determining the amount of SNCAtv2 and SNCAtv3 transcripts for determining the progression of the DLB, deciding or recommending initiating a medical regime for the treatment of DLB in the tested subject or stablishing the response of a patient to a medical regime for the treatment of DLB.

As mentioned above, the present invention provides the use of at least one human alpha-synuclein gene transcript selected from SNCAtv2 and SNCAtv3 for the diagnosis of synucleinopathies. In a particular embodiment the invention provides use SNCAtv3 for the diagnosis of synucleinopathies. In another particular embodiment the invention provides use of both SNCAtv2 and SNCAtv3 alpha-synuclein gene transcripts for the differential diagnosis of DLB.

The present invention also contemplates using SNCAtv2 and SNCAtv3 gene transcripts for the differential diagnosis of DLB in combination with at least one other marker known as being indicative of DLB. This may be rephrased as a method for the *in vitro* diagnosis of a DLB in a human patient comprising the step of determining the amount of SNCAtv3 and SNCAtv2 in a biological sample obtained from the patient in combination with determining at least one other marker known as being indicative of DLB. In particular embodiments, the other marker is one of the polymorphic sites in BChE gene disclosed in EP2539461. In another particular embodiment, the other marker is the polymorphic site at position 68974 in BChE gene as defined by NCBI Accession Number NG_009031 (i.e. position 934 in SEQ ID NO: 9, which corresponds to SEQ ID NO: 28 of EP2539461). In other particular embodiments, in addition to the polymorphic site at position 68974 in BChE gene, other variations in BChE gene are detected selected from the group consisting of the polymorphic sites at position 3687, 4206, 4443 and the poly-thymine region at positions 4780 to 4786 in NCBI Accession Number NG_009031 (i.e. positions 3687, 4206, 4443 and 4780-4786 respectively in SEQ ID NO: 10, which corresponds to SEQ ID NO: 1 of EP2539461).

The invention also provides a method of deciding or recommending to initiate a medical regime for the treatment of a synucleinopathy in a patient by determining the amount of at least one of SNCAtv2 and SNCAtv3 transcripts. In a particular embodiment said method comprises the steps of (a) determining the amount of at least one human alpha-synuclein gene transcript selected from SNCAtv2 and SNCAtv3 in a biological sample obtained from the patient; and (b) comparing the level obtained in step (a) with a reference value, wherein if the amount of transcript(s) detected in step (a) is lower than the reference value it is indicative that the subject would benefit from a medical regimen for the treatment of synucleinopathies. In particular embodiments, the amount of SNCAtv3 is determined for deciding on the medical regime for the treatment of synucleinopathies. In further embodiments, the amount of both SNCAtv2 and SNCAtv3 is determined and, when both SNCAtv2 and SNCAtv3 are lower than a reference value, it is indicative that the subject would benefit from a medical regimen for the treatment of DLB.

Lastly, the invention is also directed to the use of at least one of SNCAtv2 or SNCAtv3 transcripts to determine synucleinopathies progression in a patient, and also to a method of stablishing the response of a patient to a medical regime for the treatment of synucleinopathies by determining the amount of at least one of SNCAtv2 or SNCAtv3 transcripts. In a particular embodiment the method to stablish the response of a patient which has been diagnosed with a synucleinopathy to a medical regime for the treatment of the synucleinopathy comprises determining the amount of at least one human alpha-synuclein gene transcript selected from the group consisting of SNCAtv3 and SNCAtv2 in a biological sample obtained from the patient being treated and comparing said amount of transcript(s) with the amount of transcript(s) determined for the same patient before the treatment or at an earlier phase of the treatment, wherein an increase of the amount of transcript(s) with respect to before the treatment or earlier phase of the treatment is indicative of a good response to the medical regime. In particular embodiments, the transcript for determining disease progression or stablishing response to a medical regime is SNCAtv3. In some embodiments, both SNCAtv2 and SNCAtv3 transcripts are determined for determining DLB progression or stablishing response to a medical regime for the treatment of DLB..

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

EXAMPLES

Example 1. Study of the expression of transcripts SNCAtv1, SNCAtv2, SNCAtv3 and SNCA112 in patients diagnosed with PD, DLB and control subjects (control subjects did not present any neurological symptomatology).

Expression of four transcripts of the alpha-synuclein gene was analysed: SNCAtv1, SNCAtv2, SNCAtv3 and SNCA112, in control subjects and patients diagnosed with DLB or PD. For this purpose blood samples of 8 control subjects, 32 DLB patients and 12 PD patients were collected. The clinical diagnosis of the individuals was conducted in the Departments of Neurology of the Hospitals Germans Trias i Pujol and Bellvitge (Spain).

RNA from these blood samples was isolated by methods known in the technique and converted to cDNA (complementary DNA).

For this, 2.5 ml of peripheral blood were extracted from each of the individuals included in the study. This blood was collected in PAXgene Blood RNA tubes (Preanalytix, Hombrechtikon, Switzerland). RNA was extracted using the PAXgene Blood RNA Kit (Preanalytix, Hombrechtikon, Switzerland) following the manufacturer's instructions. Briefly, the tubes were centrifuged at 4500g and the obtained pellet of sedimented blood cells was washed 2 to 3 times. After washing, the blood cells were lysed and the resulting solution was introduced into the columns included in the kit mentioned above and nucleic acids contained in the sample bound to the columns while the remaining contaminants were eluted. Then, the DNA was eliminated by application of DNAses to the columns. Prior to the recovery of RNA several cycles of washing and centrifugation were performed. Finally, RNA was eluted using 80μL of elution buffer included in the above mentioned kit.

RNA concentration of the different samples was measured by applying 1 to 2 μL in a DS-11 spectrophotometer (DeNovix, Willington, DE, United States).

Based on these concentrations of RNA, reverse transcription (conversion of RNA to complementary DNA) was carried out by the use of the kit "Ready-kit to go™ You-Prime First-Strand Beads" (GE Healthcare Life Sciences, Uppsala, Sweden) and instructions provided by the manufacturer. For this, 1 mg of RNA was diluted in a final volume of 32 μL and afterwards denatured by applying a temperature 65°C for 10 minutes. After denaturation, said RNA and 1 μL of random primers were added to the tubes provided by the manufacturer in the kit and these tubes were incubated for 1 hour at 37°C.

The cDNA obtained was analysed by the technique of real time polymerase chain reaction (QPCR) using the primers listed in Table 1 and the marker SYBR Green. Beta-actin and hydroxymethylbilane synthase genes were used as "housekeeping" genes to normalize the expression of transcripts of interest.

Table 1.

| Primer Label | Primer sequence (5' to 3') | Comments |
|---|---|---|
| SNCA-1U (SEQ ID NO:7) | ATCCAGGAACAGCTGTCTTC | Specific primer for transcript SNCAtv3 |
| SNCA-2aU (SEQ ID NO: 9) | TTCAAGCCTTCTGCCTTTCC | Specific primer for transcript SNCAtv1 |
| SNCA-2bU (SEQ ID NO: 6) | AGTCGGAGTTGTGGAGAAGC A | Specific primer for transcript SNCAtv2 |
| SNCA-4L (SEQ ID NO: 8) | ACCACTGCTCCTCCAACAT | Generic primer for the studied SNCA transcripts |
| Primer Label | Primer sequence (5 'to 3') | Comments |
| SNCA-3U (SEQ ID NO: 10) | GTGCATGGTGTGGCAACA | Generic primer for the studied SNCA transcripts |
| SNCA-4/6L (SEQ ID NO:11) | ATACCCTTCCTTGCCCAAC | Specific primer transcript SNCA112 |
| β-actin U1 (SEQ ID NO: 12) | CGAGAAGATGACCCAGATCA | β -actin primer |
| b-actin L1 (SEQ ID NO: 13) | TACATGGCTGGGGTGTTGAA | β -actin primer |
| PBGD_U1 (SEQ ID NO: 14) | ACACACAGCCTACTTTCCAAG | Primer for hydroxymethylbilan synthase |
| PBGD_L1 (SEQ ID NO: 15) | TCAATGTTGCCACCACACTGT | Primer for hydroxymethylbilan synthase |

The reaction was carried out using 0.1 mL tubes (Strips Tubes and Cups, Qiagen, Hilden, Germany) and the QuantiTect SYBR Green PCR kit (Qiagen, Hilden, Germany). The final reaction volume for each sample was 15 μL, including 16 pmol of each primer necessary for the reaction (see in Table 2 below primer combinations used) and 1 μL cDNA.

Table 2.

| Combination of primers | Amplified transcript |
|---|---|
| SNCA-1 U and SNCA-4L | SNCAtv3 |
| SNCA-2AU and SNCA-4L | SNCAtv1 |
| SNCA-2BU and SNCA-4L | SNCAtv2 |
| SNCA-3U and SNCA-4/6L | SNCA112 |
| Combination of primers | Amplified transcript |
| β-actin U1 and β-actin L1 | β-actin |
| PBGD_U1 and PBGD_L1 | hydroxymethylbilan synthase |

Then the tubes were placed in the Rotor-Gene 6000 equipment (Qiagen, Hilden, Germany) for performing the QPCR using the rotor for 72 tubes and fixed the following protocol:

- initial denaturation step at 95°C for 15 minutes;
- 40 consecutive cycles of denaturation-annealing-amplification with the following structure of temperature variation (the fluorescence data were acquired in the last second of each of the cycles):

    95°C for 15 seconds

    20 seconds: 54°C for hydroxymethylbilan synthase, 56°C for SNCA112 and β-actin, and 58°C for SNCAtv1, SNCAtv2 and SNCAtv3. 72°C for 30 seconds

At the end of the protocol, a Ct value was obtained for each analysed transcript and for each sample. Each set of measurements performed on the Rotor-Gene 6000 equipment (Qiagen Hilden, Germany) was carried out in duplicate.

The data obtained were analysed using the methodology deltadeltaCt (ΔΔCT), based on that the PCR reaction takes place with the same efficiency for both transcripts those to be analysed and the "housekeeping" genes. To perform calculations corresponding to the ΔΔCt method the following Formulas 1, 2 and 3 were applied consecutively to the Ct values obtained previously:

Formula 1, applied between Ct values obtained for the different transcripts of interest, ie SNCAtv1, SNCAtv2, SNCAtv3 and SNCA112, and Ct values obtained for the transcripts of the "housekeeping" genes, beta-actin and hydroxymethylbilan synthase genes, within a sample or individual, either the group of patients with DLB or the group of healthy controls, permitting to obtain ΔCt for each transcript of interest in each individual:

$$\Delta Ct = Ct_{\text{transcript of interest}} - Ct_{\text{transcript of the housekeeping gene}}$$

Formula 2, applied between ΔCt values of the same transcript in patients with DLB and control subjects: $\Delta\Delta Ct = \Delta Ct_{patient} - \Delta Ct_{control}$

Formula 3: $2^{-\Delta\Delta Ct}$

After application of the formulas above, the value of variation of expression of each of the transcripts of interest was obtained in connection with the expression thereof in the control subjects.

According to the ΔΔCt methodology, it is considered that a decrease in expression is significant when the value obtained after normalization to the value of expression in control subjects is less than 0.5. Likewise, it is considered that an increase in expression is significant when the value obtained after normalization mentioned above, is greater than 1.5.

In this sense, in Table 3 are shown the average values of expression change of transcripts SNCAtv1, SNCAtv2, SNCAtv3 and SNCA112 regarding to their expression in control subjects with the confidence interval in parentheses (confidence level 95%). According to the aforesaid, the results shown in Table 3 were considered significant:

- In the case of decreased expression compared to control subjects, the maximum value of the confidence interval was less than 0.5; and
- In the case of increased expression relative to control subjects, the minimum value of the confidence interval was greater than 1.5.

Table 3.

| | SNCAtv1 | SNCAtv2 | SNCAtv3 | SNCA112 |
|---|---|---|---|---|
| PD patients (expression variation of the transcript) | 0.85 (0.83-0.88) | 0.54 (0.53-0.55) | 0.45 (0.43-0.47) | 0.75 (0.68-0.82) |
| DLB patients (expression variation of the transcript) | 0.57 (0.54-0.60) | 0.37 (0.31-0.45) | 0.33 (0.32-0.34) | 0.81 (0.56-1.18) |

The results of Table 3 are represented schematically in Figure 1.

Both Table 3 and Figure 1 show that transcript SNCAtv3 showed a significant reduction of expression in patients with synucleinopathies (PD and DLB) with respect to control subjects. Additionally, it is shown that both SNCAtv2 and SNCAtv3 showed a significant reduction of expression in patients with DLB. However, it was noted that neither transcript SNCAtv1 and nor SNCA112 showed significant differences in expression between patients with PD or DLB and control subjects.

The sensitivity and specificity of SNCAtv2 and SNCAtv3 transcripts for the diagnosis of DLB were above 90%.

Example 2. Influence of age of onset of the disease in patients with DLB on expression of transcripts SNCAtv1, SNCAtv2, SNCAtv3 and SNCA112.

Data of variation of expression of transcripts SNCAtv1, SNCAtv2, SNCAtv3 and SNCA112 in patients with DLB normalized versus control subjects obtained in Example 1 were divided into three groups depending on the age of DLB beginning of the patients: before an age of 65 years (4 patients), between 65 and 74 years (16 patients) and after 75 years (12 patients).

The results obtained are summarized in the following Table 4 and shown in Figure 2.

Table 4.

| | SNCAtv1 | SNCAtv2 | SNCAtv3 | SNCA112 |
|---|---|---|---|---|
| DLB patients who debuted with disease before age of 65 years | 0.54 (0.32-0.93) | 0.21 (0.15-0.29) | 0.13 (0.12-0.14) | 0.69 (0.50-0.98) |
| DLB patients who debuted with disease between 65 and 74 years | 0.55 (0.52-0.58) | 0.46 (0.38-0.54) | 0.36 (0.33-0.39) | 0.76 (0.52-1.09) |
| DLB patients who debuted with disease after 75 years | 0.57 (0.47- 0.68) | 0.35 (0.26-0.48) | 0.4 (0.35-0.47) | 0.96 (0.64-1.43) |

As in the case of Example 1, the data shown in Table 4 were significant:

- In the case of decreased expression compared to control subjects, the maximum value of the confidence interval was less than 0.5; and
- In the case of increased expression relative to control subjects, the minimum value of the confidence interval was greater than 1.5.

Both Table 4 and Figure 2 show that transcripts SNCAtv2 and SNCAtv3 showed, in virtually all cases, a significant reduction of expression in DLB patients compared to control subjects. The only exception was observed in the group of patients with DLB debut aged between 65 and 74 years. In this group, the transcript SNCAtv2 showed no significant difference in its expression relative to control subjects, but a clear trend to reduced expression compared with control subjects. Therefore, expression of the transcripts SNCAtv2 and SNCAtv3 is reduced in patients with DLB independently on age of onset of the disease. Instead, it was observed that transcripts SNCAtv1 and SNCA112 showed no significant differences in expression between patients with DLB and control subjects in either group.

Both the sensitivity and specificity of SNCAtv2 and SNCAtv3 transcripts for the diagnosis of DLB were above 90% in all groups studied.

Example 3. Influence of time since diagnosis of DLB on the expression of SNCAtv1, SNCAtv2, SNCAtv3 and SNCA112 transcripts

Data of variation of expression of transcripts SNCAtv1, SNCAtv2, SNCAtv3 and SNCA112 in patients with DLB normalized versus control subjects obtained in Example 1 were divided into four groups according to time since diagnosis: 0 to 1 year (6 patients), 2 years (9 patients), among 3 and 4 years (7 patients) and more than 4 years (6 patients).
The results obtained are summarized in Table 5 and represented in the Figure 3.

Table 5.

| Time of diagnosis (years) | SNCAtv1 | SNCAtv2 | SNCAtv3 | SNCA112 |
|---|---|---|---|---|
| between 0 and 1 year | 0.68 (0.64-0.72) | 0.3 (0.23-0.40) | 0.24 (0.21-0.28) | 0.63 (0.35-1.13) |
| 2 years | 0.43 (0.34-0.54) | 0.4 (0.36-0.45) | 0.27 (0.23-0.32) | 0.69 (0.48-1.05) |
| between 3 and 4 years | 0.52 (0.41-0.66) | 0.33 (0.23-0.46) | 0.37 (0.35-0.40) | 0.75 (0.73-0.79) |
| more than 4 years | 0.41 (0.29-0.57) | 0.33 (0.32-0.34) | 0.59 (0.50-0.68) | 0.69 (0.41-1.16) |

As in the case of Example 1, the data shown in Table 5 were significant:

- In the case of decreased expression compared to control subjects, the maximum value of the confidence interval was less than 0.5; and
- In the case of increased expression relative to control subjects, the minimum value of the confidence interval was greater than 1.5.

Both Table 5 and Figure 3 show that the transcripts SNCAtv2 and SNCAtv3 showed, in virtually all cases, a significant reduction in their expression in different groups of patients with DLB compared to control subjects. The only exception was observed in the group of patients with DLB diagnosed more than four years ago. In this group the SNCAtv3 transcript showed no significant variation in expression respect to control subjects, but a clear trend of reduction of expression relative to said control subjects. Therefore, the expression of transcripts SNCAtv2 and SNCAtv3 is reduced in patients with DLB regardless of the time elapsed since diagnosis.
In contrast, it was observed that transcripts SNCAtv1 and SNCA112 did not show significant differences significant in their expression between patients with DLB and control subjects.
The sensitivity and specificity of SNCAtv2 and SNCAtv3 transcripts for the diagnosis of DLB were above 90% in all groups studied.

Example 4. Influence of disease progression on the expression of SNCAtv1, SNCAtv2, SNCAtv3 and SNCA112 transcripts

Data of variation of expression of transcripts SNCAtv1, SNCAtv2, SNCAtv3 and SNCA112 in patients with DLB normalized versus control subjects obtained in Example 1 were divided into four groups according to time of disease progression: 0 to 1 year (6 patients), 2 years (9 patients), among 3 and 4 years (7 patients) and more than 4 years (6 patients).
The results obtained are summarized in Table 6 and represented in the Figure 4.

Table 6. Expression of transcripts SNCAtv1, SNCAtv2, SNCAtv3 and SNCA112 in patients with DLB normalized versus control subjects during disease progression

| Disease progression (years) | SNCAtv1 | SNCAtv2 | SNCAtv3 | SNCA112 |
|---|---|---|---|---|
| between 0 and 1 year | 0.68 (0.64-0.72) | 0.3 (0.23-0.40) | 0.24 (0.21-0.28) | 0.63 (0.35-1.13) |
| 2 years | 0.43 (0.34-0.54) | 0.4 (0.36-0.45) | 0.27 (0.23-0.32) | 0.69 (0.48-1.05) |
| between 3 and 4 years | 0.52 (0.41-0.66) | 0.33 (0.23-0.46) | 0.37 (0.35-0.40) | 0.75 (0.73-0.79) |
| more than 4 years | 0.41 (0.29-0.57) | 0.33 (0.32-0.34) | 0.59 (0.50-0.68) | 0.69 (0.41-1.16) |

As in the case of Example 1, the data shown in Table 5 were significant:

- In the case of decreased expression compared to control subjects, the maximum value of the confidence interval was less than 0.5; and
- In the case of increased expression relative to control subjects, the minimum value of the confidence interval was greater than 1.5.

Both Table 6 and Figure 4 show that the transcripts SNCAtv2 and SNCAtv3 showed, in virtually all cases, a significant reduction in their expression in different groups of patients with DLB compared to control subjects. Additionally, when correlating with the progression of DLB, it can be observed that the levels of SNCA transcript tv3 correlate inversely with the advance of the disease. Figure 4 also shows the results: blood obtained from patients who debuted recently with DLB contains lowest SNCAtv3 levels, which increased with the progression of DLB. SNCAtv3 levels remained significantly diminished but were increasing year by year of disease duration until disease duration of 4 years. Patients who were suffering from DLB for more than 4 years did no longer present significantly diminished SNCAtv3 levels in blood. The tendency is furthermore represented by the trend line overlaid in the graph (Figure 4).

It is known that alpha-synuclein expresses specifically in neurons and that its aggregation is a key event in the development of neuropathological changes in synucleinopathies. It is also known that the development of neuropathological changes begins much earlier than symptoms become evident, namely in preclinical stages of the disease and that disease progression is associated with neuron loss. It is further known that at advanced stages of disease only a few neurons remain intact.

The above observations strongly indicate that: 1- alpha-synuclein aggregation rate is increased at preclinical stages of synucleinopathies, 2- alpha-synuclein aggregation rate is also increased during the first stages of disease, and 3-alpha-synuclein aggregation rate diminishes when disease is advancing due to increasing neuron loss.

Taken together, the aforementioned observations and the results on SNCAtv3 expression levels in blood of DLB patients suggest that: 1- SNCAtv3 levels in blood inversely correlate with the alpha-synuclein aggregation rate in brain, 2-determination of SNCAtv3 levels in blood may serve to detect pre-clinical alpha-synuclein aggregation events in the brain and 3- determination of SNCAtv3 levels in blood may serve to monitor alpha-synuclein aggregation rates in the brain, for example after the treatment with alpha-synuclein anti-aggregants in a clinical trial.

REFERENCES CITED IN THE APPLICATION

EP2539461
WO2011104696
WO201069603
WO9950300

**Claims**

1. A method for the *in vitro* diagnosis of a synucleinopathy in a human patient comprising the step of determining the amount of at least one transcript of the human alpha-synuclein gene (SNCA) selected from the group consisting of SNCAtv3 (SEQ ID NO: 3) and SNCAtv2 (SEQ ID NO: 2) in a biological sample obtained from the patient.

2. The method according to claim 1, wherein when the amount of the transcript(s) determined for the patient is reduced with respect to a reference value, this is indicative of the presence of a synucleinopathy in the patient.

3. The method according to any of the claims 1-2, wherein the amount of SNCAtv3 transcript is determined.

4. The method according to any of the claims 1-3, wherein both SNCAtv2 and SNCAtv3 transcripts are determined.

5. The method according to claim 4, wherein the synucleinopathy is dementia with Lewy bodies.

6. The method according to any of the claims 1-5, wherein the step of determining the amount of SNCA transcript is performed by quantitative real-time PCR using primers with SEQ ID NO: 6 and SEQ ID NO: 8 for determining SNCAtv2 and primers with SEQ ID NO: 7 and SEQ ID NO: 8 for determining SNCAtv3.

7. The method according to any of the claims 5-6 that additionally comprises determining at least one other marker known as being indicative of dementia with Lewy bodies.

8. Use of means for determining the amount of at least one transcript of the human alpha-synuclein gene selected from the group consisting of SNCAtv2 and SNCAtv3 in a biological sample for the diagnosis of a synucleinopathy in the method as defined in any of the claims 1-6.

9. The use according to claim 8, wherein the means include at least one pair of primers selected from SEQ ID NO: 6/ SEQ ID NO: 8 and SEQ ID NO: 7/SEQ ID NO: 8.

10. The use of any of the claims 8-9, wherein the means form part of a kit.

11. Use of at least one human alpha-synuclein gene transcript selected from SNCAtv2 and SNCAtv3 for the diagnosis of a synucleinopathy.

12. Use of SNCAtv2 and SNCAtv3 alpha-synuclein gene transcripts for the differential diagnosis of dementia with Lewy bodies.

13. Use according to claim 12 in combination with at least one other marker known as being indicative of dementia with Lewy bodies.

14. A method to stablish the response of a patient which has been diagnosed with a synucleinopathy to a medical regime for the treatment of a synucleinopathy, which method comprises determining the amount of at least one human alpha-synuclein gene transcript selected from the group consisting of SNCAtv3 and SNCAtv2 in a biological sample obtained from the patient being treated and comparing said amount of transcript(s) with the amount of transcript(s) determined for the same patient before the treatment or at an earlier phase of the treatment, wherein an increase of the amount of transcript(s) with respect to before the treatment or earlier phase of the treatment is indicative of a good response to the medical regime.

15. The method according to claim 14, wherein the amount of SNCAtv3 transcript is determined.

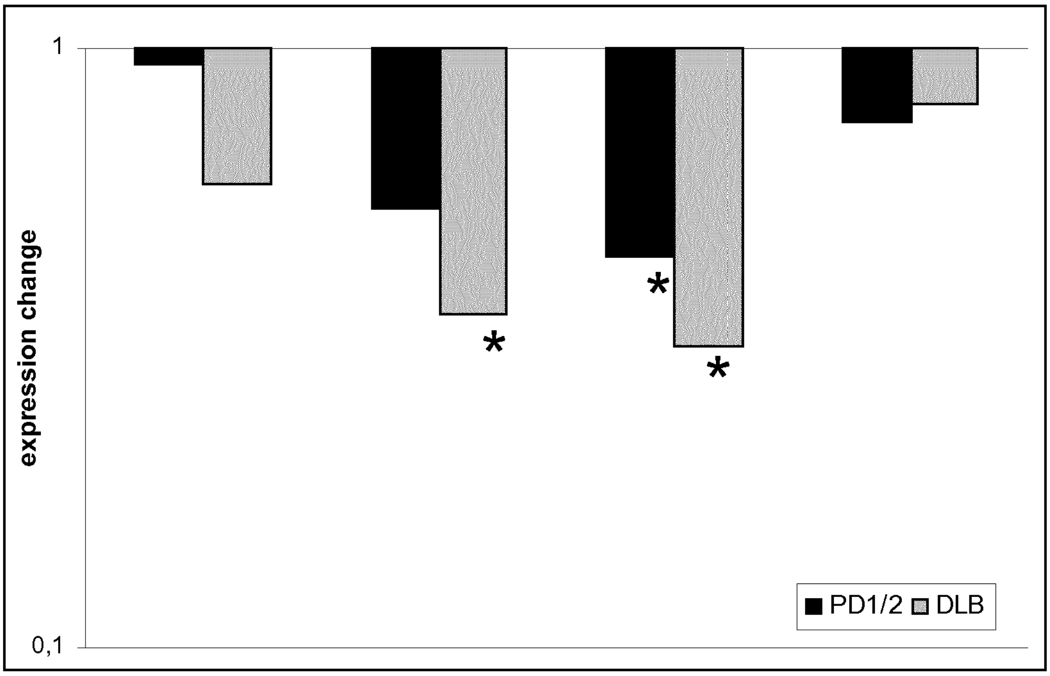


FIG. 1

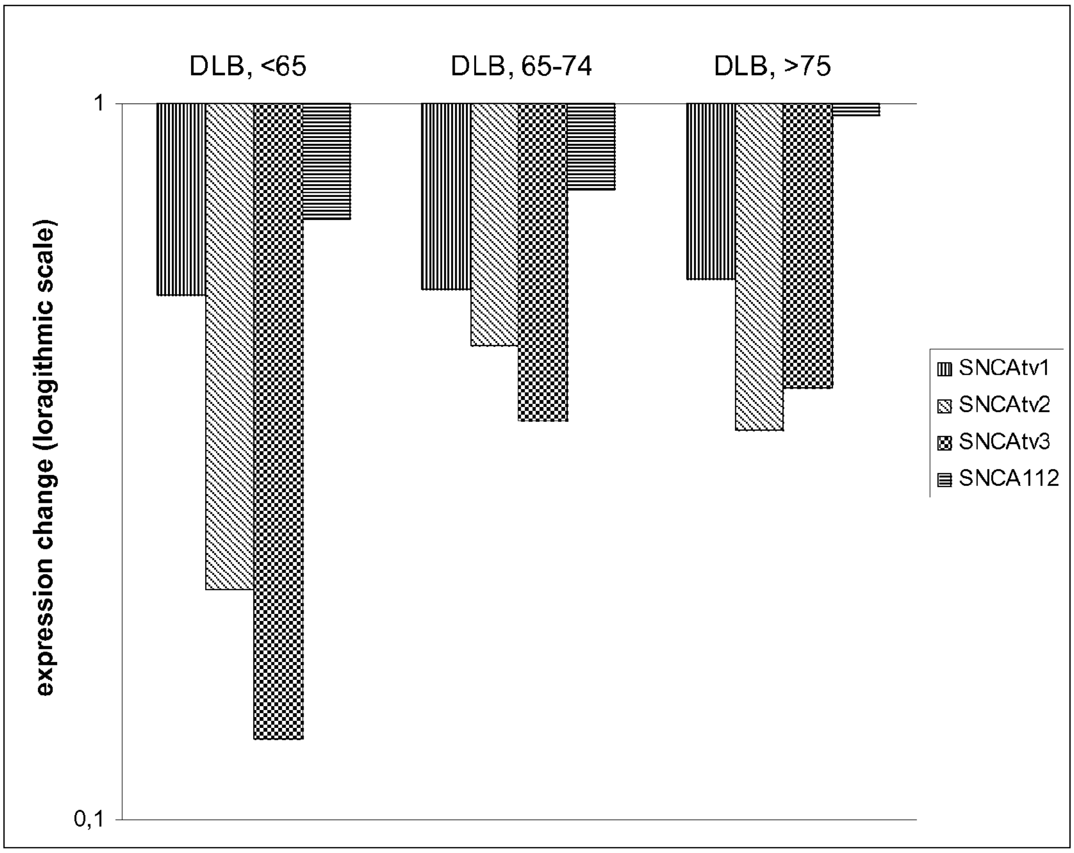
DLB, <65
DLB, 65-74
DLB, >75
1
0,1
expression change (loragithmic scale)
SNCAtv1
SNCAtv2
SNCAtv3
SNCA112

FIG. 2

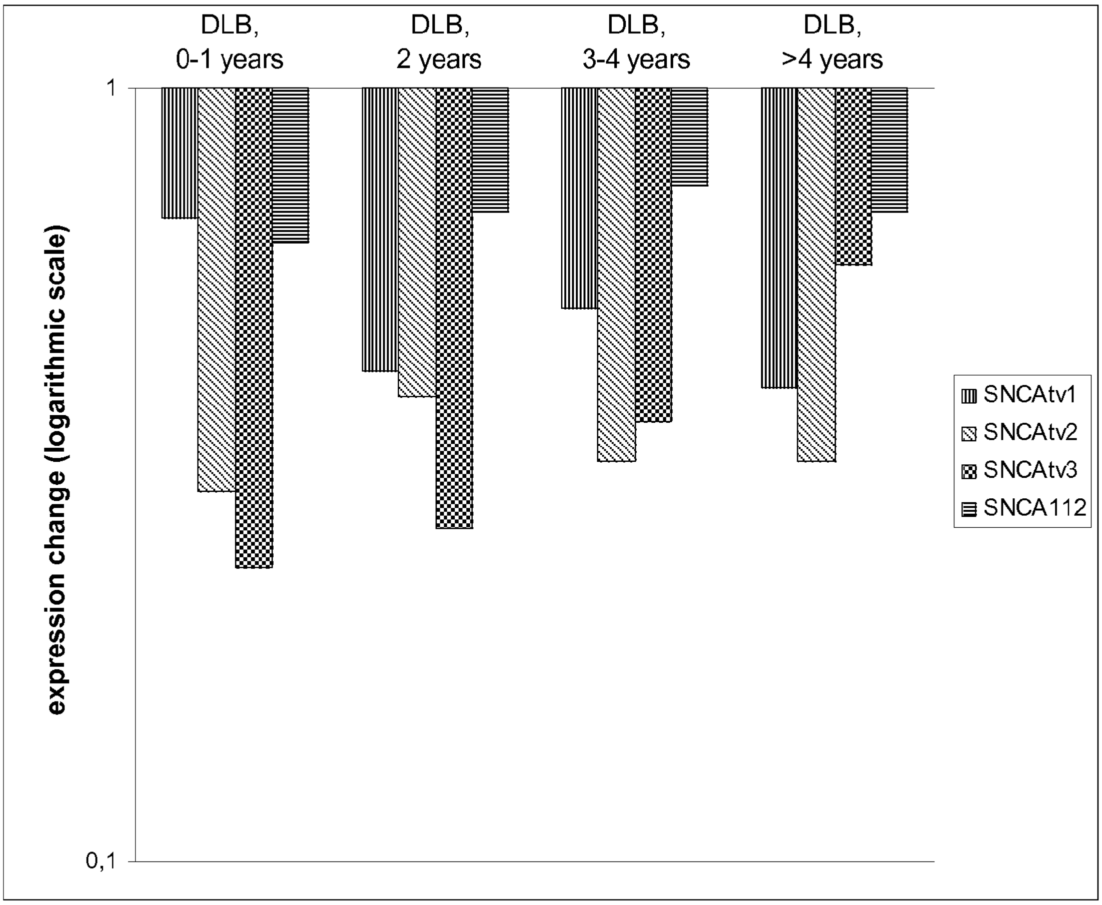
DLB,
0-1 years
DLB,
2 years
DLB,
3-4 years
DLB,
>4 years
1
0,1
expression change (logarithmic scale)
SNCAtv1
SNCAtv2
SNCAtv3
SNCA112

FIG. 3

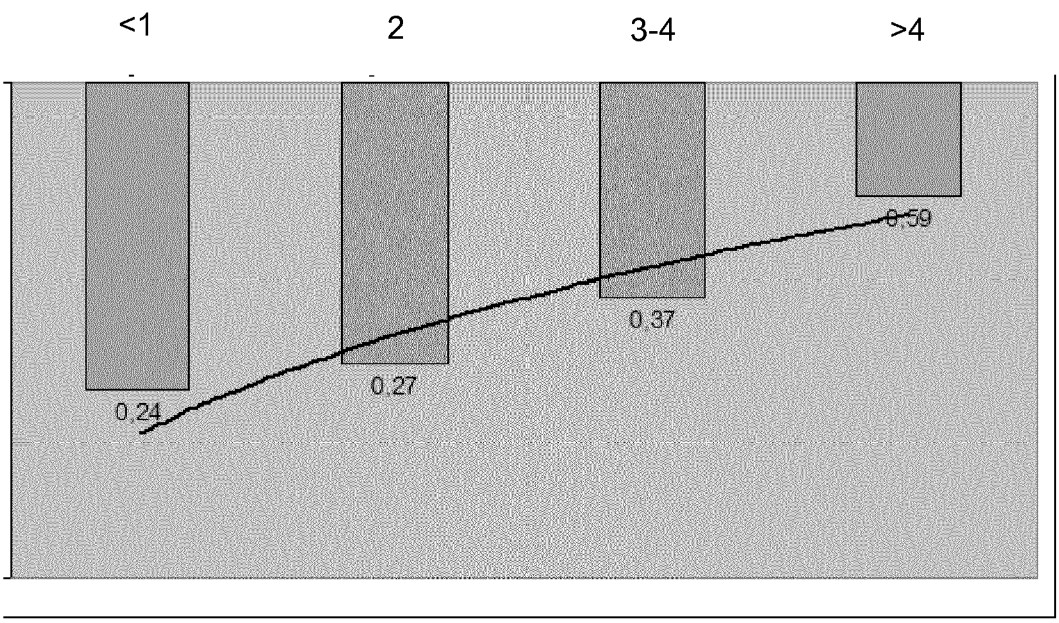


FIG. 4

Europäisches Patentamt
European Patent Office
Office européen des brevets

# EUROPEAN SEARCH REPORT

Application Number
EP 15 38 2241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br><br><br>A | WO 2012/032519 A2 (YISSUM RES DEV CO [IL]; HADASIT MED RES SERVICE [IL]; SOREQ HERMONA [I) 15 March 2012 (2012-03-15)<br>* abstract; claims 9,10 *<br>----- | 1-4, 6-11,14<br><br><br>5,12,13 | INV.<br>C12Q1/68 |
| Y<br><br><br><br><br><br><br><br><br>A | KATRIN BEYER ET AL: "Identification and characterization of a new alpha-synuclein isoform and its role in Lewy body diseases",<br>NEUROGENETICS, SPRINGER, BERLIN, DE,<br>vol. 9, no. 1,<br>23 October 2007 (2007-10-23), pages 15-23, XP019566114,<br>ISSN: 1364-6753<br>* abstract *<br>* page 514, right-hand column - page 516, left-hand column; figure 4 *<br>----- | 1-4, 6-11,14<br><br><br><br><br><br><br><br><br>5,12,13 | |
| Y<br><br><br><br><br><br><br><br><br><br><br>A | KATRIN BEYER ET AL: "Alpha-Synuclein Posttranslational Modification and Alternative Splicing as a Trigger for Neurodegeneration",<br>MOLECULAR NEUROBIOLOGY,<br>vol. 47, no. 2,<br>25 August 2012 (2012-08-25), pages 509-524, XP055225766,<br>US<br>ISSN: 0893-7648, DOI: 10.1007/s12035-012-8330-5<br>* the whole document *<br>-----<br>-/-- | 1-4, 6-11,14<br><br><br><br><br><br><br><br><br><br><br>5,12,13 | TECHNICAL FIELDS SEARCHED (IPC)<br>C12Q |

The present search report has been drawn up for all claims

1

| Place of search | Date of completion of the search | Examiner |
|---|---|---|
| Munich | 6 November 2015 | Costa Roldán, Nuria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 38 2241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br><br>A | C. LINNERTZ ET AL: "The genetic contributions of SNCA and LRRK2 genes to Lewy Body pathology in Alzheimer's disease", HUMAN MOLECULAR GENETICS, vol. 23, no. 18, 15 September 2014 (2014-09-15), pages 4814-4821, XP055225783, gb ISSN: 0964-6906, DOI: 10.1093/hmg/ddu196<br>* abstract *<br>----- | 1-4, 6-11,14<br><br>5,12,13 | |
| Y<br><br>A | JESSE R MCLEAN ET AL: "Transcript expression levels of full-length alpha-synuclein and its three alternatively spliced variants in Parkinson's disease brain regions and in a transgenic mouse model of alpha-synuclein overexpression", MOLECULAR AND CELLULAR NEUROSCIENCES, SAN DIEGO, US, vol. 49, no. 2, 26 November 2011 (2011-11-26), pages 230-239, XP028454655, ISSN: 1044-7431, DOI: 10.1016/J.MCN.2011.11.006 [retrieved on 2011-12-06]<br>* abstract; figures 4-5; table 1 *<br>----- | 1-4, 6-11,14<br><br>5,12,13 | TECHNICAL FIELDS SEARCHED (IPC) |
| A | WO 2006/039253 A2 (CHILDRENS MEMORIAL HOSPITAL [US]; BOHN MARTHA C [US]; SAPRU MOHAN [US]) 13 April 2006 (2006-04-13)<br>* abstract *<br>* paragraph [0124] - paragraph [0128]; claims 1-24 *<br>----- | 1-15 | |

1

The present search report has been drawn up for all claims

| Place of search | Date of completion of the search | Examiner |
|---|---|---|
| Munich | 6 November 2015 | Costa Roldán, Nuria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 15 38 2241

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2015

| Patent document cited in search report | Publication date | Patent family member(s) | Publication date |
|---|---|---|---|
| WO 2012032519 A2 | 15-03-2012 | NONE | |
| WO 2006039253 A2 | 13-04-2006 | AT 439440 T | 15-08-2009 |
| | | CA 2580189 A1 | 13-04-2006 |
| | | EP 1799826 A2 | 27-06-2007 |
| | | US 2007172462 A1 | 26-07-2007 |
| | | US 2010286242 A1 | 11-11-2010 |
| | | WO 2006039253 A2 | 13-04-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2539461 A
- WO 2011104696 A
- WO 201069603 A
- WO 9950300 A

SEQUENCE LISTING

<110> FUNDACIÓ INSTITUT D'INVESTIGACIÓ EN CIÈNCIES DE LA SALUT GERMANS TRIAS I PUJOL
UNIVERSITAT AUTÓNOMA DE BARCELONA

<120> Method for in vitro diagnosis of synucleinopathies using alpha-synuclein gene transcripts

<130> P3309EP00

<160> 10

<170> PatentIn version 3.5

<210> 1
<211> 121198
<212> DNA
<213> Homo sapiens

<400> 1
gcacatacaa ctttaacttc aatattttaa tgacgaaatt taaggataat ttaaatagaa 60

atggactcag aaaagaatca gtaagactta gtgaaggatc attgtctatt atagagaagt 120

tgatttaaga ttaacttatt agtaatattt aacatatata aagaattatt agactgggta 180

tatagacaag cgttttattc ttggaagaca aaaagaagaa aaattgaatt caaccgatgt 240

atacgaaaat aaaaagtaac agtaaattaa aaatagataa ttaaataaat atatgataca 300

gtataacgtt ttatagccaa gatgatgtta caaatccata tttattgaca tggatatgtt 360

tttatactaa agtgtttatc aaatagccat taagagataa cttctttgaa taatttgctt 420

tctaaatttc ttaactacat aaatttccag ctttatatgg aacaccaagt tttcaaacca 480

ttagtgatgt gctttttata tggtgttaaa aagtttcttt ctttcttttt tctttttccc 540

ccaagatgga gtcttgctct gtcgcccagg ctggagcgca gtagtgcgat ctcggctcag 600

tgcaacaacc acctcctggg tacaagcaat tctcctgcct cagcccccca agtagctggg 660

attacaggca cctgccacca cgtccagctg atttttgtat ttttagtaga gacggggttt 720

taccatcttg gccaggctgg tctctaactc ctgacctcag gtaatctgcc cacctcagcc 780

tcccaaagtg ctgagattac aggcgtgagc caccatgccc gacctaaaaa gtttcttaaa 840

cgtcacttta tactctcaaa ttatctagaa aggaaaacgt attagattcc tggatatttt 900

ggatattgta aggaacatac ttatttgctg tatatactct gtttgtaaca gtattgtaac 960

ttcagttcaa aacaatacac aaaacattac aagttcccgt gatattttaa aaattcattt 1020

attttcttcc tttctgaata caaatgctgt tcagtctgtt gattcttcac taatctgaaa 1080

tattagggac tgatttctga attggatatt cattctgaag cctttcagag ccactggcac 1140

aaagggtctg tcaaacttgg aacaccattt gttgtatcat tttatttttt tctcttggca 1200

aatccacata attcatacag gactatgcca gtgtcttttg aaagaaacaa ggtttaagaa 1260

agtaaaaatg ttaataaaga tagtgaatgt taattctgtc attgttactg tatttcttca 1320
agctgtggct gcaaactgct ttgagtgatg ttattgtaac tcgcacatta gggagagaaa 1380
gagatgtttg gtagattttt aattaatgat ccctatcaat gctccttgag ctttcccact 1440
ctatctctcc acaacttcca tccctggttg gaaatttttt gcttacccat actaagtgag 1500
agttattgat gggaaggcat cagatatctc acgtgtgttg ctggtgggat gggagactgt 1560
ggaggatggg aacaggtgga aatctactgc aatggaaaaa aaaaaaaagc atgtcctagg 1620
acacccaaaa catggaggct agataataac aatagctact tgtactgaga gcttccactc 1680
tgcctggctc tttgctatga gccacattat tcattcctta caacaatcaa acaagacaag 1740
taaaatatca tgcccatttt ttaatgagaa aactagagat tagagaggtt atagatactt 1800
gctctgagtc actagtaatg agtagtagag ctttaataag tccctgaatt taggttgtat 1860
ctagtacatt tactcttaga agtctatcat gctcaccaga gttgcagagt tgcgtgtatt 1920
tcttgggctc attaatgtgt ttttttcttt ctaaaactaa agtcatttga acttgttaga 1980
ttttgaaata tttaaatatc ttttctatct ggctttaaca tctttaatct tggaatcttg 2040
catgccttca tattcttagg accacgaaac cacaggaata tttaaaatga tatctagtgg 2100
aaacaatatg aagttggcca tggggtcaaa ttagagaatc tgaatactat gcttctcctt 2160
gattgctctt cccatttctt cagagtaacc ctattccccc atctcatgct caccccccttt 2220
ccaaaatcat acataatgat ctcccaacag tatgcattag gctttctcta ctctacccac 2280
tatgaaatta cacaagaagc ctatcgcaat ctcactacct cgtctctctc acaggtttac 2340
agaaggtgag aggaaggtgc agatagagaa taagaagcag gtggctccag catcaacatt 2400
acatcacccc ttgtgttcac aacaaatacg gaatattatc caaagataat aaacgttgta 2460
ttttcttaac ttaaacacat taaatcagtc ctctctttaa tcaattgtta atgggcagca 2520
tctttatttt catgccattc tactctgctg tctttgctat agcacaagtt taccacatac 2580
catacctaaa aattcagttg ttctatgggg gtaaacaaag tctaggttaa gcatatattt 2640
catagaatgt taatctatag caaaattaat gaattaaatc cagataaaag aatcctatta 2700
tggtctggta aaatatttat atttcactta gcaaagagaa aacaaaacat gaatattgta 2760
gttatgaaca gaatatgcat gttagtaatg cttccaaata tgttattact tcataacttc 2820
atatttctta tgaggtacaa gccattcaat tagtttaacg ttatattcag agaggctaaa 2880
gatttactga agaccatgct gtccatcaat aatgaaaaga aaaattaaaa aaactttatt 2940
ttaacttcta gttcccttct ttgtacttga gcagctttcc ctccttaaga atacagacct 3000
agaacatatg caatatcact atcaatatta tgtgtaatta aaagttcatt ggatgtttac 3060
tgtgttcaag gcattttaag gagtgacaag agttaaacat atagttgtaa ttcaaaatga 3120
caacgaaatt agtttacagt tttctttttt tgtaggtagt aagaaatcat ctccccctat 3180

tgaggaatac caatatagaa aaggcaaaac tttaaatatg aatgaactgt ttcataataa 3240
cataagttct tcttgatttc cattgtcaca tccaaatttg aaggctattt ctaacacagc 3300
tgggttctac ctttttcctt ctcactcttt accacaccca atctgtgagg cttcagacac 3360
aaactgctaa ttcaggagac aattgtgcct tctgtaacag tttctgctaa attgtctcag 3420
ctctgccact taaaatagct aggtgatctc agcatatcac caaaactctt ggagctcagt 3480
ttctctgtct ataaaagtta cataaaatgt aattgatctg cttgttatga ctaaataaca 3540
tagtacatta gtcctttgcc aaaggactaa caaattacca aataaaagtt tggaatcatg 3600
ttaaacgttt ataagaagtg caactgtcca gaaataattc tctcacattg gtctgttgta 3660
atgagaccta aaatatctca ttttatttac ctctttgact taaagcacta ggtctcaagg 3720
aggtcatggt tatactataa atatgtcatg tgaaataata tattaaataa ttgttgtaat 3780
actctattga gatactagtt gtaaagaggc acaatggaaa acttatacta ttaacagtag 3840
taaaaagaaa caacaaaaag caataaaaaa caaaacaccc attcatgcaa cgacatgaac 3900
gaacctcaca aatattatac tgagtaaaag aagtcagaca aatataaaac aaagtttata 3960
ctacgtgatt agatctttat gacattctag aatatgcaca tgaaggtaca aggtaactgt 4020
ctggaatgat gaaaatgtcc tgtgtcttca aaatagtgtg ggttacacta atgcatggct 4080
ttttcaaaac tgatttaaag ggacacaaca tctgagcatt tccctaggtg taaattacac 4140
tgcaatttta aagaatcatc taatgatatt gtggttattt ttaaacagtc cttaaatttt 4200
gtggatgcat actgaatgtt tacagctgaa aagatatata taaagcttga atttggtaaa 4260
aaaaaaaaaa aaagagggag gattggtagt gataaagtga gtggacttat ggatgagaca 4320
tgatcagcca tgcattgaaa aaatgtaaaa gttggatgat cttcacatga gagtccttta 4380
ttctgtctac ttttgcatat gtttgaatat ttcccataac aaaaagttga aaatagagtg 4440
atcacatgag ttaatctcct aatttacaaa aaagaaaact ggaaacagaa ggagaacaaa 4500
acttgttcaa ggtctcaaag ccagacagca aactagctcc caagtccaac cttcttgctc 4560
tggtcctaag caaacaaaaa atattaatat gagctactgc attaaggaaa gtctgctttt 4620
ccaaagggca gaccaatagt tcaaggaaga gtttaaataa taaatatttg tgatcttact 4680
ttcatgcttt tctattttcc actgaacaca tatgcattat cttctatatg tcttttatgt 4740
ataatcattt gcttcctgtt ccttgtggtt ttaaagttgt tttgtatgtt taaatttgat 4800
tttactcaaa tttcagaacc caaattagcg caagaatcag acaaagcata actttctata 4860
aatataaaaa caattaaaaa aaaaacatac agcaaaaacg agttgttgtt tccccccctcc 4920
tcttccagtg cttaactaat cttccgaatc caggcacaga aagcaaaggc tttctgctag 4980
tgggaggagc ttgcttctcc attctggtgt gatccaggaa cagctgtctt ccagctctga 5040

aagaggtgaa aatgtgttaa gcgatgcaaa aattgtcttg aagttcgcgt gtgtatgtct 5100
gtgtgcatgt gcgtgtggtg ggtgggggga gagaaaaggg ggtgtcaatt ctgagggcaa 5160
cgagaatcag aagtcagaaa ggtgagtggt gtgtagcatc tccctttcag aaggggctga 5220
agaagaaatt ggatatgatg gtccggtagg ctaaatcacg ctggatttgt ctcccagata 5280
aagggaggtc tgcaaagtaa gtcccatttc tagagcgaaa agccttagga ccgcttgttt 5340
tagacggctg gggaatattt attccttgtt ccactgatgg gaaaatcagc gtctggcagg 5400
cgctgattgg tggaaaggaa aatggtgata gtggcgtgga aagaggattt gctgagcctt 5460
ctcctgcctc ctcaacctgt gactcttcct tagtagtctc cctttcaccc tcaggaccct 5520
ttccggctct tcctagatta agagcaaacg aaaaccttga agatatttga actaaagcga 5580
cccctaacgt tgtaacctgt gaccgtgatt aaatttcagc gatgcgaggg caaagcgctc 5640
tcggcggtgc ggtgtgagcc acctcccggc gctgcctgtc tcctccagca gctccccaag 5700
ggataggctc tgcccttggt ggtcgaccct caggccctcg gctctcccag ggcgactctg 5760
acgaggggta gggggtggtc cccgggagga cccagaggaa aggcggggac aagaagggag 5820
gggaagggga aagaggaaga ggcatcatcc ctagcccaac cgctcccgat ctccacaaga 5880
gtgctcgtga ccctaaactt aacgtgaggc gcaaaagcgc ccccactttc ccgccttgcg 5940
cggccaggca ggcggctgga gttgatggct caccccgcgc cccctgcccc atccccatcc 6000
gagataggga cgaggagcac gctgcaggga aagcagcgag cgccgggaga ggggcgggca 6060
gaagcgctga caaatcagcg gtgggggcgg agagccgagg agaaggagaa ggaggaggac 6120
taggaggagg aggacggcga cgaccagaag gggcccaaga gagggggcga gcgaccgagc 6180
gccgcgacgc ggaagtgagg tgcgtgcggg ctgcagcgca gaccccggcc cggcccctcc 6240
gagagcgtcc tgggcgctcc ctcacgcctt gccttcaagc cttctgcctt tccaccctcg 6300
tgagcggaga actgggagtg gccattcgac gacaggttag cgggtttgcc tcccactccc 6360
ccagcctcgc gtcgccggct cacagcggcc tcctctgggg acagtccccc ccgggtgccg 6420
cctccgccct tcctgtgcgc tccttttcct tcttctttcc tattaaatat tatttgggaa 6480
ttgtttaaat tttttttta aaaaaagaga gaggcgggga ggagtcggag ttgtggagaa 6540
gcagagggac tcaggtaagt acctgtggat ctaaacgggc gtctttggaa atcctggaga 6600
acgccggatg ggagacgaat ggtcgtgggc accgggaggg ggtggtgctg ccatgaggac 6660
ccgctgggcc aggtctctgg gaggtgagta cttgtccctt tggggagcct aaggaaagag 6720
acttgacctg gctttcgtcc tgcttctgat attcccttct ccacaagggc tgagagatta 6780
ggctgcttct ccgggatccg cttttccccg ggaaacgcga ggatgctcca tggagcgtga 6840
gcatccaact tttctctcac ataaaatctg tctgcccgct ctcttggttt ttctctgtaa 6900
agtaagcaag ctgcgtttgg caaataatga aatggaagtg caaggaggcc aagtcaacag 6960

gtggtaacgg gttaacaagt gctggcgcgg ggtccgctag ggtggaggct gagaacgccc 7020
cctcgggtgg ctggcgcggg gttggagacg gcccgcgagt gtgagcggcg cctgctcagg 7080
gtagatagct gagggcgggg gtggatgttg gatggattag aaccatcaca cttgggcctg 7140
ctgtttgcct gagtttgaac cacaccccga gtgagcagtt agttctgttg cctacgcctt 7200
tccaccatca acctgttagc cttcttctgg gattcatgtt aaggataccc ctgaccctaa 7260
gcctccagct tccatgcttc taactcatac tgttaccctt tagaccccgg gaatttaaaa 7320
aaggggttaa tcttttcatg caactccact tctgaaatgc agtaataaca actcagagga 7380
ttcatcctaa tccgtggtta ggtggctaga cttttactag ccaagatgga tgggagatgc 7440
taaattttta atgccagagc taaaaatgtc tgctttgtcc aatggttaaa tgagtgtaca 7500
cttaaaagag tctcacactt tggagggttt ctcatgattt ttcagtgttt tttgtttatt 7560
tttccccgaa agttctcatt caaagtgtat tttatgtttt ccagtgtggt gtaaaggaat 7620
tcattagcca tggatgtatt catgaaagga ctttcaaagg ccaaggaggg agttgtggct 7680
gctgctgaga aaaccaaaca gggtgtggca gaagcagcag gaaagacaaa agagggtgtt 7740
ctctatgtag gtaggtaaac cccaaatgtc agtttggtgc ttgttcatga gtgatgggtt 7800
aggataatca atactctaaa tgctggtagt tctctctctt gattcatttt tgcatcattg 7860
cttgtcaaaa aggtggactg agtcagaggt atgtgtaggt aggtgaatgt gaacgtgtgt 7920
atttgagcta atagtaaaaa atgcgactgt ttgcttttcc agatttttaa ttttgcccta 7980
atatttatga ctttttaaaa atgaatgttt ctgtacctac ataattctat ttcagagaac 8040
agttttaaaa actcatagtc ttttaaaaaa taatcaagaa tattcttaag aatcaaaatc 8100
attgatggat ctgtgatttc ttttaccatc atgaaaaatg tttgtcaatt ttaatccatt 8160
ctgattttta aaatatgact ttgatatgcc cctgtgatgt gtataaagag acctatttgt 8220
ggccctaaaa tggaaagaac agattagtct ttgatagagt tacttcatgt gatcatttgg 8280
tctctgtgaa cactgaggac agagaaaagt gcttgagggc tgctactaat ctctcagaaa 8340
catttgtata gttcatccat caaatgacac acatactaaa agaataaaga aattgatgct 8400
tattacctac ttgttcctaa agttccacct tggggtatac acccaaactc tgactctctt 8460
ttctgtaact tgaactgtat tcaattgagt gttattttac aaaccacttt gaattccttg 8520
gaaaagaata gacacacact ctcatccaca ggcatagaca cacacactca acacagacac 8580
attgcccatt cttcctctct tctttctcct ctgagctttt tcacattctc tggtggcaac 8640
tatagcagta agagtcacag gatgaacagt caggtggagg atgaccacat tgagttgcct 8700
agctgaaaca tgtgctccgt ctatgtctgc aaagtgaaag aaagctacac tatctcttca 8760
acatagatca gtgggggaaa ttttatactt gggatgattt atatgaatgc atctcatcaa 8820

agttcacaac acattttttt ttcagttttt tattttcagt ttttagagtc agggccttgc 8880
tctgtcgccc aggctggact gcagtgatgc tatcatagct cactgcatcc ttgaattcct 8940
gggctcaagt catgcccccca cctcagcctc ctgagtagcc aggattatag gcatgtgcca 9000
ctgcctcatt atttagactt ttcttatgtt gacttaatct tcccacaaat cttcaattaa 9060
attacttttt ttctacctta aaacatattt tcagaaagtc attgaaatag ggtgttacaa 9120
gaggaaaaaa ttgatgagtt aattttaaat attttatgaa gtgtgaatta taccttttta 9180
gatggaattt ggaatactga atcagtgaca tgcagtttat caatatcttt ccgtttgtcc 9240
tcagatttcc aagttctgca agcacaagtt tctttgactt agttaccttt taactgttca 9300
ttgaaatcat tttcaatgtc tctcatggca tttaacacat agcacattct ataaattttt 9360
tattggttac attctgagtt ctaattgaga gttgaactta cacacagaat ttaagataaa 9420
aaatgaccat gtgaagacac aatagtatag tccagggatt ggcaaaattt tgggtaagga 9480
atcagatagc acgtatttta agccatgaga tctatgtctt ggccaggtgc cgtggctcag 9540
gtctttaatc ccagcacttt gagagcccga ggctggtgga tcacttgagc ccaggggttt 9600
gagaccagcc tgggccacat ggtgaaaccc tgtgtctaca aacaacgcaa aaattagccg 9660
ggtatggtag catgcatgtg tattgccagc tacccaggag gctgaggtag gaggatggct 9720
tgagccatac agctcactgc agaggttgca gtgagctgag atcgagccac tgcactccag 9780
cctgggtggc agagtgatac cctgtctaaa aaaaagaaaa aaaaatctat gtctcaattc 9840
tgctgttgaa gtgtgaaggt agtcataaac aataactagt gtggctgtgt cccaataaaa 9900
cttcatttat caaaacaggt ggtgggctgg aattgtcttg tatgttgtag cttgctgact 9960
actgatagag tggaaagaac atgcactaat cacacaaacc aaagttttag ttgagactac 10020
atcacttatc acctttaggg tcttggggaa gcgtacttaa catctctgag catcacttcc 10080
ctgattagta aaaaatatga tttagaaaac tgcaactacc ttgcagtttt tgtgggaatg 10140
tcataataag acaggacata tgaataattg agcacacttt tatatatagg aaccatggtt 10200
attattatca aataaactct ccaacggaat aattactttg ccaacacgtt ttccatttat 10260
tcttttatcc ttcattacat aactagtttg aaagattgga ggcgaccaaa gaccatttta 10320
taatttcact tatggctgaa gatgtttggt agaagcctca taagaaaagt aatctcattc 10380
ctttataaga atatactttt aacaactact ttttaactca ttgaagaact accttaatga 10440
tcagtgttat ttttatgggt tttgttccct ccatttttgt tatctgcgta caccaatttt 10500
caatcaacat acttcaattt aatagacaaa aatttcttca aatgactcag aaattaatta 10560
gatctaaatc caaaagcaga aagatttaat tatctttata taatgctcag taatataaat 10620
gcaataaata caagaaaatg atgatctttg agtgtcttcc aatgccactc tgctcaataa 10680
gcagcagtgg ccatcagtga aattgatagc aaattctcaa gtcaaaatgt gcttcacctc 10740

actaagctga caaagtcaac ataacatgca caacagggat aactgagttc tcaaaactct 10800

caggtattac ttctgacctt cttctccact ctgtgctctt ttgaggttgg gaagacaaga 10860

tagggtgtgt gtgggacacc tccgctcagg gaagccatca gctctggtgt ccctacagca 10920

tttatacctt gctagtcaca taaccacttg gcacctattt tgtaggtgta cgttatcaat 10980

tacagattac tcataaatta aaggctaacc atcaattaca gattattagt aaataattat 11040

gacctcaaag aacaactgat tggtttgata catggtaacc ttatgaggac tctcatttat 11100

ctcgtttttt taagttatat acctatctct ttggggttgc actacaaaaa tataaaatat 11160

gttgcataag atatttataa aaaataatta attataagtt ctaatggtgt ggtttagtgg 11220

cattcttttt tttttctttt tttctgagat agggtctcaa tctgtcattt cactccaggc 11280

tgaagtgcag tggtgtgatc tcggctcact gcaacctccg cctcctgggt tcaagttatt 11340

ctcctgactc agcctcctga gtagctgaaa ttacaggcat gcaccaccat gcccggctaa 11400

tttttgtatt tttagtagag atggggtttc accatgttag ccaggatggt ctcgaactcc 11460

tgatctcatc atccccgacc tcggcctccc aaaatgctgg gattacaggc gtgagccatt 11520

gcacccggcc tagtggcatt ctttttttaaa aataaattta attgtgtata tttagggtat 11580

gcaacatgat gctatcagat acattagaca ctaaaaaatt actatattga agcaaattaa 11640

tatattcata atctctcata gttacctttt ttgttgtttt tgtggcaagg gcagctaaaa 11700

tccacttatt tatcatgaat ctcaaatata gtacaatttt atcacctaca gtcctcatac 11760

attagatctg tacacttttt catcttacac atctgctact tgcttggatc ctatggccta 11820

tatgtcccta ttttctacct acttttccac ccctattaac cctgtttttt acgtagtctc 11880

tgtatatttg aattttgttt caagcttcca catatatgtg agataatgta atatttttct 11940

ttctgtgttt ggcttatttc acttagcata attttgtctg ggttcatcca tgttgtaaat 12000

ggtaggatct tgtttttttta gggctgactg atattccatt gtatctatgt accacaatct 12060

ttttatctac ctatctatca gtagacactt tagttgtggc tattatgttt ttcttttttt 12120

ctttttttgga gacagggtct tgctgtcacc caggctgcaa tggagtggtg ttatcatagc 12180

tcactgtaac ctcaaacttc tgggctcaag agatcctcct gccttggcct cccaagtagc 12240

tgagactaca ggcatacatt accatgcctg gctaattttt aatatttttt gtagatatag 12300

catctcactc tgttgcccag actggtctca aactcctaat tcaaatttag aatagagtat 12360

gacaattctg taaaatataa aaaacatgtc cactccgtat aggaagttat acaatgagaa 12420

gaagacaaac actatttaca ttactcttga taagtttttt acaaagaaat aaaacacttt 12480

aatttctaat gttttaaatt ctggtttgct aaataaataa atattagttt tagtgttttt 12540

aaaattcctt atatagttat aagtgatctt cctgcctcag cctcccaaag cactgggatt 12600

ccaagcaaga gccactgtgt tggggccctt ggaaacagat atgctgaaat cttttcttgt 12660
ggatctacac ccagaagagg gattgctggg tcatatgcta ctctattttt aatttttctt 12720
ttatttttag tgaatatgta ataattgtat ataattgtgg gatccagaat tatatttcca 12780
tacatgtata caatgtgtga taatcaaatt agggtaatta acatatccat tacctgaaac 12840
atttatcatt cctttgtggt gggaacagta aaaattaaaa attctctctt ctagattttt 12900
gaacatatgc aataaactat tgttaagtat atcaccctac agtactacag aatgctagaa 12960
ctcattcctc atatttggct ccaatttcat attctttaac caacctctcc atatcctccc 13020
ctccctctta ccgttgtcag cctctaataa tcataattct actctctact tctatctcat 13080
tgtctttgat ttagaatatg tttcataatt taaccaaagg tcaaattctt aggtactgct 13140
aaggcaaaga acaaagatcg cattccagct gttagacatt tcttactact agtcattttt 13200
aagacaacat ggggtgcagg tggtgaggat gagagataga gattgaaaca tattctctta 13260
aatatcagct gttctcactc tgcatagttc cagcacaaac aaattccagg tactatggtt 13320
agttaaataa caccagccac taacaacaca attcaaattt ctgttaccac agtataccga 13380
aagtcattgc ataaagtaca aactttgctg ctaactcttc agccttcaaa tcattacata 13440
aataacagaa acccattata atcagtgaca aaaccacagc acttctttca aagcttttttg 13500
gagattggtt gcttcacatc tgttatgcag ttcatacaga cagcaatgcc cggacttgtg 13560
tggccacatt gtctcccagt ggtgagccca tgtgatgttt cacgaaaatg cgcaatcaaa 13620
agaggaaact ggccagcaaa gatgaaagag tagcaaacaa aggaagtgaa acattctgga 13680
agtaaaattt gaatcaaaca taagttgatg tatacaggaa gtagctaccc tgaggatgtt 13740
gtcactgctg caattcagga gactctaaat atgcagtcag aggaacgtag tgaggtgaag 13800
gtatccgtat aatggggaaa gaggttgtga taaagagtga aggtgtccca gaggaagtgt 13860
tgctgaaaaa tacaccttat gttaaataca ctgtcagtat atcatgacat taaagtgcaa 13920
atgataacat tttgtaaact gatccaaact taaaaaggag tatgataatt ctgtaaaaca 13980
taaaaatcat gccgattcca taaattatac agtgtgaatt acactgaaaa atccaacatt 14040
agagaggata tgaatacaat tttttacaag cataatttta ataatacaca taataattat 14100
ttgtattcaa gtttagtaat gttcaaggtt tggaagaaat tctgatcctg tgtagagacc 14160
ctagtttgaa tgtgcttata gcctattatt acatgtgtaa tgttacataa attacttaac 14220
tcggattttt aatttcatca gctatttaaa atgggcataa tataactata ttaaatggct 14280
gttatgaaga ttaaataaga tgatatgtaa aatgtgtttt ttgtttgttt gtttgtttgt 14340
ctgtttgttt ttttgagaca gagtcttgct ctgttaccca ggctggagtg cagtggcaca 14400
atcttggctc actgcaagtt ctgcctcccg agttcatgcc attctcctgc ctcagcccct 14460
cccaagtagc tgggactaca ggcacccgcc accacgcctg gctaattttt tgtatttttg 14520

gtagagatgg ggtttcacca tattagccag gatggtctcg atctcctgac ctcgtgatct 14580

gcccacctcg gcctcccaaa ttgctgggat tacaggcatg agccactgcg cccagcctaa 14640

aatgtttttt ttacataatg ggtgttcagc acatgttaaa gccttctctc catccttctt 14700

cccttttgtt tcatgggttg actgatctgt ctctagtgct gtacttttaa agcttctaca 14760

gttctgaatt caaaattatc ttctcactgg gccccggtgt tatctcattc ttttttctcc 14820

tctgtaagtt gacatgtgat gtgggaacaa aggggataaa gtcattattt tgtgctaaaa 14880

tcgtaattgg agaggacctc ctgttagctg ggctttcttc tatttattgt ggtggttact 14940

ggagttcctt cttctagttt taggatatat atatatattt ttttctttcc ctgaagatat 15000

aataatatat atacttctga agattgagat ttttaaatta gttgtattga aaactagcta 15060

atcagcaatt taaggctagc ttgagactta tgtcttgaat ttgtttttgt aggctccaaa 15120

accaaggagg gagtggtgca tggtgtggca acaggtaagc tccattgtgc ttatatccaa 15180

agatgatatt taaagtatct agtgattagt gtggcccagt attcaagatt cctatgaaat 15240

tgtaaaacaa tcactgagca ttctaagaac atatcagtct tattgaaact gaattcttta 15300

taaagtattt ttaaataggt aaatattgat tataaataaa aaatatactt gccaagaata 15360

atgagggctt tgaattgata agctatgttt aatttatagt aagtgggcat ttaaatattc 15420

tgaccaaaaa tgtattgaca aactgctgac aaaaataaaa tgtgaatatt gccataattt 15480

taaaaaaagt aaaatttctg ttgattacag taaaatattt tgaccttaaa ttatgttgat 15540

tacaatattc ctttgataat tcagagtgca tttcaggaaa caccccttgga cagtcagtaa 15600

aatgtttatt gtatttatct ttgtattgtt atggtatagc tatttgtaca aatattattg 15660

tgcaattatt acatttctga ttatattatt catttggcct aaatttaccg agaatttgaa 15720

caagtcaatt aggtttacaa tcaagaaata tcaaaaatga tgaaaaggat gataatcatc 15780

atcagatgtt gaggaagatg aggatgagag tgccagaaat agagaaatca aaggagaacc 15840

aaaatttaac aaattaaaag cccacagact tgctgtaatt aagttttctg ttgtaagtac 15900

tccacgtttc ctggcagatg tggtgaagca aaagatataa tcagaaatat aatttatata 15960

atcggaaagc attaaacaca atagtgccta tacaaataaa atgttcctat cactgacttc 16020

taaaatggaa atgaggacaa tgatatggga atcttaatac agtgttgtgg atatgactaa 16080

aaacacagga gtcagatctt cttggttcaa cttcctgctt actccttacc agctgtgtgt 16140

tttttgcaag attcttcacc tctgtgtgat ttagcttcct catctataaa ataattcagt 16200

gaattaatgt acacaaaaca tctggaaaac aaaagcaaac aatatgtatt ttataagtgt 16260

tacttatagt tttatagtga actttcttgt gcaacatttt tacaactagt ggagaaaaat 16320

atttctttaa atgaatactt ttgatttaaa aatcagagtg taaaaataaa acagactcct 16380

ttgaaactag ttctgttaga agttaattgt gcacctttaa tgggctctgt tgcaatccaa 16440
cagagaagta gttaagtaag tggactatga tgccttctag ggacctccta taaatatgat 16500
attgtgaagc atgattataa taagaactag ataacagaca ggtggagact ccactatctg 16560
aagacggtca acctagatga atggtgttcc atttagtagt tgaggaagaa cccatgaggt 16620
ttagaaagca gacaagcatg tggcaagttc tggagtcagt ggtaaaaatt aaagaaccca 16680
actattactg tcacctgatg atctaatgga gactgtggag atgggctgca tttttttagt 16740
cttttccaga atgccaaaat gtaaacacat atctgtgtgt gtgtgtgtgt gtgtgtgtgt 16800
gtgcgtgtgt gtgagagaga gagagagact gaagtttgta caattagaca ttttataaaa 16860
tgttttctga aggacagtgg ctcacaatct taagtttcta acattgtaca atgttgggag 16920
actttgtata ctttattttc tctttagcgt attaaggaat ctgagatgtc ctacagtaaa 16980
gaaatttgca ttacatagtt aaaatcaggg ttattcaaac tttttgatta ttgaaaactt 17040
tcttcattag ttactagggt tgaatgaaac tagtgttcca cagaaaacta tgggaaatgt 17100
tgctaggcag taaggacatg gtgatttcag catgtgcaat atttacagcg attgcaccca 17160
tggaccaccc tggcagtagt gaaataacca aaaatgctgt cataactagt atggctatga 17220
gaaacacatt gggataaatc ggctgctatc ataatcattc ctctcccaca tcagataaat 17280
gaattaactt tttgaatagg gttatttaat ataaagtgct taagtctaat tatgagaaga 17340
aataagataa ttacacttca atggttaaag agagggagaa taatttgcat attatgcctg 17400
atgtaaaatg tttattatgg gtacatatta agtgctaact aattgttaat tgttcttgct 17460
acaagtctta atgcagggaa acaagaaatt attacatagt acctaatatt atcttctaat 17520
attaaagaaa caatttcccc taaattcatc ccattagctt tttttttttc ggtggggcag 17580
gggagaaata cagacttcag taaacttggg ctgggaactt tctacctaca aagttcaaat 17640
aaaataaatt atcctagtta gataatatca atgaaaaatc caccaactta aatcctggct 17700
gtttgatctc aggaaattat ttcagttatc aacttaatgc atcatattat agaaatatat 17760
gaaaatgtgt ttaattaaac ttactgaatg atatgttttt tcaggtactt taaaaataaa 17820
ctatgatata aagttaccta tttttcatgc aagtatagta taaagaaatt tctaacactg 17880
gagattttct gaaggttttg attcttataa atttattaca tcataatgaa caaaactaat 17940
tttcaacata ttatgattta aatttcctta gtaaattgtt tcaaatttat tttctttaaa 18000
tccatattta catatgtata tttaaatata catatttact tgtataacaa ttcaaaacca 18060
tatattaatt ttataatttt gtttaatgtc aaaggttaga tttggctata tctattctaa 18120
aagttggtat cacatttcct ttttggaatt ttattttttaa agtagctaaa gtcaaatata 18180
aacctattat ttatattaat gcagacatta gaggtagaca ctaaattcat tttagtatat 18240
tctaaattat ttattatcta ctatgaaata atataaagaa aaataaagca gaatccctga 18300

tttcaaagaa ctcaattgcc gaaaaacagt taccatttat tagacccaaa atgtactaat 18360
atgagtgtgt ctcttttcct tttgttttgt cacccgtcat ttggaatgtc agtgagtaga 18420
gagatagtgt gaaaggccct caaggggaaa aatagaggtt aaaggtcagc agagacccta 18480
ctagagaaat cagttctaca gaaatgtttt taaatgtgtc gattattgct acatgtacac 18540
tctgtcattt tgtaatgtag ccattttatt tatgattata ataataaaac aacaaaatta 18600
taataatgtg tagagtacat tttactgtgc agtgtattgc attaaaacta gattaaaatt 18660
tatacatata taaaaggcta tctagatatt ataaaattta tggctggatc tgtaaaaaat 18720
tcaaaaccta tttttaatct cgctttgaga ttttataaca agaaaatgtt cgtttcaagc 18780
aaaattttca attcacgtcc ttgaaaagga aaaaaatgac aacttgaaac acataattga 18840
ctatttttaa aggatcaaca tttcagaaat gttttaaaac ataagatttt cagtacagct 18900
tttcgctggc atttaaatcg aactttgaat tgtaaatagc tcttgctctt aaggagacat 18960
cagccatatc cttagaagtg gcacggagtt gttaggtagt tgtacaaaat tctagcctaa 19020
aagacaaata gggagcaaca ctactgtgga ccgtttctgg tcttgggctg tgtggctatg 19080
tcaggcttgc ccacattgcc tgtactaagg agaaagcctc ttgtccttac agaccccctt 19140
agcttacata gtctatttga aaacaaattg ctttgtccac accatttaaa tattggcttc 19200
aggccaggcg cggtggctca cgcctgttat cccagcactt tgggaggctg aggcgggcag 19260
atcacgaggt caggagatcg agaccatcct ggctaacacg gtgaaaccct gtctctacta 19320
aaaatataaa aaaattagcc gggtgtggtg gcgcgcacct gtagtcccag ctgctgggga 19380
ggctgaggca ggagaatggc ctgaacccgg gagtcggagt ttgcagtgag ccgacatcgt 19440
gccactgcac tccagcctgg gtgacagagc aagactccgt ctcaaaataa ataaataaat 19500
aaataaataa gtaaatattg gcttcttcaa ctggtgagat gaaacctata caatagtcat 19560
gtgaatagca ctaaacagct gacatggtgt aactcctctc agactgaggc ttatctgggg 19620
agtacaaagc atgtcaagaa aatgtgcctt catttcctta gatgagtgtc cccatcctcc 19680
actctcctcc actgttctcc tctctgcttc tatgatatca acttttcttt ttctttagat 19740
tccacatgag tgagatcatg tggttgtttg cctttctgtt tctggcttat ttaactgaac 19800
aagaaagttt ttgacatgaa attaaacttc tgcttgtaaa ctcaattcaa actatttaca 19860
ctgtcttctc aaaaatgtta acttatttta ataaatctac tgaatgaccg tatctcattt 19920
tgttttatga aaagaaattg taagggtgct caatagcctc ttcattttca tactgtctag 19980
ctcctgtgct cctattaaaa ttactgcaaa tttagctttt taagaaccct ttgtttcact 20040
acctgaagtt ctataaaaag atccaagttc cttcacaacc gtttcttatg ctgttattcg 20100
tacatatgtg ataataccac gtctgaacac gtagataata agtaggggct gggtgcggtg 20160

gatcatgcct ataatcctag cactttggga ggctaaggcg ggtggatcac ctgaggttag 20220
gagttcgaga ccggcctggc caacatgatg aaaccctgtt tctactaaaa atacaaataa 20280
taataataat aataattagc caggtgtggt tgtgggcacc tgtaatccca gctactcggg 20340
agactgaggc aggagaatag cttgaactca ggaggcggag gttgctgtga gctgagattg 20400
tgccattgca ttccagcctg aacaacaaga atgaaactcc atctcaaata aataaataaa 20460
tagaagtatg tattgtgttg cttagaaggt gtggtggaaa ttaacttgct gagtgagatc 20520
aaaggattgg cactgaattg aaataaagaa atattcatgc tgagtctggt tcaaatataa 20580
ctgcacctgt aagaattgct ttctgtaaac tttccatagt ataaaccaaa tccaaatcac 20640
tcatggcttt acattcctga tcgttaaact tgaagcactt tttaatactg catgacttta 20700
gccaaaatat cttagccaag attcaatgtt tggttgaacc acactcactt ggacatcttg 20760
gtggcttttg tttcttctga ccactcagtt atctatggca tgtgtagata caggtgtatg 20820
gaagccgatg gctagtggaa gtggaatgat tttaagtcac tgttattcta ccacccttta 20880
atctgttgtt gctctttatt tgtaccagtg gctgagaaga ccaaagagca agtgacaaat 20940
gttggaggag cagtggtgac gggtgtgaca gcagtagccc agaagacagt ggagggagca 21000
gggagcattg cagcagccac tggctttgtc aaaaaggacc agttgggcaa ggtatggctg 21060
tgtacgtttt gtgttacatt tataagctgg tgagattacg gttcattttc atgtgaggcc 21120
tggaggcagg agcaagatac ttactgtggg gaacggctac ctgaccctcc ccttgtgaaa 21180
aagtgctacc tttatattgg tcttgcttgt ttcaggcatt aacccagata aatgccatgc 21240
aaattttata attattatga ttgtttcaat ttctggaaga aagttaatga aacaaaaaat 21300
gtagtaaaat gccaaaggaa cagtgacatt tcagaaagaa tgagggcttt catgttaatt 21360
gtaagtcttg gaatttctct tccttggagt aacaaatccc tttgtgccta atttcctaat 21420
ttccaaaata aagttctttt acttatttct ttatagtgac atcatctctt attaaatggc 21480
atatctgcat attacataac agttcattgc caaatacata tttgtgggaa atgagagact 21540
taaaatacat accaaccaga gatatagttt tgaggtagat tttaaaattc tgagaagaat 21600
tttgactgaa tttttttgac aaacatggga cacgaataag attataccaa agatattata 21660
actttcattt taaatatgga actaatacag tatgaggtgt caacaacgtt gaagtttcac 21720
aaacatcacc actacaacag caaaataatt tttgcttttt ccctgccaca atgacctcct 21780
tgctatttct tgaataaatc aagcataccc ttgccctgac acgttcttgg ggaggcctgc 21840
cctaatctat ataaaattgg agccattctt ctcacctctg gtattcccag tctccctact 21900
ttttttcctt ctttctttct ttttcttttt ctttctttct ttccttcttt ctctctttcc 21960
tttctttctt ttcccttcct tccttccttt ctcccttcct tccttcctcc ctctctccct 22020
cccttccttc ctccctttct ttctttctct tttttctttc ttgcttcctt ccttccttct 22080

ttccttttct ttctttttcc tttctttgcc aaagtgttat tcacctttaa atataataca 22140
taatgtgctt actttaatgt atgattttta ttttatttct cccttctaga atgtaggcac 22200
catgagagtg aaatatattt attttgttca ttgatatttc acaagtgtct gggagagttt 22260
ccaacttaca gtagacaatt aacaaacatt tattaaatta aggagggaag gaagtgagta 22320
agcacaacaa ctttcatttc tgggtctttt ataatcatat gcttagtata agaacagtgc 22380
tattcagcta tccaaaagtt acaatcaaaa tgattttgga tgaatatctt gaaaattgtg 22440
agaaagaagt tttatttgct ggcaaactat tctgggttgt ttccacttca tgtaatccta 22500
agtagcagcc ttaccttgat agcccattaa aactctgata ataaaaaggc agaacaaaaa 22560
tatctgtgat atatttagat ttactacatg tacttacatg tctagtgtct ggtgcaatgg 22620
atgctaatga tggcaaatcc ttactgggct tctagtgaag ttcttcagct aatgtttgaa 22680
tgcatggttg gtcatggtgg taccccttttg tacaaaatat gcttttcaaa taatcttatt 22740
agggataata attatattaa ttcctggttt ccatctaaaa ttttaattct atttatagct 22800
tcgtaagatt tcacaagtta agagggacct cagattaaat tagtacacag gcaattaatc 22860
agttttgtgt ctccgaccct tttcacgggc taatagaagc tatagaccct cttagcttca 22920
gaaaaatgcg cactcacata cgcacatcaa agagcttaat gggaagtcca ttgacagacc 22980
ctctgttcag atcaatcttc tgattgtaga gatgaggaaa cagaaatcta cagaggaagt 23040
gggtagtcca agattgcaca gtcatttgga atagactgga caccagtagt acttttccag 23100
ccactatatc acttccccaa gcacttcctc aaaacttacc ttcctttggg tctttataca 23160
ttcagttatg gacaactaga tttaactaga ggattttatt gcttcagaat attaagcaac 23220
agggaaacat gtaccgtctt ttattcacct gcatttaagg catacaatat aaattgcaaa 23280
tggagcatga aagtgcttaa tcttttacaa aactgggttt gctttccacc catctaaaaa 23340
tacttctatt tattttaata tttaaagcag aaatctaagt gatgtgacaa aattaatcat 23400
ttggagatat ttcccttata ggtagtatag tttcttactg atttctaata tgaaaatgaa 23460
gccatagaac ctagaaattg cagcatagtt gtggaaataa acattggact gagagtgaaa 23520
atggctagtc ttcctctctg ctcatacacc acctgactgg ataacctttc gcagatctcc 23580
taaaagtctt tctcataaaa tgaggaagct ctactagaaa attgttgaag tctaatttag 23640
caataaagtt ctgagtttct ataataattc aaagaatact ctaataaatg tctgcaattg 23700
tggtcacatc tatgggatgc taaaaaatct ggatggtttc aatgaaagta tttaatttgt 23760
tcattatgaa ctttgaaata atttatttca ttttttaaac tttgatcaaa atgaccctgg 23820
taaatagaaa taagcaaact ctttttgctt gaaatgctta ttaatgactg cattgagaca 23880
ctcattcatc attcaagaaa gaatgtttgc tcacactgtg ccagaaactt ggaggaagag 23940

ggatgtgaca agtaggggta ctggatgtct agcttgtaga agtggattaa tggctctgct 24000
tttaagatca ggaacactga aagggagtaa tggcaccggt tttcaccttt catgcccttt 24060
gagggtatct ggtccatcac cctctagttg atgagggagg gaaagttccc tctcccttca 24120
caaataggtg gaaattaaat gacataattc tgaacaacca ataaatcgag agtaaatcaa 24180
agcagatacc tgttttgtta atttgatcat atgaatgtag ctgcccttag taataatttc 24240
taagtataag actagttaaa ggacaaatga gttatcttga attataagat tttgttttac 24300
agaacaatat taactcttgt gtttagtaca ttagaataat agatcttttg atccatattt 24360
ttactcatgt gcacataaga agttatcagt catacaattc atttcttgaa gttcatacct 24420
ttcattggca gagtagaaac aggttaaaag tgcacaggca gaaattttaa gtgcaaagca 24480
acagtgatgt tatatagaga aaatttatat ttcctacttc tattgaagaa gaaagatctg 24540
cttgttctaa gaatattgta caaagaaagt gacttgaatc agcgttattc tgtaatgcta 24600
ctatgcgtgc agtgtggagt agccactaga acacttggtc tatcccagct cctcaacagt 24660
gtcttgcttg tggctggtgc tcaaataaat ccttgctgaa ctaatgagca tctctttcat 24720
gccacatgga atgctctaaa agagttggat cctgaagttt ttatattttt gtaattttct 24780
ggagttttag agagcaaaag tcctgaataa actgtgaagc cactgcctga caaataatac 24840
agcagtcagc ttcgttatca tatcccattg agacacgact tatctacatg atgattaata 24900
gttttcacgc aagaaataag cttgaaatgt ctgttgcctt ggatacttaa aacatccagg 24960
ttcagcgatg ttatttattg ttgttcaaaa tcagaatgaa gttcctaagc aatgccattt 25020
tggaaaaatt acatcaatat attatgaaca actttttttta aatcttgatt tcaaatggat 25080
tgacacgtgt atattctgta ataatcctga cttaattcat aaaaggatag ctagccagtt 25140
gtgtgctaga tgaataaaaa aaaagcaggt tttaaaatgt caggtttgac attgtgaata 25200
taatatctaa gtatcctttt actcatttcc tttgacttac tatggctgtc atgttgggct 25260
tcatgaaaat ttatttttaa acacttgagt gttatggacc ctctgattaa atgattaatc 25320
agatgatgta tgttgccatc agctgaatca tttaatgttg atttcacaaa caagcacagg 25380
tcacaggcaa catttcagat ttctttgaag aagcacacac aggtcacagg cataatctta 25440
aaataatttt ataacaaggt agtaataaga gatgtcagga ctggagaaat attttaattt 25500
atagtaagct ttccccttaa gtgtctaata attgttaata taatacattg cctcaaataa 25560
ttaaaagttt ggttcttgtc cttgtgcttg acttcagaag ataaccagat gactattagg 25620
tatatttaga cctaaattaa aagctttgag acacaatgaa ttgcctgatt tgtatttgtg 25680
tttcgagtgg catatactat tactggcact ataatcttag attaaagcat actgtgatta 25740
ttaaagaaaa atttaagatt gatttgtttc taaaggtatg taacagtgac attttgcaat 25800
gtggtatgta aaagttggta tttctcactc atatgagagc ccactaatgg tacataaact 25860

gtccccactt agaaacacaa ttattatggc ctttctttgt atctgacaaa atttcactgg 25920

gttcaagatg gatgaatagt gaattctaat gacccttaat cctgtaaggt tctaggtggg 25980

aaagtactct gtaattatgt ataaaattat aaggaaaata ggcttactgc tatgttttca 26040

ttaaaaatca ttaactgagt acttaatatg tgccagacac tcagctgggc accatgagaa 26100

atacaaaact gagtaacata tgggtggctc ctgccttcaa gaaatgggca gttcaggccg 26160

ggagactgac atatttaccc tgggaaaaag ggagcagctg tggtctctga gaacaatatg 26220

gtttgttaca agtatatatc catcatggaa aaaaagagat ttatcttaga aatgagagag 26280

gctgatgctc tcaataaata tcatacatta aattgtgttt ttgtcagtag actgaaatta 26340

cctcacatac acgcacagat agtagccatg atattttagc tgcttagata tagagacaaa 26400

tacttccacc caaatcttag gatcagtggt taatagtctg taagcattac aatcccacaa 26460

catatgcatg actatacatc caattttaat attcaaagaa ctgattgcga tgatagtttt 26520

gtttgtcaaa gaaatgtatt ataggatgag tgggatagaa ctgcatcacg ttacaccaac 26580

aaataggttt aaatcatatt tgtgcacttc ccttgttcct tcataaatgt ttaacatagc 26640

ttaaaattct gtggactgca acgtgagagc aatgaccaca cttctgtgaa cccattttta 26700

ctgtgcatgt gctaacgtct attgttagta ttccttcact tgcaaagatg gcatgataat 26760

tttgctggtt tcattaatga gatactgtta aatgtaggat gacttcaaac ttagttgtat 26820

tgtaaaatta tttttaattg tatacattta agttgtacag catgatgttt tgagatactt 26880

atctttattt atatatatat ataatataca cacgtatata aaagtgattc ctacattgaa 26940

gcaaattaac atacccatca tcatatggtt atctttgctt ttttactatc agtgcctaaa 27000

atctactttc ttgaaaaatt accagtatgc actacaatat tattaacaat aatcttcatg 27060

ttgtacatta gatctttaga cttactcatc ttacatgact taggtttgtt tttacctcta 27120

ctaccatctg agccatattt ccactttgta atttgataat aaacttggaa aaatagcact 27180

tatatgttta ggtgacgggc ataaatagga taagatgtgt ttatatatta ttccatatat 27240

cttgtctcca actacaatga taaacaacct gtttgtccct aaaaagtaag aaataacttg 27300

acttttctgc cccttcaagc ataggctgtt agcttttaag ttttagggag acattgatga 27360

tgctatttgc tttatcaaga ggaaattgtc aaaagaggtc ttttggttct caaactattc 27420

aaagtattta aaaatcagga caaaatatgt ttacgtgata ttcaagggta cagaaatgag 27480

gtaaatgaga tgccaattgt atttgtcatg caaatatata attacgtgta tgagagttag 27540

atgatacatc tcatcaattt aattgttctt ctacaaggag aaaatgaaca atttgtcaac 27600

tcgtatatga agtaattttt ataagaaatt ttattaaaac ttttaacaac atttggattt 27660

ttaagttgca atttaaatat ccccttctac caggtgattc tggaatcact aagcagttac 27720

ttgtgaaaat tccaaagtag catttaattc ttattaatgt catagtgaat actaatgcaa 27780
agaatactga gccagaaatt atgcttgttg aataaataga ttatttattg aacaagtaag 27840
tgaaaaaatg gaaataaaga acggatatat attttatctt cctgcttaga tgtgggactg 27900
tcctactttt ctctggtgtt cacaacaaca atatgataaa tctaattgga attcagttca 27960
taggaatgaa ttcagttaca ttatggattg tgatgaataa tgtacacttt taatttaatg 28020
aaatcaaata gattttaact atctatgctt acaatggggt gacataagtc tgacaatcct 28080
taatatcaag tcatctccaa ttcacatgta tacacacttt ttttctattt ggctattggg 28140
aatcctcaca aaaatcgaaa attgcccttt cagtgtacgt tacggtattt catgccacac 28200
agattttctg aggttgtaca tacagctttg ccttgaggtt ccaatttttg ctcagtggat 28260
tgagtatata ttatttgcta tatatcagaa gaggcatgtg cttcctactt atgtcaggta 28320
actttgggat taatataatt gtcctacaaa gcatagatag atagaaatac ttcatcctta 28380
atttctaata ttatgacata tctaaagtag gcacctttaa aagttaatct ccactaaata 28440
ctaatgactg cttatagtgg caattcatct ttcatggtag tcctcctaca aaggtatact 28500
aacatttatg agtttgaaac aaaggcaatt cacaagtgtt ctgctagaga tggtctatat 28560
ctgctgtttg atccagcatg atggccagct ggccctcctg tgcatgacgg ctcgtggttt 28620
aactgcacca ttttgtttgg tcatatacag ggaaaacatg gcatggtgtg gagggcatgg 28680
gcttgaattc agggaacaga gagttggtct tctctctctc actctactgg atgatgtcat 28740
ctcccctctc taagcatgag ttttcttatc tgtgaaataa aaatgttgaa ttaaatgagt 28800
tcaaaatgct ttcagtctgt gtttaatagc ttgaatctta agacaatgta ttcaattatg 28860
cgttgccaga tccctggcaa ctcatgtaac ctttctaaac catagctact catctgtaac 28920
tggccagcca actgcccagg gttggagtgt gaatgaaata agataatgca gacaaaagat 28980
ttttaaaaat tgtagtgcat tatacagttg taatattttg ccaagaactt acattttctc 29040
taagaagtgt gtcgatacat gatcacagaa aatcttttcc atattccttt gtagtttgat 29100
gatattaagt aagtaaattg tataacacaa agagggaaaa gcatcactga acatgccgtt 29160
ttatttagct aaataaaatg taatcactat tagttttcct ctgatttccc caaagtcatg 29220
tgattccatt gagtattatg cacatggtat aattagaatg gattctctgc tcaaataatt 29280
ttgggaaaca tttaaattaa caaagtttaa aagtatctct gttaagctga agcaaatctc 29340
aaaggcctta atattgtatg taagaggaat agttaccatc tttcctaatg cctctttgac 29400
gccaaaccca tggagaatag ttctaggtgt tcagtaaaac acagatttgg gatgccacag 29460
gttaattgga actgtcccct gcaatccttt tctctttttc ttaataatgg ctgattgcag 29520
gtcctagatg aaagacattt agagagatta tcaggactca gcatcccata tcagaatcca 29580
ttcttttata gtcattttct gttacatttc ttgggacaac accaaagaaa tgaccatctt 29640

cattcacata ggctttgtac caaatgctga caaagatcct tggtgaccta gatgggggca 29700
ggtctaagta gattgcagct gtaaaattgg ctgatgaatg atctcagccc cttttactca 29760
cactcaaagg caggacagtc cattaagggg aaggagggca gagtttttcc ttaggccaat 29820
tccctatgcc agaacttttt agaatggaag catttccaga ggagaaacaa ccccaagcac 29880
agttcaaagc cccctcctcc caagttcatt tgaaagtggg atggtttatc tgcaaagggg 29940
gaaaagatga gggataggga cgggaatatc cctacccttc agagagtctg gtttcatcct 30000
gcacttttac tgcacagcca caaatgcctt ggggtgaatc tacaatatga tacatcatat 30060
ggtctaaacg tgcctggctg atcctctcta atacttcagg ggtctaaaag ggataacatg 30120
ctctcctgtt actcaccgac tctgtccgcc atatttcacc cagccagcca ctgccttcac 30180
ttccgtccga ggcctaatct gagcccatgg gaaacctaag aacccctacc acaactgcct 30240
caactcttgg gaatcagggt gtatgggggt gacaggaagt gagcatacat tctccaactt 30300
gatatgtcag cccccacgtc tgtatgaatg tttgctcaca ctgtgactgc cggccttgct 30360
cctcaggctg catcctacca gggagtaaga cccaagtcct tcctgctttc agacaacacc 30420
aagcctcatg agtccccact cagaggaagg accagagaca aactctaatg ttccactaat 30480
acttcccttc ttattacttt ccttgaaaat cccttctccc tctttctttt tatacttcgc 30540
taatgaaagg taatgaaagg gtctggcact tggaatttag aattgataca tggtttttaa 30600
cccgcggacg tattccacaa taacccttgc atcttctact aagatgtggg ctaggaaggg 30660
accagccagt tcccagggtc acagtgcctc agctgatgtt tcatattttc agcaacttta 30720
tgttagagat gtccatcaat cagaacaata tggttagaga ataaactaat aaaagtcatt 30780
tttgaggaca tgttggaagt ctatcaaaag cattgaaatt atgcatgctc tgaccagtcg 30840
catgtctaag aatttaaata tgatcataag tttaaatatg aagatgttta tcacagaatt 30900
gattataaaa caaaattgaa aaaaatagtg ctagaagttt gatcataggg acctcattaa 30960
atgcattatg gttgatccat gcagtggttt gctgaacagc cattaaaatg ttgtagaata 31020
attattaatg gtgtggaagg atgctattgt tgcagtatgt gaaaagaaca aattacaaag 31080
cagtttgtgc agcataatat ttttattttt taaaaacctg tatgtggctt atgtacatat 31140
aaagacgtgg aataaatgca caaggtactc agtttttctc agtgaagccc attttgcatt 31200
ttgggctggg taattcttcg ctgtggagaa ctctcattca ttgtaggatg tttacaagcc 31260
ctgggcctta cctctttaac gccagtaggc acccccagca tggcaacaag cacaaaatgg 31320
tctctctcat attgcccttg aggaaatttt gcaactaagt aactattact gggtcctaga 31380
ttacagtctg gattattgcg ttcctttctt atttttattt tctccaattc cctttaataa 31440
gcatgtactg gattcataaa aaaacaacat aaatggtaat tacaatattc cgcactggtt 31500

aaaacttatg taaataagca ttctgctgct ttagccacaa ttgcaattta tgctccttct 31560

ctttcttaag ttcccagttc ccacgtacat tcattcgact gattcaaaag tcattttagc 31620

ttgatagact cttaaaagtt agagttatca tttctgctat ttattctttc aattatccat 31680

ttgtccaccc atccatctga tccattttgt tgatgcatgc tgtgtataaa atactacacc 31740

agcctggtgc ggtggctcac gcctgtaatt ccaggacttt gggaggccaa ggcgggtgga 31800

tcacctgaag tcaggtgttt gagaccagcc tggccaacgt ggaaaaaccc tgtctctact 31860

aaaaatacaa aaattagcca ggcatggtgg cagacgactc taatcccagc tacttaggag 31920

gctgaaccag gagaatcgct cgaacccagg agatggagtt tgcagtgagc tgagatcatg 31980

ccaatacact ccagcctggg tgacagagca agactccgtc tcaaaaacaa acaaaaaaaa 32040

tacaatgcca agcatcataa aaaatatagt gatatataag acctatttgt tgtgctctag 32100

gcattgacat ctagctgtca accattaata tgtgtaggag tctatctatc aatattatgg 32160

actgtgcttg aagacttctt ccccaatctt tttctcttcc cattaagttt gaagtgaggt 32220

tttctgagtg aagtatcata gtacatacag tctcattatt tttcaaaaat ctctggttat 32280

agtacatttc tttcctttat cccctttgtt cccaactatc aaaccatttt ggatatccag 32340

tattggtatc cagtattatt aaaaagcaaa acagagaact attaacaaaa aaatttgtag 32400

gagtaattgg ttgtatggta tccagtacta ttagatagta aatcagaaaa ttattaacaa 32460

aaattttaga cgaataatgg attgtcttgc ccaagtgaat tgagtgattt agttgttctt 32520

tcatttttag caagtacagc tgatcatttg aggccttact cattgtttga ttttgcaaat 32580

tcttactatt ataaatgttt tgggctctga gaaagctgtt gtcttaatct gtttgtgctg 32640

ttataacaaa atacatgaga ctgggtaatt tacaaacaac agaaatttat ttctcatagc 32700

tctggaggct gggaactcca agatcaaggc atttgtcttc aggttcagta tctggcgagg 32760

gccggttctc tactcccaag atggtgtctt gtcactgtat cctccagagg gccaaatgct 32820

gtgttctcac atggtagaga gatagaaagg gccaactcac tccctcaagg cctttcataa 32880

tgttaccaat tccacttgtc agggctctgc ccccgtgact ttattacctc tgcaaggccc 32940

caccacttaa tactatcacg ttggttatta cgatttatca catgaatttc gaccatacta 33000

gttgccatcc tttcattttc atatatcctt aaaactttgc ctttctcatt ttaatgtact 33060

ttatccacag tatgccaact tttcgatact tttgttaacc tgtctgacga tatataggaa 33120

actgtaaaag tgcagttttt gatacactct ttagctgccc gtttacttct actgtcgtta 33180

gagaacccca tccatagtgc atgtgtttat tttgtgtatg aacaaagact ttatatatag 33240

tttgggtcat ttttattcat tagtgcttcc cttataatct ctgaatacca ttttattagt 33300

acatactgct attcttaata gtaactagca tgcctgatca tcccaaatgt ctaggttcac 33360

attttaaaat aagttatatc tttgggctta acagtttatt gaaaggtaac aaggattgag 33420

tcatagttgt atgtttttgg aagtagaatt caactgtaaa tagaaattgg ttgtttagat 33480
ctcactatat atgaaaaaat gaaggcttta ggagaaaatc tccccaaagt acccattttt 33540
catgtgataa atatcatgaa atgatttgag aaaaaaatgt atatttgtta cagctaacaa 33600
atatttgtgt tttttattct tcatggagag aatgaaattt cttctcttct ttacacattt 33660
ctttttctta ttagaaacta attggtgcct ttataaaaat taactgcaga gcactaacgt 33720
gtatatataa gtattatgta gggtgtaggg tatgttcagg gtatggtgtg tgtgtgtgtg 33780
tgtgtgtgtg tgtgtgtgtg tagctgtgtg tgtatataat gaaatatatg gtagtgttgt 33840
ttcagaaatc tgcttggtct tcccagagtt cattcatctt ataaattcat ctacattgat 33900
ctctattttt ggaatccatg aaatgttttt tggcagtact tcctttaata tagtgtgctg 33960
gaaatctgga aatttctagc cagattagtt acaaaaaatt agccagtggt tttgcactct 34020
ctatagaatc aaggcccaag gcctactctt gttactcagg gccttgtttt atctggcctc 34080
tttcttttca gccatatagc tctcaaatac tcaacaaaat tcttcattct aggtagacaa 34140
gtatcttcaa aatacttccc aattatctaa taactgtctt accactaaga aggcttttat 34200
gtctcctgtc tgaattttat ccatgcaaaa aagtccagcc caagcctcca gaactccaaa 34260
aagttatccc taactgctga aacacagtaa tttcactatg tgaaatttca ctttggtctc 34320
ctagcatttg cagatatacc atacatatcc ttgatccttt tcctttcata ccttttatat 34380
ctaaccctta agctaataat tttacctaca ctgtaattca aaatgtatcc ccagtcttac 34440
catgtctccc ttctctactg ttaccaccct aggctaggcc ttcatcattt ctcacctgga 34500
ctccttccct aacctctgaa ctgatctgcc tgcttccact tagacaccca acctagtcca 34560
ttcttgagca gtcggaataa ttcttttaag aaagaaacca gatcacatcc ccctctgctc 34620
ccaaccatcc agtgacctct tatcatacat agaatgaaat gcaaatcttt actgtgtttt 34680
aaaggcccta cattatctgg acctcagtaa cttcttactt cctatccctt ttctccttgt 34740
atgccaccct ccaactacac tctaactaca ctgtcttttt ccctgttctt cagacctgcc 34800
aaccatattt tcactgctca attaatatgt agaaaatgaa ttgtttgtta aatgtagact 34860
gtttccttct taaagcaaag ataaatgaca ttgtcttcaa aaacaactaa ctgcccagaa 34920
ttcctgattt taattttaaa aagacaaact gcaagaatgt gttaaacagt aaggaaacaa 34980
ttcactactt cagaattcta tatgatttca ctgcacgtta gtaattttgt atattataga 35040
atatgagggt attctaataa acttaactct atgctgtata cttatcatga tagctcattt 35100
tcttatatgt ttataacagc actacttatt gtacatggat acgtgggaaa taaattaatt 35160
ttctccttaa gaacaaagca accatttcac tcatgagata aatcttgaag atttaaaaac 35220
tacttataat taattataca ttattcatat aatgttaagt attttcttag taaaccacat 35280

aatttagaat ggcaattgga cagatgggca gaaccacatg catccactat taggcagttg 35340
gtgagcataa gatgccagaa agaagattag gaatatcaag gcagggagct tccgatcgct 35400
cttgaaaaca ttgacccttc actcctcact ctccacgatg catttccttt gaaaagtaat 35460
gccttccaaa acaaagttct ctgttttata tctaaactta ctcaatagtt tctcatggtt 35520
attgatatat aaaaaataaa gtaaaatgtt taggcagacc aaaagaagaa tttccccctc 35580
cctctgcctt ttatgccaag gtgacagcta tgaaatgtac agtacgtttc ctctgcaagg 35640
aatgtagcag tgttccattg caagaagatg agagggagag aaaggttgca cgctgaggaa 35700
tatagtgtca tttgtcactg cctagactca tcagctgtgt ggaactctga gaggcaccag 35760
gcttctttat ttatttcttc agaaacttca gcaaaaaaga tttcattagg agcagagaaa 35820
aatgtgaaaa acgaattagc ttttgtgatg gggagtagtc atctctgaat attgatcaag 35880
attaagaggg ttgtcttcgt aacttctttt atccatagtc tatactgatt taactagaaa 35940
actaatttca ggtggtattt cgggtgtggc agatctttat agtaaatgaa gaatctagtc 36000
aaatctactg aaaaactctg cttactttaa tgtttgatct ggttgaaacc attttagctt 36060
aacaatcctt cctctgaaac agggaatcaa ttgatatcct acagcaaaat tatgtggaag 36120
ggccattagc ttcacatcca atgcaaattt tgcctgtgtt tactcttccc caatccaaaa 36180
tatatcagat cctagatgcc agtgaaatcg tttgagctag atggcttgag ggtcatagct 36240
tttttcattt cctgttctca gacctcttat aattgataga ataaaatcag aagagcccta 36300
gagctgtccc acctattctg cctcacaaaa gtagaagtaa tggcaaccac tatcataggg 36360
atcatgctca cctttttctt accagacaaa tttggatatt agcttgaaat taataccttc 36420
cttaaaatgt tggaatttgg ttatatgcga aattttgctc tatttattca ttatattttg 36480
tatggaatta tttttgccct atattttcac ttaagtgttc tctacccaag attttaattg 36540
aacccaaatc agccagacac acagacatgg attttgctgc caccaaggtt aattcttctt 36600
ttaaagttaa cttttaaaat ttggtaaaat atagctttga aaatttgcat tcgtctagtg 36660
tttgttatgt atttcccccct tttgtttgat tatatgtcta tatttttctt gtagaaattg 36720
atttttaacc tgctttttat gttagctttt atgagcttct gtctgaattc tgaatatgtc 36780
tttcttaatg tcttctaaat gtttctttct ggattattaa aagatttatt aggcttttaa 36840
taattatatt tgttacctta gggaatgtgt ttgaaaatat tttaaatgga attgccagtt 36900
aacacagcat tgaacttttt cttgttagag atacattgtt ttctaggcat tttattggga 36960
gagaagttag tatgatataa tgtctttggc tgatattaac tcttctaaga tgcattgttt 37020
ctgagaacac cattgtctga tttcattcag ggaaatttca cacaagccag tagagtcaat 37080
actttttttca agacctgtta attgatatat ataaaaactt gccattgttt acatgcccat 37140
ttcagatcct ttatgtgacc taagctagaa atgcatttta acagcatttg tttttccaaa 37200

aatatttatt tatttattta ttatagagat agcgtctctc tatgttgccc aggctggcct 37260
cgaactcctg ggctcaagca attctcctgc ctcggcctcc caacagtgct gggatacagg 37320
tgtgagccat tgtgccaggc ccttgttttt atttttttg aacattgtat tttgaaaggg 37380
gtttgaaggt gatccctaga tagcaaccag taatgattcg agcagcaaaa caatctaaaa 37440
agtaatttta taagaaaatg cagaacataa atgagcccat aaaaaattat attaggttct 37500
atttacatta ctaccttctt tcacatgtaa tatttcacta acatttaatg aatttctgtg 37560
cagtgccata taccattatg aattctagga tagaagaatg agtgagaaat gttcttaggc 37620
cttaggaaga aggaacaagc atctctgtgt aatagttatt tcaactcttc ttttacacct 37680
cattcccata ttaaatctca gaaaagctaa agtaatagct atcccagatc tattttagac 37740
tccagacact tacttcaatg tcttgttctc cttatcagac tggaatcatt ccaaacctct 37800
taacttctgg gcaaccatga taatgcgaca gaaaggacac taaatctgtc gcaaatttat 37860
cttgatattc tatccagtct tacttggtac tgaaggtcac aagtaaaata aggtggttgt 37920
tttttgtttg tttttttttt tttgacagaa gagaaaagaa cactgtgagc acagagtgaa 37980
tgtctaacat tgattcttga gtagcaggaa ttctctatgc gagaggatct ctatgcaaaa 38040
agatctcata ttctagcaca atttaaggat ctctatgcaa agatatccca tattttagca 38100
ttatcaataa gctatggggt aatatattgt atgtggtgtg gcttgaattc tagaaatttg 38160
atttctagaa atggtccctg tagttaagga tatataatgt ggccgtctcc agttttctat 38220
gaggaatagg aaaatactat cattattagc tgtgtgacca tggacaactt gcttcgttct 38280
tcagttgcat catctgtata aaataagaat aagaaaattt acatctgcaa ggtgtgatgg 38340
agatcacatg ggataattgt ggtcccagag cctggcacaa aagggcttaa tatttataat 38400
cctccccatt tctccgtata ctctaaagga agtttattgc ttatcaaatt gtgccgtggt 38460
tagttgtaca gcttccctgc caaattgtaa actccaacac taatgtgacg ttacatttta 38520
tatagtgcta tgattttcaa attgtttgca taatttcaaa tacacagtaa attgcttttt 38580
attagtataa ttattgctat tgtcaatatt attattacaa cagcttcaca gtaagatggg 38640
cagaaaaaaa tttaatttcc attttacaaa tgcacttttg aggctcacag aagtcaaata 38700
gaccaaagtc acagggctag tgagggaccc agaagaaaca aattgtaatt cactgattcc 38760
aagttcagtg gttgccttac tgcatcataa aggctattac acaatccagg tgtatcatat 38820
gattcttgtc tatatattca tacatatcag aaaaagtgtt ctactcaaaa ttgctagcaa 38880
tcaacagata ctgatagtca ttagtactta aatctttatc aaatgaaata ttaataccca 38940
tgaaagagag gacaatgaaa ggtttgtatc atttgtatgt cacaagtcaa ctttttttcaa 39000
tcactcatta ttagtttaac tgtaaaaaat tatttacatt tagcgtgaaa ctttcctgta 39060

ttctcaacat atttccttcg gtagaaaagc aaacctccag ttctctgttc tttgcttgga 39120
tacttgccag tttgtaactc agctatcaaa cagtaaagct cacaaaacac ttattaaaat 39180
gactaaaatc caaaacacca agagcacagc atgctggtga gatgtggagc aacaagaact 39240
ttcattcatt cactaatgct ggcaatacaa aatggtacag taactttgga agataggttg 39300
acaatttctt acgaagctaa actatactta acatatatat ttgtccattt tcacagtgct 39360
aaaaagaagt tcccgagact gggaaattta taaaggaaag aggtttattt aattgactca 39420
cagctcagca tggctgagga ggcctcagaa agcttataat catggtggaa ggagaagggg 39480
aagcaaggca cctacttcac aaggtgacag gaaggagaat gaatgcagga ggaactacca 39540
aacacataaa accattagct ctcgtgagaa ctcactcgct atcatgagaa cagcatgggg 39600
gaaacagctc tcatgatcta gttacctcca cctggtctct cccttgacat gtggggatta 39660
tggggattat aattcaagat gagatttggg tggggacaca aagcctaacc atatcaccat 39720
atgatccaaa atcatgctac atgatattca cccaaaggaa atgtaaactg tgtccacacc 39780
aaaacctgca catgcacgtt tatagcagct ttattcataa ttgccaaaac ttggaagcaa 39840
ccaagatgtt cctcaatagg tgaatgaaca aaaagactgg cacatgtact caatggaata 39900
ttattcagtg ataaaaagaa atgagctatc aagccacaaa aacacatgga gaaaacttag 39960
gtacgtaagc cagtttgaaa ggttgcattc tatatgattc caatatatga cattctgaaa 40020
gagacaaaat tctggagaca gtaaaaagat cagtgattgc ctggggctct gagaaagtgc 40080
agagggatga atgggtgaag cacatggcat gtttaggaca gtgaaactat tctctatgat 40140
actgtcatgg tggatacatg accttatacc tttgttaaaa ctcagaattt tacaatacag 40200
agtgaattct aatataaact atggacttta gttgtaataa ggtatcaatg ttatttcata 40260
agttttaata atgtaccaca ctaatgcaaa attataataa taggggaatt gggggaaggg 40320
taatggagta tatgggaatg cactgtaatc tcagtacaat tattccacaa acctaaaact 40380
tctttcaaaa atacaagcta ttggtcaggt gtgatggctt ataccagtaa tctcagcact 40440
ttgggaagtc aagaccctca gatcacttga ggccaggagt tcgagaccag cctggccaac 40500
atggtgaaat cctgtctcta ctaaaaatac aaaaaaaaaa aaagaaagaa agaaaagaaa 40560
gaaagaacag aagaaataaa agaaagaaag gaaagaaaga aagaagaaaa gaaagaaaga 40620
gaaagagaga aagaaagaag gaaagaaaga aacagaaaga gagaaagaaa gaaagaaaaa 40680
gaaagaaaga aagaaagaaa gaaaagaaag acagatgcgg ttgctcatgc ttgtaatcac 40740
aactactcgg gagactgagg catgagaatc gcctgaactc agaaggtgga ggttgcagta 40800
gggtgagatt acgccactgc actccagcct gggtgacaga gcaaggctct gtctcaaaaa 40860
aaaaaaaaaa aagctattaa aaatatgtaa agctcagtct agatacagta ccagaatagt 40920
aggaacttta tttcacctgt cctacaaatt atggttgtgt gccacttggg taaaactcag 40980

aatccaaata tgtgaatgta agatttatgg ggaaattatt tgtatttcaa aataatcctt 41040

aatgaatgca ctccttctaa agtagccatt aataaagcag ttaatgtttc atttaattat 41100

agattaatgt acataagata tgccaggaat gcaattagga actgggaagg gggtgttatt 41160

ctaataactt ccacatagca ttgtgagaca ttttctgctt tcttcaaatt tcatttaatt 41220

acattttaaa caaatatttt tgtgagccta ttatatagtc cttcgctagc actgaggaga 41280

catgctttgt gaccttggtg atttcacatt caaatttccc tttcacctac actcttcctt 41340

gtttttttcat gcctgtgtag attgtaaatt cttcctcaga ttaagacatt ttattcacct 41400

ttgtaacatc cacagtatct agcacaatca gtgccttcaa aaacaattgg cctcaagaat 41460

tgattgactc aatgagtgac tgaaagacta aattaataag tacacatcta tttgtacttc 41520

cctgcttact tataaggtat gacaatgaaa tactgagaca gttatacatt acttacggac 41580

tcaatctcat ttctttacaa tctctattct tcttttttga gtataatgtt attttacaat 41640

tccactaact tgtcactctt tattataaat tcatatctcc atttcacctg agaataataa 41700

aggcaaggaa gtattttaaa tgatcttgtt ttttataact agcattcatt gagcaaatca 41760

aagtatgaaa ataatatagg tgtcagtgat tattataaag ttgtatgcac aaaacattcc 41820

aatgattggg gccaatacag agaaaacatc tcaatatttg gaattttgct tttctgtaaa 41880

tactttgata tgtacttaca tcatatcaat tataactcct gctgaaaaca aacagtgcac 41940

acaaatttgg tagttggagg agactttata aagggactaa ttacgaaggt ttagaccggg 42000

ttaggaaaaa cacacggaat agtgcaatac tttaggatgg caacagcgag caccgttata 42060

accactaggc caaaatgaac taaatgaaca gggagattac catttatcag aaaaagaggg 42120

agaaaggaag gagagatgac caagcaagtc ctatgtgaag acggctgcct gacttgagct 42180

gtgtgatctt tggactgata ccacctgcct gcactggcct agcagggcga gaatagtcaa 42240

tatctggaaa atggatcacc tgaccttact ttcctccctc cctgtttcct ctttgtggtg 42300

tttccactgg ccaaactcac agcgtagaca aaaggagtgc attgatgtag cagtggttct 42360

aatccagggc caattgtgct cccagggaac attagtggtt atcacagctc aggggaggaa 42420

gggagaggag tggagtgcta ctatgattca ctgagggatt tttttaaaca tctacaatgc 42480

acaggacatc cttccacaac aaagtatcca gttaaaaaat gtcattactg ccaaggttga 42540

aaaaccgtgg tgtagtcagt acaattcatc ttctccaggc acagtgcagg agtggggtgg 42600

agtgtctgaa ggggaagaag gaagaaacca gcacacccca caaaagtaac caatgcaaat 42660

accaaatagg aaaagacagc acttaaaata caaaagtctc aggaatatat ctgatagtgt 42720

tttatggaat ttattaaaat ttagcctgga gtgagtaata tttagcaagc caggtttgtc 42780

tttagagaaa tccttgtggg gtttatacaa ggatttatta acaaagggca cacacaatac 42840

tcatattaca gtcagtctgg ttatgtaaaa catgggcaag aatgtaatag gacaatgtga 42900
tgtattcaca aaggatttta ggactacaca gataatcctc taatgctttc acttacgtac 42960
tatgaaaggc tatagtttgc atagtgatat agccacgtaa gatagtaaac ttgacattca 43020
tgcagctata catgtttgca cacaccagga tgcatgccct ttctacctgg ttgatttttt 43080
attcttttat taatctctaa tttattcccc agaacactct ccataaaaac tttctcacaa 43140
cttaaatctt taatctattg tgtggatttc tgactcattc tccaagcttt tcctcttccc 43200
tccgcaatgc cttatagtct tatgactatt tatccctttg cctacatttc tagccagatc 43260
tcttgcctga tacacactct catatttctc tttgcacgct acacattttt atttagatat 43320
cacactacta ctttgatttc aacaggtctc agtttaactt aatttttcct tcaagcaagg 43380
agtcccttca tatcagttat caccattggc accagaattt ttcttatgac ttcccatgac 43440
ctacaatata aaccatataa atcactgatg cctccatagt tccctccctc tcaaatttag 43500
ccataagatg attttaggat ccttgttttt tccaatctct ctttcattct ctcccccatc 43560
tcttccatta tgaaggtttg gataggacac aactcatgcc tagattagtg caatagatgc 43620
tgagcctgtg cagcggtagt ttagctttct ctcctggtta actttaactg ccacatatat 43680
cacttcacac gtcatttttc attcaaacgt atttaactgg ctcttcattc ataagaagct 43740
ggaatttgtc gtttgactga tattttaaag attttatatt ttttctccat cctcgttcta 43800
atgttgtatc ttgtgtcatt tgttcattca taaacttaag acttagctaa ccactgagca 43860
tccaggaaat tcagtatcta tcatgtgaat tctctaatac tggttgatcc attgtcacca 43920
gagcatagca ggcttctcct gcctttatgt atgtttgtca tatagttcat gcctaaaatt 43980
ctttcttaaa tcttaaattc ctaagataca cacttttgcc caagatcaca gtaatctctg 44040
ccataatctc tgctggaatc tgttcactgt gttgctcctg ctaaacttct tacagatgac 44100
tttttttctt tttggtttcc ctggtatcta gtataatttc ttatataggt actcaataaa 44160
tgtttcctgt tgatctctac acctactctg tacaatacca tagtgactag acacatgttg 44220
ctatcaagca tttcaaaagt agctagcctg agttgagata taggggtaaa atacacaaca 44280
gatttcaaga catattatga aaaaaaccca taaaatttct cagtaatttt tttatagatt 44340
acatgtagaa actataacat tttgaataag ttgtatcaaa taaaatataa aattcacccg 44400
gttcttttta atttgttaaa tgtggtggct agaaaattta aaattacata attggctcac 44460
agaataatta taatggatgg tattgcttta gatcaagttt gtctaacccg tggcccatgg 44520
gccacaagcg gcccaggatg gttttgaatg agatccaaca caaatgtgtg aacttcctta 44580
aaacattatg aattttttgt ttgttttgtt tttgtttttt tctcatcagc tatcatgagt 44640
gttagtgtat tttatgcatg gctcaagaca attaattctt cttcaaatat ggcccaggga 44700
agccaaaaga ctggacaacc ctgctttaga tagtaaagca tatgagtagt taatgtgtac 44760

tataagcagt gtgatctgat agactattta atgttgtttg atggtacatt attcaagtcg 44820
attattatgt ctacctatgc agtttaacga cggtaatgag agagggcagc ttgattacag 44880
gtcttatctt ttgactaact tgctaggcca cctgagaagg acccaaatta tctgaatgct 44940
taactcaact aatttgtatt cacttgaaga atttcaagga tgtttatatg ccatcaactt 45000
gctttaaatt ttttctctca gtgaaaattt ttcttaaaat gagtatgtgg tattcaaatt 45060
tatccttgtt ttctatgatt atcttttcat agcactgtgg tttccaggaa cctttttttt 45120
tttgagatgc attctacatg taactattgc acagtttgca tgtagtaagg ttcattattc 45180
ttctactttt ccaaacacct ggcatgttta cttgaggttg gtacaccttg tatcccagat 45240
tttgctgttt ttaacttaaa tattgaatat tttgattaaa cattatggaa agtttaaatg 45300
ggtcaagaaa aatagctttt cttcccatga agaacaatac ggcataggag ttaagagcat 45360
agatttaaag tcagaaaacc tgtgctgcct acttgtgcaa agtcacttac atgctgtact 45420
tctgtttctt catctgtaag ttctacccct aggtatttac ttaagattaa tggaagcata 45480
tgttcataca atgacttgta cagaattatt cacgatagca ttactcttaa tagctctaac 45540
tggtaacaac acaataatca atcaacaatt gtgctgtatt catacagcag aatactactt 45600
agcaacaaaa atggaatgga ctactgataa cctcaacaac atggatgaat ctcaaaacta 45660
tcatgctgtg tgatgccagg cacaaatcag tacatactat aattccagaa aagacaaatg 45720
tcatccatgg taacaacaag atccatgctt gctggaggta gaggcatcag ttcagtcatt 45780
caggaagctg attccaagat ggtgttagaa ttacaaccat ccacaagaga tttattgcag 45840
gcaatagcta tgaaaggtag aaagagaaca ggagaaaaac caggcaagga aaaaccacaa 45900
tgtagttgtg atatcacttc aaagggaggc agaaggaagg agaattgggt aggaatagcc 45960
acagattaca gtgcagttac aagaaagtct tggcttccaa caaaggttac ttgttgagga 46020
gtcatgcatt aggcagacat gtctgggctg tagtttcctt gctgctccca gtcattggct 46080
ggaggccagt ctgggttcct gtgctgtggt ggatcccatt gctgctgcag caggaggcca 46140
atagcactcc tggcagctaa ttggagagaa aagatccaag aggtgtacct tcatggctac 46200
ccccatgggg ctggggtgga ggtggaggag aaggagaagg aattaactag aaaaaggcac 46260
aaaggaaaat tggggaaaat aatgaagata tatgatttct caattgtggt ggtcgttaca 46320
tgggtttatt aatgcatcaa aactcaagaa atgtacattt aaaatgagtg catatgattg 46380
taagtgaatt atacctcaat atagttaatt ttttaaaaat catagatttc tttatattta 46440
atgcatgaac ataaacctaa gacactcctc cactccaaaa cttaattacc ttgtgatcag 46500
cagagcagaa ggtactttgt gatatatagg tagagaagat gaagtcttgt gacatttaac 46560
aagggacagg aaaatggacc ttgtcctaag ttaccaaact gcaaaaatat cacctacaaa 46620

ggctattcat aacatacatt ttcaaggggg ttacaatatt tgcctactat aaaattttgg 46680

atctgtaaag gggttaaatt atttgtgcag gggaataaac atcaaagaaa cattaagagg 46740

tccagagaag taaaatagga agggtctttt ggctagagga gatatttaac tttcagaaca 46800

tgtggaatta agttgtattg attatgatct gatcttcttc cccctaaatt tgatcctctt 46860

cctgtaatct attgtttcca tcatcttcaa ctcttccctt tccctctccc ttgtccctca 46920

gttctagtca atcacaaagt cctacagttt cactttctgt ataccttatt tctggaattc 46980

atctctagac ttcaaaatat atatatatat attttttttt ttgagatgga gtctcgctct 47040

gttgcccagg ctggagtgcc gtggtgcaat ctcagctcac agcagcctct gccacccagg 47100

ttcaagcgat tctcctagtt cagcctcctg agtagctggg attacaggca tctgccacca 47160

cgcctggtta atttttgtat tttcagtaga gatggggttt cgccatgttg gccaggctga 47220

tctcgaactc ctgacctcag gtgatccacc cgcgtcagcc tcccaaagtg ctggaattac 47280

aggtgtgagc cactgcttcc agcccaaaat atcttaagta gataattgca cgactaatct 47340

ctgcttttct ctcccagcag ccttccaaat tcatgtctca cagctgacag agttgttcct 47400

gccttcagat tcatgacctg gctctgtgtt ctagctcagg ctttctctct catatcacct 47460

cttgcctctc tgttgccccc atattttccc ctctggttgg ttggtgctcc tttggaaccc 47520

tctgcatatc ttttcaagaa tattatgact tattatgcct ataaactttg tttaattatt 47580

tatttctaaa atttgacagg gaactttccg aaggcaggta ttgtgtcttt ctcatttaaa 47640

agcaaattct cgcctggcat ggtggctcat gcctgtaatc ccacactttg ggaggctaag 47700

gtggacagat cacttgagcc taggagttca tgaccagcct gggcaacaca gttagaccaa 47760

aaaaaaaata tatacgaaaa ttagcctggc atggtggcac acccccgtag tctcagctag 47820

tctggtagct gaggtgagag gatcacttga gcctggatgg ttgaggttgc agtgagctgt 47880

gattgtatca ctgcactcca gcctgggcaa aaaagtaaga tcctgtctca aaaaaaaaaa 47940

aaaaaaaaat tagtgaatcc tcagtgttta aaaagtccat aaacatacta aacatagaag 48000

acctccaaat gaaattaatc aattattatt tagtgggttg cttctctttt gttttaatat 48060

agttttaaca aagagtaaaa gttatgatct ttttatatgt aaaataaata atgccgggtt 48120

tgacataaat tttaggaaaa ctagagacgc tacttcctaa aaattttctt tctataatct 48180

tcctaaatat ttttccataa agtacaaaat aatagaaaaa aattaagaga ttgagtatcc 48240

tttcaggaag tgatatgaca aatagggttc gagaactatt tgaattctca ccacttttca 48300

taagggcaga tctcaagtta aatttttcta ttcgaattta aatgactttc actggaatac 48360

cattacagaa aagcttctgt gtttagatgg caatatggag tttcttttct tggaatatta 48420

attgaaggag aagtcttaat tttttaagtc tatatctccg tatatatttg aacctatttt 48480

atatgttagt ccttctcttt agtaaccttc atccacagtg aacaagattt acccttacct 48540

ttaagcagta gcggctactt tatgtgaagt gaacagctgc tttttttatc tgcatctaga 48600

catcaagtag tccagagtcc tttctaacac cctagcaata gaagtaagaa tattttgacc 48660

attccatgac ttgatgatac ttctagtaat aatactgtat tattaaaaac aaacaaacct 48720

ttgtgcagtg gtaattgaag cagttccttg ggaacatgta ttaagtactt tttagcagtt 48780

aagtccactc tctgtaggtt aaggaatatt taaataaaat aatgtggcaa atgagttcaa 48840

gatgataaat gcgatgagaa ctaaaacagc tttaatttta tgtgggaaat aaatagagga 48900

aaagtacatt acagggctcc tggacttatt tctttcttca aagtgtttct cctagcgaat 48960

attattacta ttttttctct taagtaaaaa atacacaaag tatgaatcta cacaggataa 49020

taatattgaa gttaaggatg atgtctcctc cttcactctc caaaatacta tttacttggc 49080

ttcatggaaa tctctctcac tccaattcca ccgtgtcaac tgaggtcttc tgttctttct 49140

ctccctatag catattcctg ttacataaat cctaaactgt gtcgtgttag tcacacactg 49200

taacctctag ataagcgcct gtccagaggt tctcaatcag agccttgcaa atatgtatta 49260

aatcaatggg tcatcttcag tgtctcagtg ggcccttgga tatgttttgc agactgctgt 49320

gagtatgtag ggatgtccag tatcgaggga agtgtggatg gctttcattg gttcttatag 49380

ggctgaagaa cacatagagc agtaagcact tctactgtag ggagagatcg agcttctccc 49440

atccccactg ctggcaccac caccacccta caccccattt tgagttctga aagtgaatcc 49500

ttgagaaaga acacacaaaa caaccatcat aatagtgggc acagctgtgg gtggtagaat 49560

aacattccca agcttctttt cctacacatg attaatatta attcagcaaa catttattca 49620

gctcctactt ttaaacaggc actattctag gtactaaaga catagaggca aagcatacaa 49680

gactctgcct ttgtgaaaca attaagaaat aagtaaaaag aaaagaaaca gaaaaggcaa 49740

tttggatagt gtcaggtgct ataaagaaaa caaaatgcca ttttaataaa taataataat 49800

acaatgtttt catactatgt gctagacact atgctagtag gtatttatag acataacctc 49860

aattaatcct caaaatggca tgttgatatc aataccccaa gtttacatat gagacttaag 49920

atgtctgagt atattccccc aggtaacaat taatatgcac aataaaactt tttgctcatt 49980

catttattaa cctatgttga ttgagtacct attttgtgtc aggcatcatt ttaaggcacc 50040

tggatatagt tatgaacaaa caaataaaaa tctctgccct caaataatta atatctcaca 50100

gaggttaggc aaaatataat cagaaaataa gtataacgta taggatgcca gatcatgaaa 50160

gaagctatga atggcatcaa gaagctggaa aaggcaagga gacagatttt ctcctagagt 50220

ctccaaaaca gaacacagtc ctgccgacac cttaacttta ggctagtgag acccctattg 50280

gacttcagac ttacaatccc acaatgtaat aaatttgtgg taattcagta ggggaacaat 50340

agaaaactaa tacgatatca aaacaaatta tatcatagaa caagaaaatg taattgtgac 50400

aaataatacc tacaaaaatg ttgtaaatgc taggcaaata atgtgtttaa agcacttagg 50460
ccaatgttca acgtaaagta attcatgcta taatatcatc atcatcatta ccaatattta 50520
ggggctctaa caaatgatgt acgtgtaagc agatgtaaga aaatttcctt gctgaagagg 50580
aggtattaat agagtatata acaatagata acaaattcca aataaaggca aactaaatgt 50640
tttattggat taaatttaat tttaaaaact acaagaggcc gggcgcggtg gctcacgcct 50700
ataatcccag cactttggaa ggctgaggtg ggtggatcac gaggtcagga gatcgagacc 50760
atcctggcca acatggtgaa acgctgtctc tactaaaaat acaaaaatta gctgggcctg 50820
gtggcgcgtg cctgtaatct cagctatttg ggaggctgag gcaagagaat cacttgaaca 50880
accaaggagt cggaggttgc agtgagccaa gattgtgcca ctgcactcca gcctggcaac 50940
agagtgagat cccgtctcaa caacaacaac aacaacaaca acaacaacaa caacaacaac 51000
aacaaaactg tgagatccat ggtgggcttt taagaggaaa atgcaagcta aggtttgttt 51060
agactctgag tactgcatgt gtaaaaataa aggcatgatg aaaagatcaa gagattagag 51120
tgatactttt tatctactag tgtcagagtc atgaccaggg gattggctat gagaatacat 51180
aagctgtgcc aggagtaatc caaggagatt gtttcaattt ggaagagtgt ccacagaatg 51240
attctcatac tagacgttgg gctattgtaa agaaagttgg taggtactcc atcgctagga 51300
tcatatcagg gagaaattga acaggatggc cctaatgacc ctgttgtacc cctagcttat 51360
ggattaggca agtcacttct actcgtatac cctgtttccc catttgtaaa taagaggatg 51420
tgttactcta aggatctcta agattctttg cagttgttaa attgcatagc tctccactga 51480
ttccatggtg gaaatttgct attctattac aaatattcta aatgtatgag atatcagaca 51540
tactcattta aaaaacaaaa tacaaaaaat aagtattcta caaataaaca cagataatgt 51600
ttaaattcta tatgtctttg tttctcttca gaagcatcca aaatacaaac catctaagag 51660
gcaagaaaat gtcgtgatgt tcctagtgca agttaaaaag atttgctttc ctcaagtcgg 51720
aaagcccttc tcatttttga ggttttttc ttcttttttt tttcaagtga aagcattttg 51780
gaggagtcaa tatccatctt taaaggtagc caggtcacat gtatacatat gtaactaacc 51840
tgcacaatgt gcacatgtac cctaaaactt aaagtataat ttaaaaaaaa agaatttaaa 51900
taaaaaaaga aaatcagaga gaaaaaaaaa aagatgcatg tgcaccctga tactaccatc 51960
catagtgata cggtttggct ttgtgtcccc acccaaatct catcttgaat tgtaaccccc 52020
atgtgttgag ggagggacct tatgggaggt gattggatca tgggggtagt ttctccatgc 52080
tgttctcatg atagtgaatg agttctcata agatctaatg gtttaaaatc atggcacttc 52140
cttttgctct ctctttctcc tgccatgtga ggtgtgcctt gcttcccctt cccttctgc 52200
tatgattgta agtttcctga ggcctcctca gctatgcaga acggtgagtc aattaaactt 52260
ctttctttat aaaaaaaaaa aaaaaaaaaa aggtagccag gtaaaaatta cttgtttcca 52320

ggacattttc acctgaaaga agcattgtca tataacatag aagcaagaaa tccagtagtg 52380

ggggttattt aaaaatagct ggaaaatttc aatcagcatg agtttgaagc aacaatttat 52440

catcaccttt tatggtgggt ggggttaaga acatttcagc gggcaaagtg gtggtgatgg 52500

ggaagagaca ccaggggagg tgattcccat tgcattgctt tgtaaacaga ggcacaggtt 52560

cttcattttt gtcacacaaa atcacagcta tgcagaattt attaatttat tcttctgaga 52620

caagaaaaaa gccaccaaag gaaaccaaca gcttgctcct ctcacactgg gggaaccata 52680

tgagagactt atctatccct gactttaatt ttgacctgag gagagctcct cttaaggaaa 52740

acaaattaat tcaatgacta tactacttaa tcattgacct ttatttaata agagattttt 52800

ccataggata tgctgagctg tctcacttac atcagttgtg tctcctgagg tgggtgacag 52860

gagaccacaa atattgcata gcacacaaat cgttaatagc agctgtatac caaaccatta 52920

cctaaatatg tagagtacaa ttcattctca ctaatgtcag agagcatgct ataaaatggt 52980

gaatccggac agctgaagat actgaataat aacctctatt ttgaacaagt ttacagtgtt 53040

ccaatcagta attaaattga tacctgatga atatatgtgt gtgtatgtat tcatagcaga 53100

gatggttttc ctgagataag gattttgtta ttcggatagg ctgctgctgg aattgtcctt 53160

ctacccttgt ttctttgtcc ttagtcatca ctcatacctc tttccactct tctgccatca 53220

cttttgtcac caaagtcatg gtcctttccc cgccgattgc tgctgcaggt ctagggcacc 53280

aagacttagg cagcactcac catgtgccaa gaactggacc acaggtacca tccagcattg 53340

ctcatggaga ctctgtccct ttctgtagga caccctcctt ttagctagca acccctccac 53400

cacctagagc ctctggacct ctcattttaa tattaagaac taggaaaact taccgctgag 53460

aataactagt acaactagaa ctggtagaga aatctgggtc tcttgggaat ggatttttag 53520

gctttattga ttagaggtgt attaataatg cagtgttata gtttcatgac ataacgaata 53580

aaaaagttca ttttggactt gcctttcagc tccctaggag ctaaaagacg tatttaatgt 53640

aacttgtgtg gtggaaataa gttctttttt caggcaaaag atgtgcaaac ccatctgggg 53700

aagaaacatt aaaaactaag gagacagtgt cctagataac tatgttcttt tcctgtttta 53760

gtctaaaata atgattagtt ttcttatata tcttcatttg tcttggttcc ttttagccca 53820

atttaataat attattgcag atattgatga aaacctttac cttcctctta attcatcaaa 53880

gtacttgata aaatttatac atagtacatt aattgggagg tttttatgag attaattaat 53940

ataatgaact gatgttgaaa ttatttaaaa cctgaattat tattgtatta agtaggacac 54000

ttaatacagt taatcagttc tgtctttatt catttgtgag aatttttggc aagctattgt 54060

gaatattcag ggaagggaat gtatttttag caggaatctt atacctccta catagaaatg 54120

aagcatttac tgaaacatcc atgaaacaaa atgtttctga atgtgtacta tacacttgtt 54180

ataagcccct tttcttctgt agctatattt tggagaaaaa tctttgcttt gacaaaaaaa 54240
attatgttga cttacacata tattttataa ctaagcagtg tttggtttgt gataaaggat 54300
acaaaaatat aaaaatgttc agcacacgta agtaaggcct tgttgacagt gtgagttatg 54360
ctactggata ctcaaaagga acattcagtg ttctcaggtg gtctctagac tgtctcaagc 54420
ctaggaagat attttataag caaaggaata agagaaggaa gattcagatt taatccaagt 54480
gaagaattca gttttgtgtg ccttatcctg ttattttgag aggcagccaa aagatgctgg 54540
tcagcaagga gaattgtaag ttgggcagcc aactctgatt tctcaacctc ttagctgttt 54600
tcttaaactc agaattttta atgaatttaa atgtccatat caggtagact ttggggatgc 54660
ttttaccagt gattttcaga atgttacttt ctggcatttc ttttcacgta gcattatatt 54720
aaaaatgaat tcattcatcc accttccctt gtccttacta attttccctc ctactccctt 54780
ccccettgtt cttgccatgg ggacatgcaa acactggtgg ttgatgtctg agcaaggctg 54840
ctgacagggg gaggaaggag atgtcaagca gaggtcaatg gcagtgtgcc cagcagccta 54900
ggaagtagga gggaaaagag agagagacag agatggtgga tgaaagagaa agccaggatg 54960
attatggtgg ttatgatact tgtcatgctg aacacccaat tgagcaccca ataagcacat 55020
aataatttaa tcatcctctg gcttggatgg cagtgttcta tcagtgttga cttcctggtt 55080
gtgacagttt tacagtgtta gtgtagaaga gaatccttgc tttagagagg tacttactga 55140
agtacttagg gttaatgcac cattgtgctg gaaaaagata cgcacacaca cgcacacaca 55200
cacacacaca cactctcaca cacacgcaca aatacatcca tgtgttaggc agagggagca 55260
aatgaggtaa aatgttaaca attaggaatt ctgggtgaag tggatagagg gactctttga 55320
ctgttcttga aacttctcta tacatttgat ctgtttcaaa ttcttcagaa aatcaaacta 55380
caaaaactta attcatttag tgaacatcta ctgaacatct gtatattaaa tagtgttaaa 55440
tgaatgtcaa ttaaaatgct caaacacagt agaggttgat tctcattcac ataagtccat 55500
ggtaggtgtt tttggcaggt gggtgagttt ctcccttagg gagattgagg aacccagact 55560
cctcccaagt tgcagcccca ccgtcttctg aggggatgca tccataccca cttcgaagta 55620
gcatacatta tttcctttct cattcctttg gataccagcc acaatttatt caaggtagac 55680
agaaaattgt agtatatagc catatgccct gacaaagaag ggagaacaga ttttggtgga 55740
caactagcaa actctgatac aatctgttat taagcactgt gtgtggatag atgctaacta 55800
gaaggagatt atcttccctt cagcaaatat aaactgaatg ccgtttattt ggttgaaact 55860
aagctagatc atgggagtat agaaatttta taagaagaca tagtcacttc tgtcagtgag 55920
ctcaagaaga attagtatgc ggaatgtaat catacctaca gggggcttgt gccacttaag 55980
taaaatgaaa cattattttg agtacaattt agcaataaat gtactacgag atcattaaaa 56040
atcatgtttg aatgttattg tgtcaaggat gggaaaaaga cttttgggtt gtagacttga 56100

taattatagt taaaaacagt ttttattctt gtttagtctt attttttatg tttaaacata 56160
tttatacttg ctaacattta tacttgctaa gtaaagactg tttttacaac catgacaaga 56220
acaaaacata ttagtaatgc aaatgccaca tttcctacaa tcaactaatc acactaacat 56280
atttgcatgg aagaatcact gggattgatc tggccacgtg tgtagtcatg cccaaaatgt 56340
gaagtccatc tgttttgcaa ttttttttaa ccactgttat ccaaatgctc cttggatttt 56400
ttttattagt ggatatattt tggaggtcag acaccctctt ggctagatca tcacctttat 56460
aacaaatata tatactattc tcatggaaat atatttagac attgccctac tgggaatttt 56520
tttcaagtaa ttaatgtaca gcttgtgcaa cagcttgatc ttggcttcat ggaaataatt 56580
cactcttagc agcatctaat gccacaaagc atttatggat gtcagctcag aacttacttt 56640
tatttatctc tgagttactt tttttttttt ttttttgaga cagagtctca ctctgtcttt 56700
ggcttgtccc taacctctta acagacttaa tattaagctc catttcactc agtcgttctg 56760
ttgtcatata aatgagacat tctacaagca tagtttttag tttctgccag agcatcatac 56820
aacattgtga gctatgatga agataaagac ctagagaaga tatttaatat gaagttcatt 56880
atctaatatt tggtatgtgt ggcaaaatag caatctactg cttggttctg ctgtaatcta 56940
tttacccacc catcccatct ttctttcaat ttaaaaggat aatgatttta gtcacgatta 57000
tacataaacc cattaccata ggcaataaac aatggggcaa accattggtc ccatagttgg 57060
agtgtggtct gaagtgtgtt ttggtggaga gagatctatg tctggagata gctaacatgg 57120
atttggatcc cagatctgct cctacctgtt gctgtgcctg tgaccaaatc atgtgatctc 57180
tctggtttca gtttacttgt gaataaagta aataccttca tcaacacctg tttttgaata 57240
caatgttttt ctgtaatttt tgcttcttat aatgttataa tgatcatcct tacatctaaa 57300
tcttggttta cattttcatc aattcttttg gaaagattgg agaagtaaat tttggagatg 57360
tatgtcggct attaaaaatg tttaattttt taattaaaaa ttaaaacgtt gaaaaatcct 57420
gatgcaaaat aaatgcatta tgcttagtga actcttctca tttcgaagtt tattcacctt 57480
cttgtttttg caagtttcct gaaaaatgca tataaagtca ctaagttagc agaactttat 57540
aaaattatat aactatatat aatcttttga tatcagtgaa gccagctgat cctatagaaa 57600
taatgtagga attataatca ctagcacata atttaagagt cctgtggtct tattcatgtt 57660
atttaccctc tctgaatctt acatatagta agagggttat tatacataat atgtgtacat 57720
gtatacaggt aagtaagtat atatgcttat gtgtaaaagc agagttattg tgagagtcaa 57780
atggaaatgt gaaagtactt tgtagttttt tattactatt attaattttt aataaaatgg 57840
taacattcat ttaataatca ttagttttaa cttcagattg tactggattt cctctagtat 57900
ttcttaagat tagtgaataa agtatttctc ctaataaata tattgactac tgtctttcga 57960

tcaaacatat taggtatatt tttacagtag catcaggcag tgaaaatttg aagctcttta 58020
tagaggactg atttatgatg aaaaggaata acatgaacaa atggaattat atgaagcttc 58080
cccagaaata tctaagaggg gccaatttta agaaatatct gacttctttt tcatggacat 58140
ttcaaaataa acctaactca tatggtacag tttttaagag ggaaaagaaa aaaccatctg 58200
agaatctctg gaattctgcc gaaagtatca cttggcattt tattctacct tctggatgca 58260
gttgattgac agtagtgtta tgatgccagg ggtatagtga ctagaaaaag aaaaccaggg 58320
aattcagtgt tcttgctcat gaagaacagc ttggttcttt aaaaacaatg agattttgcc 58380
accccatctc acaaacctat gatttgtgag aacaatccct tttgtgttgc aagactttta 58440
catttctctt cccacactat attagaagaa taaacattgc ttcataagta ccgattgata 58500
gtctcatttc atatttttaa aatagagtta ctttaaggtt aaatttttca tgtagattaa 58560
aatgactaag taaccattca catatttcaa ataaaatata tttttactac aaaaggaaaa 58620
taactagatt cttaagtgtt atagtcaagt gtaattgagt aatatgaatt ctaaatgaat 58680
ttctaagatc tgctcagctt tcactacttt aggaaggaac aacttaagaa aaattttaat 58740
aaagatatct cttcacacac atggcagtgt tgtacttaga gaacatgacc caaaattttt 58800
tatgactgca tattgaattc ctgatactct tgggaagctc caaaagcacc agtggagttt 58860
ccagatgtaa ctgtggctgc agacccgcca gtcccggtgt tggaagggat cattataggc 58920
tcttgtgtgc agactcatct tcagacccag aggaattaaa taacttgccc aaagtcgcac 58980
aactttctca tggtaggttg ggcactagaa taaatattgc tttttcttaa gagttttagc 59040
ctccgtatta tgaaatcttc tatgttctgc tgatgatatc tcctttcttc atctgttttc 59100
tatttttaag caatggaaat acaaacttgc aactccccat ttccaacaca acttagaaaa 59160
aacaatattt aaagaaaaaa ttacaggcat ctcatctcct ttacctgaca gatgcttgat 59220
agtaatggcc tctagatagg gatgacatct aatataaatg tgtcctttca agtcaagctt 59280
tctctgttca ttagtagaaa tattgtatat caagtgtgca aaaatttctt tcaacaggga 59340
gctttgtttc cctcctttta ttataacaat ctgagctttg tggtcccagg gtctcctagt 59400
gcctgtcttt aggtctgttt attcacatga agaaagcatg tcatatagta ttatctaaga 59460
ctcaggctgc ttatgcatga tgacagaagg gttcccaggc acaaacattc atccatgcat 59520
tcatccatcc acctattcat ccattgattt ggctgataat tattgactac tgttgagttg 59580
ccctcagatt tagtttctgt ccttctgcca tggggaaata tggggttaag ccacaacata 59640
ctcttctctt ctttttctgc accttcttag tatatttagt tccattttgt ctagccctgc 59700
ctctgacttc tttgttgtac ttcaggtttt ttatcattga aagttatttc tggatcatag 59760
atcattctct tggtcacttt gcttgttcac ttataaaatt aattcagaaa aaatgaccca 59820
cagtaattac cgtaaatcac agaccataaa ctataatact gtatattgta ttatagtaca 59880

gaaatattta tactttaaaa tgttttaaat atagatatta taaaaagata tgtctcatat 59940

aagtaatata aatacttttt tattacctct tctctcccta ttctccaggc cagtgtttta 60000

aaaatccatc tttatatgtc catcctggaa aaaactcatg atcataaatg agtttctcaa 60060

tagagtttat aagcccacag ttgaaacaca attgtcttag catccattta gttgtcatac 60120

ttttaagatt taatggcaaa tattatgttt tgtttcttca aaagaaatat tttaaaattt 60180

tagtaaaggc agttagagaa ggtagagata atggactgtt taatcctact tttcatccca 60240

caagtgaaca aaaaaatgat aaaacatttt tcccaaaatg tagctttaac tatacttaaa 60300

tttggactaa aatgggagat atcttttcta ctattgaaaa gccgtgtctg tagattaatg 60360

ctaaaatcgg gtgtaaaagc aaaatttgtt tggcttgatt gccaatggcc cattcatttg 60420

gctacagaaa caatagcaca tagcaacaga taatgatgtg agatcaccta gctcaagtaa 60480

gagtgtctga tccgtcaaaa atatatacat caagattcaa aagaaatgtg tgttttctca 60540

agtcatctct gtaaaaatac attaaataga ggaatagaag tttgactttg aaaatacatt 60600

gcagacccaa tccgtctttc ctattttctg gtgaaaagta tcaaatatgt ggaacctgga 60660

actgctattc tccttcttaa aaatctttct taatattcta ttgataactg gtgcaagcct 60720

aactttttgt cttacccgat tcttctcaca ccaaagtgat aggaccttca ggtagccttt 60780

ggatagaaga taaataataa tttaactatt gatggaagtt agtattagaa ttagacttgg 60840

aagtctatgg aataaaatga ttctacaaca atttgtactt cagacattag tataacaaaa 60900

catgtttgcc cgtgcatgcg gaaacaacca atttcatgtg gatgcttata ttcacaaagg 60960

agtaaccacc tggggtttcc cactgttgct ccagagaaaa ctagcagcag gagaacttct 61020

ctgaaggtat caagacatct ttaaaaaaca cttgttaagt gttggttcag ctaaagcagg 61080

gagttttcag ttagtaatgg cttttaaaaa ttaaaacaag tttagcatgt aggtcattaa 61140

ccttgaatca ctgtcatgat tattattaac catctgttct caaatcgaaa gatatttttc 61200

ttttctagat cacatttatt ctcacattgc tcaatttcac tatatatcaa gacatgaaaa 61260

ctgtaaaaat cacaccttct acattattat ttttattgaa aaattcctaa tgaaacagtg 61320

cgctctggga tagagaaagg aactaactga cattttgctt cttaacttgt ttttatgcaa 61380

gttctaagtg gtttctggcc atgtacataa aagacaaata tctggaaaaa aaactagcag 61440

aagtcagtta tttggctcta tctactttga gaattatgtt atataaatgt taggaaattt 61500

tttgtaatat tcttatttag aaatgaaata taaaaagttt taaaaatatc taaggacagt 61560

atacagtcct aaagtaaagc tgttaggtaa atgctacaca atcctcttat tacagagtca 61620

cttacctgag aatataagaa gagggcctct tgtttaagag taaatgtgag ctgcaatcag 61680

gattctgcac tcatttggac acttagtttt gtttttccat gactggtgtt gcctgttact 61740

gagacaccta cctgtcatgt gaccacagct tatgttacaa tgtgtctagt cagacttaga 61800
gatgtgtgaa agagcagtac ctagacggga aactatgggt ctataaaggt tttgccttct 61860
tgggcggagt tcaaactagg aagccacaaa acttccagtt gcattttcac agattaatga 61920
aatatatttt acacttttcc tgaaagatat tttatttgtg caaaccttgt tacaaagtac 61980
agccagttga ttaatcgatg aagtgatttg tagtggattc ttatattttg tgtaagggta 62040
tatgtgaggc cctatatatg aggctttcta tataatgaag tataattcag ttcagcattt 62100
caattcagca atcacttatt gggcctctac tcagttgcct tcagggcttt ataatttaat 62160
tgataaaggg aggttaatta attaattata acaacagatc gcttaatagt gtaactacta 62220
atttaattaa tgacaaataa caatacatta aaagaaatgc attaataaaa ataatatatt 62280
ggtgttatag acaataattt tctgattaac tttattatta ttatttcaat agcttttggg 62340
gagcaggtgg tttttggtta tatggagaag ttgtttaggt atgatttctg agattttggt 62400
acactcataa cctgagcagc atacactgca cccaatgtgt agtctttcat tcctcacctt 62460
cctcccaccc ttcccctcaa gtctccagag tccattatat cattcttatg cctttgcatc 62520
ctttagttta ggtggcagtt ataaatgaga acatgtaatg tttggttttc cactcctgag 62580
ttacttcact tagaataatg gtctccaact ctatctacgt agctacaaat gccattattt 62640
tgttcctttt tatggctgag tagtattcca tagcatccac acacaccccc ctatgcttta 62700
tatatatatg taaatatatc acattttctt tatccactca ttggttgatg ggtatttagg 62760
ctggttccat atttttgcaa ttgtgaattg tgcagctata aacatgcatg tgcaagtgtc 62820
tttttcatat aatgacttct tttcctctgg gtagatacct aggagtggga tcgctggaac 62880
aaatgattgt tctactttta gttctttaag gaatctccat aacttttcca tggtggttgt 62940
actagtttac attcctacca gcagtgtaaa aaaatgttcc ctttttacca cttccatgcc 63000
aacgtttatt tttttatttt ttaattatgg caattcttgc aggagtaagg tggtatcaca 63060
ttgtggtttt gatttgcatt tccctggtca ttaaagatgt tgagcatttt ttcatatgtt 63120
tgttggctgt ttgtctatct tcttttgaga attgtctatt catgtcctta gcccactttt 63180
tgataggatt atttgttttt tcttactgat ttgtttgagt tccttgtaga ttctggatat 63240
tagtcctttg tcagatggat agtttgcaga tatttctccc attctgtggg ttgtctgttt 63300
actctgatga ttatttcttt tgctgtgcag aagctttata gttttaggtc ccatctattt 63360
atcttttttg ttgttgttgc atttgctttt ggtttcttgg tcatgaactc tttgcttaag 63420
ccagtgtcta gaagagtttt accaatgtta tcttctataa tttttaaggt tttgggtctt 63480
agatttaagt ctttgatcca tcttgagtgg atttttgtat aagttgagag atgaggatcc 63540
agcttcattc ttctacatgt ggcttgccaa ttatcccaac accatttgtt gaataggatg 63600
tcctttcccc accttatgtt tttgtttgct ttgttgaaga tcagttggct gtaagtattt 63660

agctttattt ctggattttc tattctgctc cattgatcta catgtctatt tttatagtag 63720
taccatgctg ttttcctaac tatagtcttg tagtatagtt tgaagttggg taatctagtg 63780
cctccagatt tgttattttt tgcttagtct tgctttggct gtatgggctg ttgttttgtt 63840
ccatgtgaat tttaagattt tttttcttgt tctttgaaga atgatggtgg cattttgatg 63900
ggagtcgcat tgaatttata gattgttttt ggcagtgtgc tcattttcac aatattgatt 63960
ctgccaatcc atgaataagg gatgtgtttt cattagtttc tgttgtctgt gatttctttc 64020
agcaatattt tgtagttttc ctgtagagat cttccacctc tttggttagg tatattccta 64080
agcatttttt ttttttgcag ctgttgtaaa aaggctcaag ttcttaattt gattctcagt 64140
tttgttgctg ttggtgtata gcactggtac tgatttgtgt acattgattt tgtatctgga 64200
aactttactg aattaactta tcagatctag gagctttttg gatgagtctt taggttttct 64260
aggtatacaa acatatcatc ggcaaagagc aacagtttga cttcctcttt agcagtttgg 64320
atgctcttta tttctttctc ttgtctgatt gctctggcta ggatttccag tactatgttg 64380
aatagaagtg gtgaaagcag gcattcttgt cttattccag ttctcggggg aaatgctttc 64440
aaattttccc ccgttcaata taatgttggc tgtgggtttg tcataagtgg cttttattac 64500
cttaaggtgt gtatcttata tgccagtttt gctgagggtt ttaatcataa agcaatactg 64560
aattttgtca aatgcttttt ctgcatctat tgagtttatc atatgatttt tgtttttact 64620
cctgcttata tggtgtatca catttattga cttgcatatg ttaaagcaac cctgcatccc 64680
cggtatgaaa cccacctgat catggtggat tatctttttg atatgctgct ggattcattt 64740
agctagtatt ttattgagga tttttacatc tctgttcatc agggatattg gtctgtagtt 64800
ttcttttttt gttatgtcct tttctggttt tgatattagg gtaatactgg cttcatagaa 64860
tgatttaggg aggattccct ctgtctctat cttttggaac agtttcaata gaatttgtac 64920
caatttttct ttgaatttct gatagcattc acctgtgaat ccatctggtc ctagactttt 64980
tttgtttcct gacatttttt ctattattgt ttcactctca ctatgcatta ttggtctgtt 65040
aataatttct atttcttcct gttttaatct aggaggtttg tatatatgca ggaatttgtc 65100
catctcttct tggttttcta gtttgtgtac gtaaatgtgt tcacagtagt cttgaataat 65160
ctttttattt tctgtggtat cagttgtagt atctcccatt tcatttctaa ttgagcttgt 65220
ttagatcttt tttcttgttt tcttggttaa tcttgccaat ggtctattga ttttgtttat 65280
cttttcaaag aagcaggttt ttgtttcatt tatcttttgt attgtatttt gtgtttcaat 65340
tttatttatt tatttattta tttttatttt tattttttga gatggagtct cactcttgtt 65400
acccaggctg gaatgcaaca gtatgatctt ggctcactgc aacatctgcc ttccaggttc 65460
aagtgattct cttgcctcag ctgcccgagt agctgggact acaggtgcct gccaccacac 65520

ctggctaatt tttgtatttt tagtagagac ggggtttcac catgttggcc aggcaggtct 65580
caaactcctg acttatggtg atccgcctgc cttggcctcc caaagtgctg cgattacagg 65640
tgtgagccac cacactaaga ctcaatttta tttatttcta ttctgatctt tgttatttct 65700
tttcttctgc tgggtttggg tttgctttgt cttgtttttc cagttcctag aggtgtaagc 65760
tcagattgtc tatttgtgct ctttcagact ttttgatgta gatatttaat gctatgaact 65820
ttgctcttaa catggctttt gctgtatccc agaggttgtg ataggttttg tcattattat 65880
tgttgaattc aaatattttt aaaattttca tctttcttga tttcattgtt gacccaaaga 65940
tcattcagga gcagattatt cgatttccat gtatttgtat agttttgagg gtttcttttg 66000
gagttaattt ttaattttat tccactgtgg tctgagagaa tacttgatat aattttgatt 66060
ttcttaaatt tattgagact tgttcatatg gtctgtcttg gagaatattc catgtgttga 66120
tgaaaaggat gtagttgttg ggtaggattt tttgtaaata tctgttaagt ccatttgttc 66180
tagggtatag tttaagtcca tgtttctttg ttgactttct gtcttgatga cctgtctagt 66240
gctgtcagtg gagtactgaa gtcccccact attattgtgt tgctgtctat ctcatgtctt 66300
aggtctagta gtgattgctt tataaatttg ggagcccaag tgttagatgc atatacactt 66360
aagattgtaa atttttcctg ttgaactaat tattttatca ttatataatg tctctctttg 66420
tctttttttaa ttgttgttgc tttaaaatct tttttgtctg atataagaat tgctattctt 66480
tctcactttg agtttccatt tgcatggaat atctttttcc accccttttac cttaagttta 66540
tgtgagtcct tacgtgttag gtgagtctct tgaagacagc agatacttgg ttgatggatt 66600
tttatccatt ctgccattct gtatctttta agtggagcat ttaggccatt tacattcaac 66660
attagtattg aggtatgagg tactgttcta ttcatcatga tagttgttgc ctcaatacct 66720
tcttgttgtt gctgttgtta attgtgttat tattttatgg gtcctgttaa atttatgctt 66780
taaggaggtt ctattttgat gtattcaagt tactgtttca agatttagag ctccttttag 66840
catttctcag tgctggcttg gtagtggcaa attcagcatt tgtttgtctg aaaaagactt 66900
tatctctctt tcatttatga agcttagttt cactggatac aaaattcttg gctgataatt 66960
attttgttta agaggctaaa tatagggccc aatctcttct ggctagcagg gtttatgctg 67020
agaaatctgc tattaatctg ctatgttttc ttttatagga tacctgatgc ttttgcctca 67080
cagctcttaa gattctttcc ttcatcttga ctttagacaa cctgatggct gtgtgcccag 67140
gtggtaatct ttttgcattg aatttcccag gtgttctttg tgcttcttat atttggatat 67200
ctagatctct agcaagacta ggaagttttt cttgattatt ccctcaaata agtccttaat 67260
gaccccacta tataacatga aatatctgtt attggtactg aggtgctggc cacaaacaat 67320
tctgtgtgtc ctgaaaactc ttcagaatat tcgtcatctt tagcacttgt tatcttagtg 67380
tttgggcttg gcttagagtg atacatctca taacagggca acagaaagaa ccaggaacca 67440

agatttatat aacataagtc agtaaaacta gaggcaccag aggtttacat ttacattagg 67500
ttacattttc taacaggtag caaagcacat gaatgaagtt cagtggaagg ccttcctcag 67560
gaatccagta aaaaccaaac atacacacac acacacggac atccgtgagg caggaaggga 67620
tgtccactat agtacagaca agcatcctgg aaggccatca aggagtaggt gggtttcagt 67680
tgcctcagga atgtggcatg gacccaaact aagtgagtac agatacttgt cattgaggag 67740
aagattcaaa atagcatcct aggtgtaaaa actgaggcac ctggggcagg ggaactaggt 67800
ctctggaatg ttggcttaaa agcacccctc tcaggaaagg cctcatatgc catgcagggg 67860
gttatatatg tgttgtggga cacagatggc aaggagataa ttctatgcac caggctccac 67920
tactaacagg taaacagacc aacattaaca gagacttagg taaaaaggta ggtgcccagt 67980
ggtcagttct caggcacttc caagatgcac ctaacagaaa tgtaacttgg tgtctattgt 68040
gtcctaggtc taacaactga agagaagtga attagtacct cttgtggaca gagaaacagg 68100
ggcagagacc cattacaaag ctgtctcaga taggcatttg aagctgttta agtatgtaga 68160
ggcttaagtc aggctggttc tgaaatgtga gagagggtta agcttcatgg gaaatcagca 68220
gggtagtttg ctatttttta ttataaccaa tctcacaata gtttgggaca tcaaatatca 68280
aattgttggg aatatttatc catattagtc tttttgccac taatatttaa aaatagttta 68340
caatatacaa caaaaagttg taaaatttcc atctccactt aatcgatctt atgtaaccca 68400
tacaatacat caaatgtcct ttccccactt tatgttttta tttgctttgt caaagatcac 68460
ttggctgtta gcatttgggt ttatttctag gttctctatt ctgttttatt ggtctgtgtg 68520
cctattttta taccagtgcc atgctgtttt ggtgactatg gccttatagt atagtttgaa 68580
agcaggtaat gtgatgcctc cagatttttc tttttgctta atcttgcttt ggctatgtgg 68640
gctctttttt ggttccatat gaattttagg attgtttttt ctagttctgt gaagaatgat 68700
ggtggtattt tgatgggaat tgcatttaat tgtagatttc tcttggcagt attacccagg 68760
cttttcttat tttggcaccc tgtgctgctg tctccttttc cttctttctg cttctcttaa 68820
ccaactgtta cctacacttc aatactttct gagggcaatt catcctccag taagtctccc 68880
tgaatcttct cttccttccc tggcttatta tatatccttc ctcttggttc ccatagcacc 68940
tatgcacact tctgtcattg cacttgccaa tttgttttat aatgatctgc tcatctgtct 69000
cctcacttag actatgagct cactgagagc aatggctgtt gcattcacct tatatcctca 69060
acaccattct gaaggcaaga gaaagaatac ccagaggtgg agctgggaag ctggttgtcc 69120
aagtagtgaa tgactctagt ttgaattgaa ctctatagcc agtgggcaat gtggatgtgt 69180
tgacagtttt ttaacagggg actagtgaaa acacattttg ggtttagaaa aaattgcaag 69240
tctgatgaca tacataggag aagagattag agataggaat ttcacttcag aaatttaacc 69300

acaagagcaa gtgacagatc acggaagtct gaaccagact ataaatgtga gaatagagaa 69360

aaaagttaac aatttgggtg tgaaagggcg agggagagag gtgtgaagaa tgactaagtg 69420

tggatctgtt tttaaggatt gaatggaaat ttgagcattt tagctaatca ggcctaatat 69480

tgagcaaagc aaaactcttg caaattgtta tttcaagtgt gggctgagaa aatgaaaaaa 69540

tataaattct cacgttataa cctcttccgt gtgtctgatt tgatagaatc cagccccatt 69600

gcctccaaat tccattgcat cttagaccag caaacacaag tgaattctac ttaaccccag 69660

aattctgtat gaaaatctta ctgccttttt ttttctaatc atgtgtcaaa gtgtgggaag 69720

aacttttatt tatgttttaa taaattgtca gtataaccat ttttacttga aaatattata 69780

atttttcaag taaacaaatt gtttctctaa gttgaaaatt ttatgatgga ataaaagtat 69840

ttttcctcaa aacacataga aattttacaa caatatttta gagttaacta aatgtttctt 69900

tagtagttta gtcacttaaa aagtgatatg attatgaaaa tacttaaact ttgtctttta 69960

actatttcta ataatgctat tggtataatt tcatattttt atactgatct tttctccaaa 70020

ctttagtaaa acatacttct gtaaacccct gcccacaaaa ctgaagtcca catttacttc 70080

tgaatgactg ataagtttgt aaaagtatgc atgaatttcg ttattaaatt aaagttttta 70140

ttatatttta tgcacaatgg tataaattat taaattaatt ttcaagctta tagaacattg 70200

ataaagattg tcattagaaa accctgagtt gattgttata cattacataa cctttcattg 70260

gtggattagt gaatatgtta tagggtgacc atgaatccaa agaatcaaag ctggctacag 70320

caaacagagg gtcaaaagga tatggaacta tgcatgatcc agcaaaacac tcaatatctg 70380

ttttcctgga atgttaaaag acaaagaaga aaacttgggg aacactagat gcatatagtt 70440

ctggttcttt aagaataaaa atatgggccg ggcccggtgg ctcatgcctg taatcccagc 70500

actttgtggg aggccaaggc gggtggatca caaggttagg agttcaagac cagccaggcc 70560

aacatagtga aaccctgtct ctactaaaaa tacaaaaaaa aattacaaaa aaaatacaaa 70620

aaaaaaaata gccaggtgtg gtgacaggca cctgtattcc cagctacttg ggaggctgag 70680

gcaggagaat cacttgaacc cgggaggcag aggttgcagt gagccaagat agtgccactg 70740

tgctccagcc tgggtgacat agtgagactc tgtctcaaaa aaaaaaaaaa gaataaaaac 70800

aagaatggtc agagtcctag taccttgtcc agtgtagtgc tgccttgaga ttgcattgca 70860

atctgtctga gagatagtaa aagaaagtga taccttcctt agccctgttt ctctttagac 70920

tatgctttcc cctctccaag ttaatatctc tcagtctaaa gcctgggaaa aggtgccaat 70980

tttgtttttc tttcttcctc acacctccta gaagttacac tgggacacta ttactttttt 71040

ccaggctttg gccatgtgta ttgttttgga gagtcaactt cctttttttct ttcattctgc 71100

aaatagtttt gagctgtcac tctgtactag gtgctataaa acttacaggt gcattttaca 71160

tgcctatttc ctataggcca cgatttaaca aaatgttcat aaatgagaat taggagtgca 71220

tgtattgaat caccacacat taactgaaca gctttcattg gccagagact atattgacag 71280

tggagattca aagataaact agagaaatct catgcttaaa taactttcta taataaatta 71340

tataagagaa gtaggttcag ggatcttggg agctcagaag caggatgagt taaacaaaag 71400

ttggattttg cctttagctt ggtttcatta tcctgaagga agagcctgaa atatagtgta 71460

gggtgcaagt agtatatgtg ggtggcaatc tcgggaaaca ggagcatgtg atgaataagg 71520

agaaaaagcc aatataaagg tactgcattg agggcaatga gggctctaat tctctgcacc 71580

ttctcaagca ttgtgcagat tggttttctg gattatcagc ctgaaggaca aaacgaagaa 71640

acagccatta gctcctgtct cccattgtct gagagctgcc actaggatat taacttcctg 71700

aaattctgca gaaatctcct cttactttgg cactggagat gcccatacgc agaaagcaaa 71760

aaggcacagc atatttaagg aagctcataa gaaacagtgc atccagaagt ggcgagaatt 71820

ggaggaatgg acatgagact ctaagaacca gcgcctttga tgttcctttt gatctgttat 71880

gtagctcttc ttgtacacag gtgagcaaag gcatgctgga caaatggatt cacatgtgct 71940

aaagcatggg gcaaaaacca catattaatt caggaaaaga caagatgcgt ggccctctct 72000

gtctctgtct aagggtgaat taaagagggg atatatgtac agagtggcag ggcaggactt 72060

gagataagaa ggctaggtgg gtgctctcat gctagtagca ttatagtaca ggtgatgaga 72120

agctcctgaa gaatcatctt aacatttgta ttttagagca acagtattga gttctgactt 72180

agagacagca aaactaaaga cagaaagact attttgatta ttaatgatgt agatataaga 72240

atatcgtcaa tgtgaactaa agcatgaagc tacttatgat atatcattaa aaggatttaa 72300

ctgattggag acaaacgaga gggatgggga aaagaattca tttgttttta gttgctcttt 72360

ttttcctact tattcctttg ttccgagtgt gaataaactt tgtaaacttt tatactaaaa 72420

cattctgctc attcatactt atttctttga tgaaacaagg aaacccttgt atagttataa 72480

acgtgtgaat caatttaaat attaggaaat ttttttaaat aaagctagtt ttctgaaggg 72540

gaaaaacttg gttcaatttt ttgctggcaa tctgctttgt gatttttgaa catgatatct 72600

acatctagac tcatgttttg ctagctggaa tttttttca aattaacgct accattatta 72660

tatgctttac tatttagctt ttgcagcctt ggaaatctat gattaataca aataattctc 72720

tatggcaatt ttaaaaatac atgtaaaagc cttcaatcta cattgctact gtgtcgtagc 72780

acaaaaaaag aaaatgtgat caaattttaa taaaatctac aatttattcc cttctaaata 72840

cagtcctagc tcaggagaaa ggaagctatt tgtatttttc agaatcaaat ttccctaaat 72900

gaatatagag aaagaattat aactgaaata ttgttgaaac agtggtcatc tcaaatctga 72960

aggtcattcc aaaaaagttt ctgagttttc attgcctcaa tctaaaagtt ggccttttttg 73020

gtaatagatg aaagtaaaat aattgaaagg gtctgttgca gttttggaat atcttgaaaa 73080

tatagtagag tgaagccttc ttcccttaaa taaaagacaa gttgctgatt gttttctttc 73140
tagccagata agaataatgc cttctttctc ttgttagtct taacacctca cttgttacta 73200
tgtgtcagaa aggcgagaca ccataaatgg agatactact gatggaggtc atctgacatg 73260
gggctggtag gcagtgggaa gactggtatg gacacaggtg gcttaggggt tggggaatga 73320
tatggaacta aggaaatgat aattagcaga acccagtgtg catgtgtgtg cattcgtgtg 73380
tccgtgtatg tgtgtactgt agcacaatgc aagaaagaaa aaacaaggca gacttttcat 73440
aatttcaggg ataaataaat cctttatcac ttcatgtaga atattggcta cttggaggta 73500
tatctaaacg taaatatata actatataac tacatgctaa ttaaaaacat acaaagaaga 73560
agtgcctaaa gaattacaac agaaagtggc atagtgatta ttagagttaa tataatataa 73620
ataaggccag gcatggtggc tcatgcctat aatcccagca cttttggagg tcaagttgca 73680
gggatcactt gaggacaggg gatagagaca agcctagcca acatggtgaa acccatctct 73740
actaaaaata cagaaattag ctgggtgtgg tgatgggcgc tggtaatccc agctactcaa 73800
gaaactgaag caggagaatt gcttgaaccc ggaagctggg gctgcagtga gccaagatcg 73860
cgcactgcac tccagactgg gtgacagaga aagacccggt ctcaaaaaat taaaaaatag 73920
tataaataat atttcaaaac acaagtctgt taagataaaa ggtacagagg aatggtgaga 73980
tgactttttt atttgtgtga taagggactg ttttctgtga ttgtgagaaa gaccaggagt 74040
taagaaaaag tggccatcaa taaatcagcc acttatgggg aagaaccata aaccactctc 74100
agatgaaata caaatgcagt cattatttaa tattattgga atatttgtat tagtttttgg 74160
tatgtgctgc tagtgctggt acattttagt agtcaattaa tattttgtta atcttaattt 74220
ctaactaaat tccagagtga aatggaaata ataatgaaaa aattttattt acaaaacaga 74280
ttttgttttt ttctgttaag aatgatacac agttgtcctt cagtagccat aggggattgg 74340
tttcaggacc tcccttgggt actaaaatct gcagatgcct aagcccctgt tataaaatgg 74400
cttagtattt gtatataacc tatgcacatc ctctcatata ctttcaatca ggggtcccca 74460
accccagggc catgaccagt actggtccat agcctgttag gctgttcgat accaggctgc 74520
acagcaagag ctgagctcct cctcctgtca gctcagtggt ggcattagat tgccatagga 74580
gcacgaaccc tattgtgaac tgcacatgtg agggatctag gttgtgcgct ccttatgaga 74640
atctaatgat aaatgtaatg tgcttgaatc atcccaaaac cattcccctt cccctcacca 74700
tccctgtccg tggaaacatt tcttccagaa aaccagtccc tggtgccaga aaggttgggg 74760
actgctgctt taaataatct ctagattact gataatgccc aatacaatgt aaattctatg 74820
taaatagttt ttatactata ttgtttagag aataatgaaa agaaaaagtc tacatgttca 74880
gtttaagtgt tgataagtgt gtagagaaaa gggaaccctt gtacattgtt ggtggaaata 74940
tagattggtg cagtcattat ggacaatagt acggaggttc ctaaagaaat taaaattaga 75000

attacctaag acccagcaat ccctcctctg gatgtaccca aaggaaataa aatcatcacc 75060
tcataaagat atctgcactg ctatattcat tgcagcatta tttacagtag ccaagatatg 75120
gaaaccacct aggtatgtgt tggtgcatga atggataaaa gaaactgtgg tatatgtata 75180
tacaatggaa tattattcag ccttaaaaaa ggagaagacc ctgtcatttg ccacaacatg 75240
catggacctg gaggatatta agctgtggga aataagtcca acacacatcc acacacaaaa 75300
ttgcataatc tcacttatat gtggaatcta aaaagaaaaa gttcaaatat aaagttagaa 75360
taaaacagtg gttaccggcc ggatgtggta gctcacgcct gtaatcctag ccctttggga 75420
agccgaggtg ggtgaatcac ctgaggtcag gagttcaaga ccagcctgac caacatggtg 75480
aaatcctgtt tctactaaaa gtacaaaaat tagccgggca tagtggcagg tgcctgtaat 75540
cccagctact caggcagttg agaaaggaga atcacttgaa ctcaggaggc ataggttgca 75600
gtgagccgag atggcgccac ttcactccag cctgggcaaa agagcaaaac tctgtctcaa 75660
aataaaaaaa caaaaaacac agtccacaca ctggttacca tgagtgaggt ggcagggagg 75720
agattgggag atgtagatct aaggatacaa agtagcagat atgtaggagg aactaaaaag 75780
ctgacatgca ggatgacaac tatagttagt aatagtgtat tgtattcagg atttttgcta 75840
attgagtaga ttatagctgc tcttgccaca ggggaaaaag tgggtaacta cgtgagatag 75900
acaatggatg tgttaatttt tgtcactata ataacctttt caccatatac attcatctta 75960
taacagcatg ttgtttactg taaatatata caataaaatt tatttttaaa tatctgagta 76020
tgatttgatg atttgtgaaa atagagtgaa ttataataat tttaaatgta agttaatgtt 76080
attagaaaag aaacagaaag aacataccac acagaaagtc tgtctgaagg atctttgttt 76140
tctccaccaa tacaagtgtt cattgattca gaggtggatt atgagatatg accataaaac 76200
aaaaatttca agggaaatat attttattca atgaaaaatt ctcaacacaa ctgttatatg 76260
ccagtaaaca ctatatcttt taaataacag gtcatatcta ttatatttaa aattcaagga 76320
gagactacat tagagatgct attagatcaa cttctaattt caaagatttc taagatatgg 76380
aacagttact ccttatacaa attaaaaaag caaatgctga agaaattcag ctacatggat 76440
acaccatgag gtggaaagat gctccataac tcttagttaa actgcactaa ttacacataa 76500
aaggaaaatg tttcatttca ctgtaatttg gaaaccaaag aaagaaaaga ctgaattttt 76560
acatactgtt aaagagattg cgtatctgtt ctaagtttaa gacagaggca aaatgtattt 76620
tattcatttg tcctgcaccg tttagaaata aaattcaact tccttttaat ttttttaag 76680
aataaaaaac tcagtctaag gaaagtctta aagttttcat tttaagtgat ccactgttct 76740
agaagtttaa tattttgttt aaaatgttta tgttctgtat tccaccaagt ctagttttaa 76800
aacaaaacaa acaacaacaa aatacttctc taacttggag tttaaggtga aagaaaccaa 76860

ttacgtggtt tggaaatgtc acacttttca tctctttttt aaaaaaattt ttaattcagg 76920
acagaaattg tatggattta gtgtaagtct tgggatctca caagtgtcag tatttcactc 76980
tcctccatat cttgatagca ataacttgaa ataggatctc agtagctcaa gcaatactgg 77040
gctctgagag ttggttaaaa attatttggc tgagcgcctg ttgctgaggg aagaactaat 77100
ctcgagcata tttttggagc caaataccaa attgtttgtg cttagcaaca cagcaccagg 77160
cttgcccttc agaatgattc tagaccaaat gccagaaatg ctctggttct gactacagag 77220
ttctattcac aaatgacagg aggcaagagg tcctcctcac tttcagaaga aaggtccttt 77280
gctttcttag tcaatggtag gaaaaccatt gtggttttca ttgcattaca taatttttaa 77340
ggtgattact tcaataagaa gtgctctgtg tatatgtgtg tttatagacg catttttttaa 77400
acactggaga atttctgaaa gtagtacaaa ccttgtaatg tcaagtagat gtgggaaaaa 77460
gggagtttac aacattctct cctgacattg ctctcctttg gcatctgcat ttttaaaatg 77520
ttaaaaatgt ttaaaaacgt gtgcttaaca cttaatttgg tgatagttgc tgttaccaag 77580
gcaactctgt aactccaccc agataaaaat aaatcttgaa gatgagtttc tgtgtctctg 77640
agcaaatatt tttgtgaata gtagaagcag agaaagttaa agatacctga gcttttgatc 77700
tttactagtt ttatagatat gtttatagtt atacattttt attcatacat tttagataaa 77760
taactttgta aagcaattga ttcttcttgt aaaaatcaag tatattctta atagactgat 77820
aaactttctt tttttgagac agagtcttgc tctattgccc aggctggaat acagtgccat 77880
gatcttggct cactgcaacc tacctctgcc tcctgggttc aagcaattct cctgcctcag 77940
cctcttgagt agctgagatt acaggtgcat ggtaccacac cccactaatt tttgtattct 78000
tagtagagat ggggttttgc cattttggcc aggctctgag aaacttttta aggtctcttt 78060
tgcagccagc tatttgtcta ccttatttca ttcttaatct cactagccaa tatttttttct 78120
gtttaagtgc tttcagcaaa tattaaatgc ttgtgccttc agtcttatcc tgtggaaaca 78180
ctggtaatga caaaaacaca tatttcaacc taatatacaa tagaaacaga atgccagtta 78240
ttcatggagg agaagaatag acttctgtat ttaaaataac attttgctct gtgttttaaa 78300
atcattcttc cttcatcaat tgtaagcatc ttgactataa tttatacacc taaagataaa 78360
taattcagta gcaatgataa ctgaaaacag gacacataca atgaactagc taaattacca 78420
tacattctca tccatttcaa aaatagctct gtactttttt cagattttgt tagaagaata 78480
ttcaatacaa atttttattc aatgaacact tcagatgtca agattgttac ccacatggac 78540
aacagtaacc taggtaaaga ttctgcagcc aggcgtggtg gctcacacct gtaatcccag 78600
cactttggga ggctgaggcg ggcagatcat gaggtcagga gatcgagact atcctggcta 78660
acatggtgaa accccatctc tactaaaaat acaaaaaatt agccaggtgt ggtgtcatgt 78720
gcttgtagtc ccagctgctc gggaggctaa ggcaggagaa tcgcttgaac ccgggaggtg 78780

gaggttgcgg tgagccgaga ttgcaccact gcactccagc ctgggtgaca gagcgagact 78840
ctgtctcaaa aaaaaaaaaa aaaaatttta tacctgggct ctgtgctcac cagcagaagg 78900
ggtaacatgg cttcttagga caaccttact tgaccattta cttctttgac actaggggta 78960
ttcttagatc agcaggtcct tccctccact tatgcacatg aggctcacag agagtctggg 79020
aggcagggaa tttatgattg gaaacagtat actttttatc taagaaatta ttaatgtcac 79080
tgcattcaag tgattaacac catcaatatc ttcaagacta aggggattac atgatgtgta 79140
aaattagaaa actgtcatct actagtggct aggcacttta attatattaa gcatgcaaca 79200
agagaactct tcaaatgaat ccatctctcc tctgtattat ttccaaccct tggatcccca 79260
tctgtttctg cagacaacag ctatgctgct gaatgtctta atggtttgct gccccaacta 79320
gcttcaagat actgcaggtc aagcatagca tcttactctt ccctgcatct ccagcacctc 79380
tcagaatgtt ggtcacatag aagatgtttg ctgaggagtt gaataagaat atgtacaagg 79440
gacacaatta gcattgttta aaaaagatgt aacaagatag ggtaaaggaa agctttggag 79500
gataaatctt tagaacaatc aataatatct tctcctctgt tggttagttg cccttcaatc 79560
tcagccactg aatcaaatac aacataatta ctattctgat atgttcttga atcgaatatc 79620
caataataag atattcggat gcatagccat gtctaatatc aaagcccatg cttttcgcta 79680
ttattgtact ccatacatta gcttccaaat ttatttgcaa tccaaatatt aaaagcaagt 79740
cataagctta gtatcgccaa tgtgatacta agtatccact tactaaactt tattttcaaa 79800
atgtggtttt atctcagttt aatgaacacg gcatgtttta atttacactt tcatattata 79860
tagtaagggc gtggttacag atatgttaat ttcctgtgct gcttcacaat gatggaacat 79920
aatagcaaat gaaactgtta atttgcagat acccataggc ctttggtgtc tgaatagaaa 79980
taaacacacc tacaactgag agaggaagca tgtgaagcat tccagtgaac agaggccatt 80040
tattcagtca cagacacagg agaaaaacaa caattaaaaa aaaatctctg atgaaaagtt 80100
cataaaaagt tcactcagtt taagcatatg tcctataact acttaaaata gagttcttct 80160
taaatatcat tctttgctgt ttttagattt cttctgcctg tatcaaatta atagaacaca 80220
gcatactttt aatttgctct ggtttcttag tggggcattt attaaacaca ttaaaacaat 80280
agtctcaggg ttttactgct gatgttaaag ttctgctttc ctacttacca actgtgtcat 80340
cttaaggcac atactttgcc tctctctcaa atctcccaaa tggagaatga taagaatacg 80400
tacctcaatt aaagaagcta taacaagtag aatgtttgga aaagtgccgg gtacaccata 80460
agcccactat gagtattgga ttgtattacc tctgaaagct gcagaatgga attctcaaag 80520
ttatatgtcc ctaaaatcct cttaagtgac agaaatggag aaattagcag tctgtctaag 80580
agagcttttc tagagtctgg gcatatgttt ttaggacaag acagttcagc ttcagcttaa 80640

aatgagagag cacgtctgtg tccttactcc tgggtgccag gtttcttgtc cccatcttaa 80700
gacaaataat tttggtggag aagaggcagt ctctttgatt tcgctctaaa aaccttttct 80760
ggaggaggta gacactctcc acccccgttt tgagactcat gcagctgagg atgactggct 80820
gagtacaagc aattgttcct tctaagcagt ttcaattctt ataacttgtg gagatattct 80880
taagtccagg ggattttgtg tatggtggat ttttattaca aagtcctgta cttcatagga 80940
acaaaataat tcaaagtcag gaaccagatc aaagccacaa ctcagatatg gcaccttgag 81000
aagttcattt gtatttcact tgcataaaaa ccctcaccac tgctatctga ttttcacaaa 81060
tcattcaaca gctatccatg aagcacccac tgtgtgtctg gtctctgtgt cagtccctgg 81120
cttcatgtgt ctttccttct gtaccctgac tccccaactc atgaacacat gaagtaaaaa 81180
aatgaaaatc tttttctgac ctctcttcaa aatcactttt ttcaaaacaa acacctctca 81240
cctgctcatc ctccagccag taaatcacag gggcctagaa atgtcactta caaatatttt 81300
ctgattctgt ccctcccttc aagcttgcca acattatcac agtttagggc ctgctcatct 81360
ttcccccaat ctccaattag atctctccac aatgcaattc tgcacattcc ctgttacaac 81420
ccttcaatta tttcccagcc catccaaaat aaaatctaag cctcttacta acacattcag 81480
gaactctgtg gcctacggtt ttctacagac taattttcca gcagttgact tccagtgcaa 81540
gtgaaaacct agtgtcatgc ctgcatgata gataaatttg aagctgaaga gcccaaatgt 81600
atagaccatg ccatgaaagg tttatagtca tgacacagtg gccctatagt acagtgcttg 81660
aagctggctc tctactgtca gacagaccac ttgccagcca tgagacctgg ggcaaaatgc 81720
cttaattttt atgtgcctca agttctcatg tgagatgaga ataaaaatta cccctatttc 81780
ataagatttg ataaagtgtt tagcataata cctcataaca attgcaattc agtggtggtt 81840
attattataa agaaaagatg attaacttta tcttaatgtt taacttgttc tgatagttat 81900
tgatctatag ctttgatatg gaggtttgag aatgacctgg aaagaattgg ccacaatgat 81960
tgaagatagt gatacaagaa taaaagatga ctgcaaaatg taaacctgca ataacagaaa 82020
gaatgaagtc actggtctca tgggaactga tatgggagaa aaaaacagat caaaaggcta 82080
ttcatgtttt gggcctcttt gtcaaaatgg aaatgagaaa ctggggaata aaaattaaag 82140
caattctagc atctggtttt aacataattc ttatccctaa aaagaatcta taagaaactc 82200
ccaaaatgac aggcagccgt gggtagcatt gcatttcaag taatctttta attgttaaaa 82260
tttaagtttc caacatgaac ataaaatttt caacctaaaa gaaatgagtt ccaaatctga 82320
gacaagtgaa aaaggataaa gcctactagg gggtaaattc catctcttta gagatctagt 82380
acccaattta gcaatgtcca atcaagcctt taactactac atttgaacac ctcatcattt 82440
caaaatgtta cttaatgatg ccaattaact gtacaatgtc tctgcatagc acatagccct 82500
aaaatgattt gtgcaatgtt actgtcagta aaactgaact acagggaatg ctcatattct 82560

atgtcattat atacagaaat gcaatatcaa taaagtgata tctgttggta ttagaaaaaa 82620
gtgaaaattt tcatatcttt ctattttctt ttttcctcaa tgggatgctc ttgttaaaga 82680
tagctctgca tagtaaggtt tgtataaaca ttatttagct aaagttaaaa ggggtaacat 82740
actggttcta gcacagatat taaaacaaat tagtttgtag gtagggcagc aatcaattat 82800
attactaacc atagctttgg tccttttatc ctttcccatt tgattttaca cagtgggatg 82860
ttaaaggttg aatgtctttg gtatctataa acttaattga aagctgttat ttgtttgttt 82920
aagtctgttg atttttataa tcataatttt actcctatag atttcttgta ggagtactat 82980
atgaatttat gttgcactga attttgttat gttatacaaa ttaataggct tttatttatg 83040
gaaagctact attgatctgt catttcttaa aaaattacta aaaagtgtta aaactttaaa 83100
tgttggagag tttatatttt aaaagttaca tgctagaaaa acatgatgtc tgagtatatt 83160
agaagttata gataattcat ctgtcaacta taaaactctc caacactgcc tttctttaat 83220
gaataatatg aaatttagca gtgaaaatgt gacaatgtac aatcctaaat aaatcaacaa 83280
atttagagat gtacctctaa aaccattgta aattcaacag tgtaattttc cattggactt 83340
tcacttattc attcattaaa caaatgtttg tgagtgcctg caatgtatga gacattgtac 83400
tgaagctagg cagtgtgagt tatcatatgg gattatcctt taaatacttc tgagggcaaa 83460
aaaaaaaaaa aaaagaagag aaaaggtgtg aggaaagata aagggttaat tcattaaaaa 83520
ataacacttg aggactgttt tctttgcaag gcataaagtt atcacccttt caaacagtag 83580
atatttcaca tttaggatgc gagactccag ttccaacaaa gctcattgca cagctgctac 83640
cctgattaaa ctgctacatg aactctgagc aatgtagcat ggtagccgca tgcttctgct 83700
tgcatgatgg ttaattcctt ccattctcat tagtgatttt ctgagctttg aaattctgat 83760
ggtacctagg atataaagca tatttatcta actgaaaaac agataattag atgtaacata 83820
aaatatgaat ggctttgtca ctttattgta gcagagaatg aatgtgggat aaattaaagc 83880
tgatgctaga acatatgcct atttttagc tggaaaattt caagatttat gtactttggg 83940
cttgagaaag aaatggagtt tattttttat gcactgacat ctcttttttt tttttttgg 84000
aagagctctc ttaggaatga atggtatgta aatacagtag gaatgtaatt atagattttc 84060
ctgacccagt tcctaaataa tagatatcat ttcagaagtg ccccaatacc tgacctttg 84120
ctccaagcca tatcaaagca cacatctagt ctacttttca ctctcattcc tagccactat 84180
gacaatacta ttcagataaa acttctagtc ctctacttat gtgactcata ccaacttgac 84240
cttacgatag tgactggggg tgcatatcta ggttcatgct gtttgtccat tattatggtt 84300
ttgtgagaaa aggcaaaatt tctaggtaaa gtgttatgag gacgaataat ccaccaggca 84360
accaactgac cctttcattt gccatcttgt cacttcaaac agctctccag aacctgcagc 84420

cagcacagac caaagtcagg tttgtctcct cttctgttga tgaacaaagg ttgattccat 84480

atcgtggcta ttgtgaatag tggcagtaaa catggcagta ttgtatgaaa atatcacaga 84540

tagcccttaa atatgtgcaa ctatgatgat ctatcaaaat taaaaattaa aatttatttt 84600

taaaagttca gttagaaagc ttgtagttcc tggcaaacta ctacctttct cggcaaaaga 84660

atttgatatc tcttaaatat tttctgccta atgctgatag attgtattta catattccat 84720

taatgcaata aataaaatta caccaaaaca tcagcattat ttatttccag gggcatctct 84780

caaaataaat tcctccaaaa ttcacaaaac caaaaccaat gtgaaattgt actcagggat 84840

gcaaatgtag cccagtgaag catttgccca cttgtttggt attattgaag cacaattaga 84900

aaaatgtgca atgtatgccc aaaaattcta taataagggc caggcgcggt ggctcacacc 84960

tgtaatctca gcattttggg aggccaaggt gggcaaatca tgaggtcagg agatcgagac 85020

catcctagct aacaccatga aacccagtct ttactaaaaa tacaaaaaat tggcccagac 85080

gtggtggcgg gatcctgtag tcccagctac tcgggaggct gaggcaggag aatggcatga 85140

acccaggagg cagagtttgc actgagccta ctctccagcc tgaacgacag agcgagaccc 85200

catctcaaaa aaaaaaacca taataagaac tttttaatat actatattat aatgtaaaaa 85260

gactagatgt caaacaaatt aggtgatggg aaggaattga gggagaattt tagactaagc 85320

aattgagcag cacctgtttt tcaccacaaa tctgttacat gtattgctca attgtgctga 85380

atccatattg ggtcctggtg gctatgtaat agtctctttc ttggataaat gtttgtcctc 85440

tcttatggtt tactaatggt gtacagaaca gcattgaata gtggttattt cctatgactt 85500

cctagatatc tctctcataa tcctgaatgt tttaaagatc attcttagat agagtacagc 85560

tagacacgaa ccatagtgga aatcaggtag acaaaattta aaaggagtct taattgaagg 85620

tcattttatt gtcctcagta ttaatcttac ttaaaacaaa cctgtcactg agcagaactc 85680

aaaacaccag agccctttgc caaatgtgat tttttacaac aggagcgctg gcagttgaga 85740

ggagtattct gtcacacttg agagaattcg agtccctgaa gatttatatg aatgcttagc 85800

tattatcgaa ccatctcttc acagatgact tagtaaatgt ctgcctttgc atcagataat 85860

ggcttacaag ttaatctcct cttgctccct gttacacaca tatacacctt cttcctaaac 85920

agctcataag gtgaaagaaa gactcagatt tctgactatg taattgataa tatcacacgg 85980

actgcctgct catcatctgc tagtcacatt ggcagagttg acagttttgg agacactgaa 86040

gacagtgcat atattaggaa ataagcagtt tcctgatata aattttcttg tagtttataa 86100

attacatagc atttattatt ccctcatatt ttataacatt taataataga actgacacat 86160

atattcattt taaactcaat tgtgtataat aactatcata gcaacccttc agtgcctaaa 86220

tatcaaatct tccattcctc ccatgaacat cttgaatata taggtactgt ggttagctcc 86280

aacaagcttt tggttagaat tcattgcact gatacataga cattgtttta aaggcaattt 86340

caaatcaaag ctgtcagctg tgaatcaagc acaccttaaa aagtgacaca tttgtcacta 86400
gattccagcc tctcaaatta ctgacacgca tcctttttat gtaaagatga cattgttctt 86460
tcctgatata ttgcattcct catgaatttc ttatagtcat agaattttta taaaccattt 86520
cagaatcgct gaaataaaca tcaatatttt taactttttc attctgtcaa aaatattgta 86580
tgcagagata ttgctgtaag tgtgtatacc tgtgcttaag agactagggc tgaagagaag 86640
taatcaaccg aaccactggt gtaaatgtgc gtcacatttt tagtgactag aaattgaaat 86700
aattccaaca aatttatgtg ctttgggctt gagaattcag actgccttag gctaagataa 86760
aaatcttttc ctggtactat ataccttctt ttattgaatg actacctggc tctttctatt 86820
atatatgcag attttgtacc tctggtcatc tttgtaaatg gtgcctaaaa gatatttgaa 86880
gaataagtga ccagcaataa gaacaaatgt ctatacaaaa gcacccttta gttggatgta 86940
attcactact ttgagttgtt aataacctct aaggatgaca gtagctatta gttgaataaa 87000
ccattatgtc tattattaga acactagata gtttataagt ccaaacaatg cataaaatac 87060
ctatctcatg ttaccattgt ttaggttacc agataattgt tctgtccaat tattccactt 87120
aatttttttgc ttgcccatta gctaaatggc aagataaaat ttgtcaaacg ggggggaatg 87180
tattgaaaat gctagacaac tacacttaaa atgaaaacag gccaggcgcg gtggctcagg 87240
cctgtaatcc cagcactttg ggaggccaag gcgggtggat cacctgaggt cgggagttca 87300
agaccagctt gaccaacatg gagaaactcc atctctacta aaaatacaaa attagccggg 87360
catggtggca catacctgta atcccaacta ctggggaggc tgaggcagaa gaatcgtttg 87420
aacccaggag gcggtggttg cagtgagccg agattgtgcc actgtattct agcctaggca 87480
acatgagcga aactccatct caaaaaaaaa aaaaaaaaga aagaaaagaa aacaaatgca 87540
taatttgcaa atattatttt tatattgtat gttatctagg gcttctaaat gcattcttct 87600
tataagccta ggtttgcaat aacattcatt tagaattgag taattttaaa tataatattt 87660
tataaaataa aatataataa tttctcttaa ttctttgaaa atattaaatt aaaagggggt 87720
tgcaaactct gcattccaca tttccatccc aacatttaat tttagcaatt ttgtagtctg 87780
cctaaaatgc aatccatcat ttactgttta gaaaataggg aatgtacaca aaggcctttc 87840
agctttccct gaactccata aaaatctttt tgcttcttta ctgccccccct ttgtcaggag 87900
ttctgaggaa ctgtttttta tcttaagtct cacaaagcat ttaggagaat atttaaactt 87960
aaattctttt aaaacttatg ttcaggacaa agtaacattg tatgcattgg tgtcatatgt 88020
atttaaattt tgaaattttt aatactggca aaatgaggtt tcaattttaa tataaattat 88080
ttaacaatct taaatcatta aatatattac ttaatatatt taatatatct aaacagtcac 88140
aattttccca tactaataat cataaaaaat cttacccaat ggtcatatag atatacttaa 88200

tggagttttg ggggggtatt tttgtatatt aaaaaattca tatatttgcc ttacttagaa 88260
gaactgatta aatgaaagta taatattaac aaacatattg ttattttata tttgcatttg 88320
tgataattat atttgaaacg ttcaagattt tccaatgaat ttcttttgca tttgcgtatt 88380
tgtgcctttt tattataaaa ataggtggct ttttagttcc actgcataag tttcaacata 88440
ggtctacaaa tagtgcatct ttttgaagtt aatcattata atcacaaatt gaagttgcct 88500
gagctccaat tggagtctaa atggatgact gaatcttatt attcgaaacc cactgttgct 88560
acacaatatg gccacacaag agagtacaca agacccgtct gattcagcct cagtgccata 88620
aatattttaa tggtttcgtt ggaatctgga aatggagctc accacaggag atgcttcttc 88680
ctttgactct cattattatt tcctttacaa attaattaat aaaaacttag atgctaaatt 88740
agcacttgat gaaaacttat atagccttga cattttgatt ctgtgagtga ataaaaatac 88800
ttggagaaat aaaaatccta atcatgttca ggaataccca caaggtaaca agtacatttt 88860
taaactttaa aaacatttat tattcatgat aaaacatgtt gtgtgattta aatataaatt 88920
tttattattt gctttaactt atttccggat taaaaagtaa atgtttacct agctgttcta 88980
aatggtaatc ctcatgatta aaacagcaat ttgtcatatt tcagttacaa atgatctttt 89040
attattagtt atagaacata agtttcttca ttgactgagg cgatgtttca agtagataaa 89100
tctgttaaaa aaattgtggt catattctgt taaattctca taccaggcaa tttgtttgat 89160
attcaggaaa aacctagcca ctgaccaaaa actctacctg ccttctcagt tgtatcctct 89220
tggacttaaa ggggactggg aaagttataa gatggttcat gatagtccat caacatccca 89280
agaacaaaaa cagatgttgt actgacagca tcatatgatc atatgcatgt aagagcacat 89340
tcatattgcc aaatcagttg gaatttttca cggttgaaag ttaaatgaaa tgcttagatg 89400
tatgagtcat cggagttaaa gacaattaca gccagattta tggctgtgct aaaataaagc 89460
tagttagaaa acagaccaaa ttccatgacg ataccaagtc tgactaatga ttcaccttaa 89520
atttcggagc aacatttatc ctcacttgtt tgtttatttg acaatgtgcc cttatccatt 89580
aagtaactag gaggaaggga aaagcactac gtgggtgagt gacaagacac tgacactgat 89640
ttgtgacttt ggataattcc tggatgctgt tatctgtttt ggcatagaga tggatctgta 89700
actgctaata attgccgact gtgaccatcc cagaggccat ttacttaacc caggtatttc 89760
agacctgaca gcccgaggat aaacacgatt tccctccatc actaacttca tctgcagggc 89820
ctaagcctcc ttcacagtct ctccagtgat ttattggcat ctccaagggt atctcacatg 89880
tgctgaagaa caaatctgct cactttcatc tgcttggttt tccctttgga aatctgctgc 89940
tttaaaatta ctaagggagg aatcatgcct gctgctaccc ttgccagtga ccttgcagtt 90000
tgtgccctga ttgttccaat taccacaatc aaaacagaag cgtttgcagt tactgcagtg 90060
ctctctctgt ggatgtcagg tctgactcag agagccaggc tggggaacag ccatttccac 90120

tcttgtacct ctgcaaaagg acttccatgt tccgtaaaca gactcccacc tctcattttc 90180

cccccaagca aagcatcata aattagagag catgtaacgg gaaagaaaat ccattagcca 90240

tttgggttca gtcagacaag ccagctcatg gaaagtttat acaggaaggt cacatttcaa 90300

ttgagatcag gagggtgaaa gggtccagct gtgtgatgag agagagaatg ttcgggaatg 90360

tggaacagag gtatccaagg cagaacaaac tcgtatatga aggctttaag ggtgtgcaaa 90420

tctagcatat tttatgacat aaaagagtcc tgattagcta gaatatgatg aatgtgagaa 90480

gaggtgaagg ctggagatag gaaaaattat tccagatctt ataagctata gtaagaaatt 90540

tgcatattat atatagactt gtgggaagcc attggatttt gtaagaagga gattaacatt 90600

atcttattta tgttatttgt gatttataac cccaaatgtg ccagatacaa acaaaccaaa 90660

aataataata ataataataa gaagaagaac aacaacagca atggaactgt ggtgatggtt 90720

ttggtcacaa aatgcatata tatctatttt tcacaatgca aaaatatttc attatttcaa 90780

attttaacat aaatgtgggt atgcatgagc ttacaaatct tgaagtttat tggggaatat 90840

tggtgagcat ggtttttatt gcatggtcac aacttactaa tgggaaacat ctgaatacct 90900

attgagttaa tgcatgcaca tttttatttt cctggaatac tgagaaaaag gttgctacat 90960

aatgtcttga tagcttctaa gtcatggctc aaaagtgaat gtggaatctg ctaatcggaa 91020

tggactcaga ttcagccaag ttctcaaaaa catttgcttt catagatgtc ttcaagaaac 91080

aaggagtctt gaatttaaat tgtgaagtgt ctatcttaga atagagagat ttaaaatctg 91140

actgtatttt gtttaaaaaa gcctatataa ctgtattata taaaattatt tatactacag 91200

ttaaaaaaag aatcccatcc tatttgtgcc taaataagtg cctgcttgta gcatgaaaac 91260

tatttgttga gggtccttag atcctcagag catgctgtga aagtaggtac aattgttctt 91320

tctatataag cctcttaaga taacagataa ttgccagaaa tacagcacac agtacaaaat 91380

taccttgttt tacttttgcc acaaaaaaca atttcttttg gctttgagca ataaagtcca 91440

atgatttttt tcctttcaaa atatcttcct ccctctccat aagttttata tttattcacg 91500

aaggaatatt ccaatatcgg atgtttttgt ctgtgtctct tcctggaaca aatgttaatt 91560

aatctctttg ggtttgtatg tcaagtggag gggtggggat tggggacagg tgatagttgt 91620

ctagggagtt aacttcatct ctataggaga gtggatagac gctgtatacg aaaagctctt 91680

gaaaagggaa atacagcagc cacttcctca gggcttccat ggtggtcaga ctccttgatt 91740

gctttagatt aactctggct tttgtccttc ggaggccacc agattgggtg gatagacatt 91800

gtccttgctg ttcttttgac ctacctactt gtactttagg ggaaaaaaat gcctgtaata 91860

ggttaaatgc tttctcaaag atcaccaaag tatataacac atggcaaata gacagagaaa 91920

tgagacagta taatcagtat aatttataaa agtaccttac agcaggatcc catgggatat 91980

gggttttttt taaaaaaaat ctacctaatc ttttcattga actcctattc aggattcatt 92040
atattgaata tggctcagag acctggaaaa ttgtttccac ctttttaatt tattcaccat 92100
catttatgga agttttcaag gacgtttact tacctacctc agttaacaga ttgtactact 92160
tgggaagtct ataaatatga gcttaaagca ttttctgagt tttaaaataa tttagattgt 92220
gtagaatgtt aaaactaaaa gaggaaaaaa ttattcagtt cctcagttga acctagcaat 92280
ttatcttttc acagtgtgct caagtatagt ttttgaaaag taaagaagat ggtttttata 92340
caaacataaa cacatttcaa agattttatt caactaatta attagtagtg gagccaataa 92400
gctggtaaga ctggtttaaa ggaatatctg aggaataaag atttatagaa acagtcaaag 92460
aaattctaaa gagaattgac taatagatat aaatctagta aatatttgat taataatagc 92520
agtaacctat ggaattatgt tttctactga gcataaatga gcatgaatct ctttgggttt 92580
gtatgtcaag tggaagggtg gggattgggg acaagtgata gttgtcaagg gagttaactt 92640
catctctata ggagagtgga tagatgctgt ataagaaaag ctcttgaaaa gggaaataaa 92700
gcagccactg cacatctgca catataacct gtagatctgg gggctctaat aaaaaagtta 92760
atggcaatgt caaaatctgg tgttttatct tagataactt catagtcatt gattgagccc 92820
cttaaaaata acatttaaag gacatgtagt cattctgttt ctttattgcc aagttttcag 92880
caatttttct catgagaatg agtgctaaga aacttttggt ggagcgtggt ggctcaagcc 92940
tgcagtcttg cactttggga cgccaaggct ggccaattac ttgagatcag tagtttgaga 93000
ccaccctggc caacatggtg aaaccttgtc tctactaaaa atacaaaaaa aaaaaaaagt 93060
gggatgtggt ggcatgcgcc tgtaatcctg gctactctgg aggctgaggc acgagagtca 93120
cttgaacccg ggaggcagag gttgcagtga gccgagatcc tgccactgca ctccagcctg 93180
ggctacagag ggagactcca tctcaaacaa acaaacaaac aaaaaagaaa cttttaaaat 93240
ataacaatag agacattaca taggcccaca aaaccacctc caaaaaagca ttctatcacc 93300
tgcaagaaag catatatata tatctgcttt tgtgtatata tatatatata tatatatctg 93360
cttttgtgta tatatatata cacacacaca cacacatatg tgtgatatca gcatgtgtat 93420
ttacacatat attttgtgca tgtatatttt taactaaaaa tgtgctagga gttagatatg 93480
aactgatttt ggaggaggtg atatgctgta gagagagaga atgggagaat agcagtatta 93540
taatctctct ccattgtatt cagttttttt ctttgtctga atttttaata gaagtcagcc 93600
agaagatgtt agtttctggg aaatgtgttg agatttacag tcaaatccag agagaactag 93660
aggcttatga gtaaataagt aaaggttatg cagagaaagt attctttttc ctgtgtaaac 93720
ttgaatattg gccaggcgcg gtggacacct gtaatccagc actttgggag gccaaggcgg 93780
gtggatcgac tgaggtcagg agttcatgac cagcctgtcc aacatggtga aacccattct 93840
ctaccaaaaa tacaaaaatt agtgggtgtg gtggcaggat cctgtaatcc cagctactac 93900

ggaggctgag gcaggagaat tgctttaacc taggaggcgg aggttgcagt gagctgagac 93960
agcgccattg cactatagct acggcgataa gagtgagact tcatctaaaa aaaaaaaaga 94020
aaagaaaacc ttgaatattt cttgtacttg tgttcaaatc atacagttat gaaagtttac 94080
ccctagctgt tacacttaaa atgtacttct gaaatataca gagagatgat acagactatt 94140
aatgagttcc actaaacttt taatggttta gaaaatacaa atattttctt atttttctgg 94200
aattccagcc attaatgtaa aacattggtt tcaacataaa taacacactg gcatgcacat 94260
atgcctaagc atgggccccc acacatacag acattctgaa agaccacttt ttaaaaatat 94320
tcagtaccgt atattgtgca ttccttcttt atccacatac ttaagctgct gcaagcatcc 94380
cattgataac accagtaata aaagatggga ccatcagtaa tgagatttga aagccccttt 94440
tgcaagaaag taaggactag aaggtggaaa tcactctgtc ttagagtcat atggattggg 94500
gctttgctag aagtgtgtgc tctcagggaa agctgccttt ttattttctc cagagaaaag 94560
ccttttttgtc agtaaaagaa gatgtatcat ccaatgcata tgtaaaattc taaacagcag 94620
ataaaacaac attcactatt aatctctgca aaagaagata tattgaaaaa atcctcaagt 94680
gtccctcttt gggtttcttt gttatatatt aaagcagtta tctttagatg catgagaatc 94740
acctgaagac cttatttttta aaattcagat tcctgtcagt tcactcccaa agattccgat 94800
tcagtagtta agagacaaag cctaggaatg tgaatttaca atcaacacct caggtgatag 94860
ccatgcatgt tcttaatgct ctactactat ctatgcataa aaggaagata aagttttaaa 94920
aacttgaaat gtggtataac agtttagtat tgaataatat acatttttac ttattgtaac 94980
aaattatgat atctacttgg ggcaacagta tcttttattt tggatctgaa tcctaatttt 95040
ggctaggtat cactgaggga ttcttagtct aaaacaatta aatggagtta gtggtttttt 95100
ttagtaactc ttgattttct gttttttttcc attggcatct tacaaaattt attcattcat 95160
ttttcccttt ttcacttggc attatttgtt agacagtgga caaaagaact atagaaagta 95220
gagaagcatg tgatgttgtc ctgctcttag attctcgcaa ctcaggagag gacattcgct 95280
tacaccaatc atctcaaaac atggcagttt atgctgaact cagtccaatg ggagagcatt 95340
tgactgagca catagggaga gaagttagct ctgttgaagg ataatcaacg aagaattctt 95400
aggaaaggta cagtcattca ttgaatattt gctcggcact tactaggtgc atatgtgcac 95460
taagatctaa ggatgggctg atgaagaacc caggtccctt ttcttctagt ggacatgcag 95520
actggcctaa aaaaaaaaag gtaactggaa aatggataag gaaactgagt cactcggttt 95580
atttattatc actcggttta tttgcttttg tttgtatttt cattttgaca cagcacagtg 95640
tcatcttaac gcatcctcca aagtgaagga tggggtggat aacactttag ttggcatttc 95700
tgtagccagg agccaggatc tttctcccat aattgcatta acctgggaag gcaccctcta 95760

ggtagatttg tatagcaccc tggttaatca attatcagtt tacttcttgt ctcactaagc 95820
tttaacacct tacatttatg aagcagtgta aatataactt tagcatcttg atcacagcaa 95880
gcacctgatt tgtatttttt tattagctca agtgaaatca gatcagagaa gtacattaca 95940
ggtcataaaa tatgtgcaaa tttcataatg acctcctttt aaaatgtgca aaaataagat 96000
tgttaaggca cattccagag ccttgggggg tgtgtgtgtg tgtgtgtgtg tgtgtgtgtg 96060
cgtgtgtgtg tgtgcttgtc ttttgagaat atctgtatat cagaaaattt ggctgagaag 96120
caatcttctt cttagtggtt ctttttctct tttgaaaata aagtactaaa aatacttaaa 96180
gatgcagaac agcaacctgt tcccagtgag actctcgttt aattaatgtg gtgatctata 96240
tagagaaaag ggacaattgc aaaagtccct caataattat ctaaccacag tctttaggta 96300
attacagcag aaagattttc aagacacaaa acaccctgga aaatttgacc tcttattttg 96360
attcaggcct ttcatttctt aaatattttc tttaatgttg atgtttatgc ttgacaaggt 96420
cagcctaatg ccagatgaat ccctggaact caaaacattg ctgaattcac agttgaagga 96480
ttttaatata atataccagc ttttaaaaat cctacagtga gaataacagg actgaataaa 96540
aaaattaaga aatgctcagg tagaaataaa tagagaaatt tagaaaaaaa ataaaacgta 96600
ttcaaaataa gtattaagca ttggcaaaga aaaaatagta gcagacaatt acatgttcca 96660
tttgtaaaga tgattattaa ttagtggtct tgcaaaacat tggagaaaat ttgctgaacc 96720
atcacattca taaatattaa aaccacccat tagtgaaaat ctttttacta aacttcacaa 96780
ctgatagtca aataatgttc agtttttctc cattgcaata aaaaataaag gcttttgcct 96840
tcagatcagt ctctgggcct tattaattca gtcagccaga agccacatgg aaatattttg 96900
ttttgttaaa agccagcttg ccctcatgat cttttaaaat cttttaaaaa tcttccatca 96960
gccctctccc tgacttgaat tatggcagtg ctttctaaac tggtaaactc aatctccttg 97020
gtgtgcctca agatagagta cataaaccct ccttagaaat tgagctctca attctaaatt 97080
gcactctcca tgagagcaag caagaatgct ttgctttgta ttaagtggtc acaatattaa 97140
atataaccat agacagcact gtattttcta aacaccttat tttcttttaa tgactgacat 97200
aaattagatc ataagtatac aaatgcatat ctgttgtatt tttcagcacc atgtgttttt 97260
ttttcttttt tctgagttat tttcctgctt tcggcagcct tttctctcag gtgccttgtg 97320
atccacagtg gtgtgtgttc acactaacca aagcaatagt cttacctgcc agaaatagct 97380
gtgacattta aagagaggtc caggggaagg cacagtgctt aacatccaag tctgaagagc 97440
taatagtgaa attggggcat cagctacaga gagatttagg ggaagtaaca ggcaggttaa 97500
atattttatg gaaatgattt ctgttctgta tatgattgca attaacacat gtcaatctgt 97560
ttcattaatt tgttaactca tctattatgc tatgccatga agaaaataaa attggagttc 97620
tttatttttt tgagatggag tctcactctc ttgcccaggc tggagtgcag tggcaggatc 97680

tcagctcact gcaatctcca ccacccaggt tcaagcgatt cttctgcctc agccacctga 97740

gtaactggga ctacaggtgc gtgcaaccat gcctggctaa tttttgtatt tttagtagag 97800

atggggtttc accatgtggg ccaggctggt cccaaactcc tgacctcaag tgatccgcct 97860

gtcttggcct cccaaggtgc tgggattaca ggcgtgagcc accgcgcccc gccacaaaac 97920

tgaagttcta agcttcagtt tagatgctca ctaaatgctt gttttgcaat acctgactgt 97980

aactggcagg aatatgtttt gaaagtcctc attttccagg tatgcagatg aaatataggg 98040

gcattatcta ctatgtcaaa ttataatgat ttatcagtgg cacatgaaag tcgcctcaca 98100

tttcttaatc agtgatatac cattatgtca tgccaccttt taatgtaata tgtttacatc 98160

tttctttaga tgtaagcatt catttagttc atcacggtgg ctttcacact tactccaaga 98220

acgctatgag ttcctttgat gtgctcaagt ctcctgcccc agggagaaag ggagtggtga 98280

gcaggaatcg ctttaatcta tttacacaga tattttcttt tccatttatt ttaaaggaat 98340

ttttttaac ttaatgagta tgcagtgacg gtggtgatga tgatgatact aaggtttaaa 98400

tgattagata gtcaaatctg ggctggaatt gtaatactgt tttgactttt aatcttagag 98460

aagctccagt ctgcttattt tctgggcata aacacatgag aacaataaca cagttctgtt 98520

atctgaatgt tgttatattt tgtttgaaac attcagtgac tttcaaatat tgtatttgcc 98580

taagaaaatt caacagagtc agacattctc ttccaggtta aatttggtga gtctgctagg 98640

aaaataaatt ttgtgcactg gtcattctga tctagtggac gttctaataa aagcaccttt 98700

gtgctgccta cgtcttcact ttaaagataa gatacctggg tactcgacac caaattatag 98760

tttgagatct caaaaatggg atagggaaac cacagctcaa aaacaaaaat actagcactg 98820

gaaaagatag aactagtgaa gatgaatcat tctctagact ttaaattcag agatatcaaa 98880

attaagaaaa agtaggagga ataaaaaaag agggtaagca aaacaatata agtttgtata 98940

gcaagagggt ataaagcaaa tacaatattt ttcagaaaaa ttaaataaaa atagatttac 99000

ataacattgt ttttaatctc aaagatcaaa tttcaatttt catctcattt taaaacccat 99060

atgcacagtc tcctttatat acatcagttg ggtgtcaaag tgactttttt cttgtttcca 99120

aatacagtta tttttaaaat ttaattgtat gatttaggaa tttgaaagca agccagtttg 99180

cacacacata tgttattata tgtgtgcttt agacttggtt tttagttaat gtaacatgac 99240

agggccacct gagttatttg tttacaaact agctggaaag ccaccctgga ggagaaacct 99300

ggcaacaaaa tggtctgcag ctttgttatt gttatctata ggattggatg ccattattgc 99360

tgtaaaatag ttcacaagaa ctcagtctat gggaaagact caaaaattct ttgcctgtta 99420

aagaaaaatc aggatattgg actggttagt ttaactaaaa agtgatgata ctcagattct 99480

gcttggattc actgcttctc agcagttgtt ttgtttcttt ctaattgata ttttattttt 99540

cagagaaccc attataaaac tcttcttctt cccttaaaat cacaaccaca caacagcaat 99600
taaaacatgc tttgacgtaa gactgatatg gttttaaacc cagcttgact atcgaatttt 99660
ttactttagg caaaacacct ctgacattta tgtcttatcg tcagtaaaaa ggggtgatta 99720
acagttttac aagattattc aataaataaa tataaattcc tccttttcct tcctttcctt 99780
tcttcatctt cagcatctgc atgccataag ctcattttag ttctctggac tcatgttaac 99840
atgtcccacc tttcccaaat taaacatcat ctctgttatt ggctccattc ttttcctctc 99900
atttgagaca attctttatc aaccaacacc ctctctgctc tgtattgtga aactctgctc 99960
ctactacatt aacagtctct tggtttcttt aaaaagaaga caaaacaatt aaagaacaga 100020
agcaaaaaat ctactcaaat ccccaattgt taccctcaaa attaattgtc ccacccctag 100080
ctttctcatt gcacaactct ttgtcaaaat gttttctacc atcacagcct tcaatgatct 100140
ttctggttcc tttatctcct gaagtctgac ttctacctcc atctttttct ggactattca 100200
acacactttg agaaaaaaca tacttttgtt aaacaggtat gcatccctga agcataaaat 100260
acatagtact gaaagtgcac atgtgtggtt cttcccattt tttttacagc acttgaaact 100320
gacaagtagt agtaccaatt acttagtaaa agaccttttt catttcattt ctgaaatatt 100380
gttattttcc tttttcatct tccatctctg actacacctc caattttacc tctttgctgc 100440
cttccttcct aagaaagttc ttcatgcaat gccatcttgt ttttcttcac ttgcctcttt 100500
ttctcacttt aattttatga actctgatga cttacctctg tagtgtaact actcaaaata 100560
tgtatttctg aagtctcaac tccaatctca tattttcaac ttatatttat ggaggcatct 100620
cagactcaac ctacctaaaa aatggcttat ctgccctaaa atctactttg ttcttttttt 100680
ctctactgct aataattatc ttcctagttg gtcaagctca aaacctaatc atttttactc 100740
cttgtccctg tgtcagctgt ccacattcaa gcagcgtatc atttctgcac atttttcaag 100800
caagtcagta actgcctttt gtttgggact gtcttttcat atagtgaaca gccttggaag 100860
atagaaatca tttctccttc taaaacaaaa ggcaggtgtg cttgcagcct tggatagagg 100920
tagtgcctct ttctaaagca aagggacatc tttactggcc attataaaat atccatgttt 100980
cctgagctct gcgttcctct tttctaatgc aacccactga gcatgtaggt gtcacctgag 101040
cttttctgtg ggaattgcgg cttgaggaat cagtgcaaga aaatcatgat actcttgcta 101100
atgctattaa tgtgagtagt aaagttaatt gtctctgacc cagcactatt gtgtctttgc 101160
ccagcactca aaagactggc aggcttgcaa gtaggacaaa atgttagatt tttcacagtt 101220
cttctgctta taagtacttg ttaaaaccaa ttaaaacaca acttgtagtt tgcacctata 101280
attttgtagc atttgcttct tatctatgtc actaggatgt gcttagtgac agacccatct 101340
101340
atcatctatt actcaagttt ttggctgtat tcctaggcaa cagagagaag gggaacaaac 101400
aagaggacct gtgcacagtt tgagaaaggc aaaacaccga gcttaattgc agacttgaat 101460

gtagctagca aacgaagtaa ggcaaaaggt tcctttttt tttttttaga tggagtctca 101520
ctctgtcgcc agtctggagt gcagtggtgc tgtctcggct cactgcaacc tccgcctcct 101580
gggttccagc gattcttctg cctcagcctc ccgagtagct gggactacag gcatgtgcca 101640
ccatgcccag ctaactttg tatttttagt agagacggag tttcaccacg ttggccagga 101700
tggtctcaat ctcttgacct tgtgatccgc ccattcggcc tcccaaagtg ctgagattat 101760
aggtgtgagc ctccgttccc ggccaaaagt ttccattttt taaatagttg ggtttttagt 101820
ttcgattctt tccaaaaaaa ggttttctta aaaaaataaa attagcaata agatgaaata 101880
taacaacaat ataatcttat taagacaata tatgatatac atttatcaaa atacttatat 101940
tttcaaaagt gcttaaaata atctagcaca tagtagatgc tcagtaaata tttgatatta 102000
tgactgtgca tgggtcatta taggctactt tatgtatatc atttcattta gtacaacatc 102060
actctgaaaa atgttttatt gttaccgttt ttcagttgaa acatttacgt tgctcaagat 102120
ctcactggta ccatctacta ttaggtcagt ctgccaccaa atctcatgct cttaaatgcc 102180
ctttttctcc tgagcttcca acaaatagtg tactgtatat aattgttgaa gggaggggac 102240
tgtgagacaa aatatttaga gtgaatgtgt agccacaatt tcagttcctc aacaaagtga 102300
taaaattagg aatcatcctc aatatatatt cttccaacac acacacacac atacacacac 102360
acacacacac aaataccaca agcccacttg aatgcacccc acctacacat tgcaaccata 102420
gagacaattg cagcattaaa tacagaatat tctgtgtgtt gtttgtttgt tctccctttg 102480
ctacaaaaat cagaatttct actcaataaa cagcaaaggg agatacaaat gaaccaaatt 102540
aaagaaggaa aaaatgttga aaaaattata tacagaacta tgtattgatt tattgagagt 102600
tcagtaatgt aatccagaaa taatggatgc cttaaaagta attaaaagaa tgcaaataaa 102660
catttagtgc caattaaaga aaaagaaata caacattaga caaaataaaa gatattcatt 102720
tgatgcaatg aggaaataat cttttattcc tctttaaatt ctctgtggaa taaggcatgg 102780
ttataaataa ataaacatct gccccatgga cttaatggat cgttatattt tattgcgata 102840
atcataatga aattgttggg agggattagt atctctagtg taatgctaag aaagataaag 102900
cctgtgccca ggcaaaagct ttcttggttg gtcaaaaggt ttgaagacat ttcaaactat 102960
tctaaaacaa acaaacaagc aaacaaacaa aaaacataca atgtctttgc cacatattta 103020
ggaaacaaaa tgaacaattt atttctgaca acctcatagt ctttgttctg tcagaacaat 103080
aatggaaagg tctaaaccag aaaatgctat gcattgaatt tataataaac tattttttcc 103140
tgtaacaaaa aattgataaa cttgatattt gcagatttaa tgattatgtg tttaaaaaaa 103200
atctggtttt tgcccttgca aaaaatcata tatatacaca tagatatgta tgtgtgtgtg 103260
tgcatagtat atatatatgt atatacatat atatacacac atttatatat ataaacattt 103320

cctttaacct cctattttat tccaataaaa atattggtat tagagatagt tctgatattt 103380
catcatgaat agttaacatt gcatttggaa aggattaatt tttttgaaac gtaattttac 103440
cttaataagt agcccagcgt aatattttag taattacaca gatttttttt tcaagacatt 103500
tgacaactaa tattgcataa tagttaagag tgtgggcttt ggagccagac ttcctatctc 103560
tgttcattca ctgataaaat ggagacagta gtaacttcct caaagagttg ttttttaaga 103620
tcaaataatg catataaaac tcttgaaatg gtaccaaata cagagtaagc accaaataaa 103680
cattaactgt tattgttatt ccatgtccga ataacacaga aaagtaagaa ttttaatatt 103740
tcatttgaat gaccttttaa ggatacacct agcccattat ctttcttgat aatcttgtaa 103800
gatgattcct tttttatctc cgatctgttg aggcatggat agaggttttc agagaaaaca 103860
ttttctaggt aactgaaaga aagtagcaac aacaaactgt gacaaaactt aacaatgaga 103920
gaatttacaa gatagaataa ttgcaactcc ttttgaaatc aaccactatg gtcctctggc 103980
tgggatagct aagcaaagat attccagcct gaaggttgag atctacttga agagttttct 104040
atccagattg tgagggcccc tcaaacttca cttagtatct gtttctatta gtatggaaac 104100
ttctggaacc ttgtggtatc acattcactt gactacttta ttcctgctct agctatctta 104160
aagcctttct taatctttta tcttttagag aagatacttc taggttttaa atccaccgat 104220
cttgaagcta ttgccttcac tctctgcttc agagcccatc cttttgtata tgagtagttt 104280
gttttgccta aagtactttc tcccagtcag attttaagtc cagtttctca tctgtttttg 104340
agagcaaact cctgggcctt ggctcactaa catcttgaca gcatatttct tctttcctat 104400
gggcttttca gcattccctg ggtttttcta aaatatgaaa gcagactctt tatctcttac 104460
tttgtcaaag cctaccctcc ccactgattt ctcacccagt tgctagtttt aagacctgcc 104520
tctggccggg cgcagtggct cacgcctgta atcccagcac tttgggaggc caaggtaggt 104580
ggatcacgag gtcaggagat cgagaccatc ctggctaaca cagtgaaacc ctgtctctac 104640
taaaattaca aaaaaattag ccaggcgtgg tggtgagcgc ctgtagtccc agctactcgg 104700
gaggctgaag caggagaatg gcgtgatccc gtgaggcaga gcttgcagtg agctgagatc 104760
gcgccactgc actccagcct gggcgacaga gcgagactct gtctcaaaaa aaaaaaaaaa 104820
aaaaaaaaaa aaaaaagacc tgcctccaaa tatcattgta tttgcaaaca tgaaatgact 104880
tattgattct gagctcagca caagagcaaa cctttctcag cttgacccat cttcacatcg 104940
ttaatgtctt attcagtcac tacccaaggg gctgaccttc aagattctaa tccatgaaag 105000
cttaaaatag taaacaaatt tgaatatagt ttaacataca taataaattt tatttctaga 105060
agaggaggat cagcccttag acatgaaaag taaaaatagt ttattcccag atttcccttt 105120
gtgcattagt atattcaacc gagtctatcc aagtaacagg acaaaaaaag ctggcagttg 105180
ttgctgcgct gtgaagtctt attaggtgag tcagctaatt atatggcact accataaata 105240

cagcaggcac tgccctgctt gttaggcttg ccaaggaaaa taaggattta aagcagcata 105300
ctacctcttt gctatataat gacattttct tcttaaaaat gattttgcac caattcctga 105360
tttatccacc aattattttt taatttatgg ttgaatgtat ttaaacctga attcagagat 105420
aaaactagta aatagctccc caaaataacc ccaaatatat ttaatatatt agctttactc 105480
tctcctccac tgccaaacct ttaaaaactg aaataaattg tttttatttc atcttttctc 105540
tttttctctc tctctaaggt gattgccaag actaaagaaa cagctagaag ggcaaaagac 105600
aagaaaatca gtaagatagt aacagattat ccaaagtaga gcacggctca ggtgcagtgg 105660
ctcatgcctg taatcccagc actttcggag gctgacgcag gaggatcact tgagtccagg 105720
agtttgagac cagcctgggc aacataatga aacttcatct ctataaaaaa aaaaaattta 105780
aatagccgag catggtggtg taagcctata gtcccagcta tttgggaggc tgaggctgga 105840
ggatcacttg ggcccaggag ttggagacta cagtgagcta tgattgtatc actgcattac 105900
agcctgggca atagggcaag accctgcctc taaacaaaag ataaacaaag tagagcataa 105960
atggcttcta aatatatgtt atttatgtgt aagactgggt tctctaaagg tatcatttaa 106020
ttaaaataga tttgcattct caatctgtag gtatggatta tgtataatgt atttaagata 106080
tgacttacag cgttcaccaa tgtgactatt cccaagtgat ccagatggct gatgacatag 106140
taatttgtac atttgctgag acctgatctg agtaggtatg taacataact gagggagagc 106200
aagtccattt gccgaaagaa agcctagcat atgacccagg agccacatct tcactcagcc 106260
ttgttgctag gtttggctta gcatatataa tagcatagca tgtataattt atgacaaaaa 106320
attatacttt gcacttttta attagaacat tcaaaatgat ctcaggaagt ggcaccagag 106380
atcatcagtg gtctactgta cttcgtgtgt atgtgtctgt gagtatgtat gtgtttgtgt 106440
gtgttcccac attctaaggc atgtctttta caggttagta gaaaatgttg atagaaaatt 106500
atagatttca acatctaaaa cacagtaggt cactacattg ttaaaacttg gaatttttta 106560
tcttgttgta aagtcaggcc aaccaaacct aaaatactgc tacattgaaa tagtgcaaaa 106620
tattcaaaat actatagtta tagatttggt agtaggactg taccagacct gtcactctat 106680
acaagactta tgccttgccc tttcacttac ctgttccctt ttacatctat cttactagat 106740
gtaatgctat aaattatatt tctaatatat tataatttat catgtattat aatgtatcaa 106800
atattacaaa ttatgttgca actcccctta cctttcgtct gcatattgcc tcagaaagaa 106860
cagatggatc caacagactt caaccacagg ccccttagtga caaatagctc ttaatgctgg 106920
gcttgccact ttgatgcatt tctaaagtta tagaatgtta aatgcaccaa gtcctttggt 106980
cattttattt ctaccttaga tctaagccat aactatactt tcccaaaaat taaagtttga 107040
attttaactt aaccatatat aattggaaaa ggaggttggg ttcgttaagt gtaattttat 107100

catgctttat tatcctttgg gcattggata cagcagaaca tgccaatttc tatggcttct 107160
catgtgacag aatatactta ctaggatgca attaaatact cctcagagta tgtaaacaat 107220
aaatgtaatc attacattat ttttatattg ttctttctta tgcataatag taagactgaa 107280
aatatagtgt tatttctgaa atatgcatat tgttttgctt ttgatgatta aataacattg 107340
tccaaagttt taggtttttt gaaatcttat attttttaac aaaatatcta gcctttccaa 107400
aacaagacct caataattcg tttaagaccc agagttgttc ctctccacat agatctctta 107460
aaaaggcaga ggatttatga cctcaagaga aatcagagta tccaaagttt gctttaattc 107520
aatgttttaa aaataaaatt ccttagattt tatcaaaaat tgagattagt ttgattttga 107580
atcagatgcc ctttgctccc caccccaaaa tggcattatg agcagactag gaattgataa 107640
tagaaaattg aacatatgaa atatatcttt accttgcttt ttaacaaggt attcatgtct 107700
atcgccttca tttttaagtg catcaataaa atacatggta attctcttag tgaaatatac 107760
tatctacact atgtacacac tcccctgtct gaggtagaga agtagagaat attcacattt 107820
ttgaaacgtc tatgctattt ttatttaaat acgagttctg ggcttgattt cattttggaa 107880
cacgggtgtg tgcttaagtt gaaccttttt ttcctcttaa gtcaaagttc ttttttagtt 107940
tcttctttta tctttttggc tactatctct ctccttcatc ctcctggtgt gagttgttga 108000
gtgaaggtat taattccatt atttgaggct aagtgacatt gttcaataat gcagcaaaac 108060
aatggttcta cccaaaatat cttcaagtgt aaaagcagtg ggcaaaagag aaagtgcgct 108120
tctgctgctt tgaatgttta aggctgtgaa agttgatcac acaaattggg tcattcttgt 108180
tatacccaac taaaacaatc aagaagcctg ggaggaaaag cattcaagaa acatcacatt 108240
gctccaaaag tgtaattttc tacaagtccg catgctgagg ctgcctgttg taacctggga 108300
ccaatttttt ctgtaactgc tgaaaaaact tgctgcagct ctaggactaa ttttgcccac 108360
cactgtcact caccaattga agcttactag ctccccagaa cctttctagt gccaatgaac 108420
tttctcaaag agcagcgtgt atcatttctc tttttcagaa cacctccaac ctcctctttg 108480
ttctttgggt ataccaaaga ccaaccagcc ttgaatttca atttttcttc ccacataaaa 108540
gttttaattt agaaatgtat ctctacattt ctaactttga caaagcatag ataccagata 108600
attgatgaaa ccttgctatt ttaacgatca ccatggatta cttcccagtg tcttcagata 108660
accctcaaca tttgccaaca tttgatggac ttcaaaatga gcatatcttt tttaaaaaaa 108720
attattcaca ctgacagcaa gtacattggt atactctata ttaaattata ccacagggtt 108780
tacaaacaat tggtgatgtc gggcagtggt ttccaaggaa catacttaac aagacactca 108840
caaggcccta caaacctgca tttttaacaa gggccctaga tgattctaga agagtgtggt 108900
ttggaaagca atttttgcct ttattatgtg tcattttaaa tatatttaaa attaaagtta 108960
taagtcatag aattgaataa agataatttc cttacagaaa gtattactag gtatctaaat 109020

acaatatggt tcaaaacagg aaatttaaaa agattatgta aattctgtag ttgtattcct 109080
aaagacagta gctgaaattt tttcctactt ctccttgtat cacttccctt ttccttcact 109140
ttcacttccc tggaattgta cttcccaata agctattagc agtgaaggaa gcttcgtctc 109200
atgatctgtt ttatagagca cttcagctgg gacgagtacg aaatgataat cagttatatc 109260
agctattcaa ccctacaggt ttatttaaaa agaacttgaa taagcttttt agggagaaag 109320
aggtcagtct cagccatttc tgtttcctaa tatagctttt aagtctttcc ttattagcaa 109380
tgagggtcat tccattgtaa ttttttgata accatttttc tttctgtgtg tcaaatgcag 109440
atataagata ctgaactgag tctatttcac tgttcgtaaa acaatcccat ttgaaaaaaa 109500
aaagtctaca gctattccag ggatagggcc tagtagagag agaataaaag gtattttctt 109560
actatgtctc tatatcctac cctgtaggtt ctcttattaa gcatacaggc atataccaaa 109620
atccagacgt ttttctcatt tattttattg ccctaacata ttctgggtta atataatatc 109680
ataatgaaaa tttgagaaaa aattgatttt ttcaaaagtg tttaacattt gttatattgg 109740
tagttttttt tcttgtttgt ggtaaaaata aatagaaggt gcacttcaca ccttcaagta 109800
tgattatatt ttgaaaacaa gtcatgaata ctcataaaat gcaaatttta atgttctttt 109860
tttgttacag ccaaactata ttaggcacag ttgtaaattg gagttgaaat ttaatatttc 109920
tttatagata acaatgtttt tagaaatagg tttatgaaac agtaaatata caggtatagg 109980
gataaaattg tgtctgatgg tcatatgaag tgtttgttgt tatattctcc ttggaatagc 110040
tgccaaatat tttagtatgc ttaaaatcta cgaatgtgat agagtcaaca aatttagatc 110100
acatattcag aaaaacatag ttagagaact aactattgaa atgagcatac agcagtcttc 110160
ctttatctac agggatacat tctgaaaccc ccactaggac acctgaaatt gcggatagta 110220
gcaaacccta catatactgt tttttccaat gcttatgtac ctatgaaaaa gtttaattta 110280
taaactaggc acagtaagag attaacaaca ataactaata acaaaagaga acaattataa 110340
taatatactg taataaaagt tatgtgggta tggtctcgct ttctctttcc ctctctctct 110400
gtctctaaat atcttagtat tttggggttg caattggtgg tgggcaactg aaaccatgga 110460
aaacaaaacc acggataaaa ggagactact gtatatactt tttaaaactg atgaaatatt 110520
aaactcatgt ttcttctata tcccacccat ttcccccacc caaacctaga tagatatctt 110580
atttgatctg taaacattta attaatttgt aaaagttaag aactttttga agtaaaactg 110640
caatatatca tcacacctaa agaaataaac aataattctt aaatatcaag tcagtgttca 110700
aatttcccca actacctcat atgtgttttc catttgctta tgtagggttc ccaatgagaa 110760
tgaaataaag ttcttaggtt gcaattggct aatgctctct cacttctact ttaagcggca 110820
ggttcccact aacttctttt tagttgcaat ttacttattg aaattagacg tattctttgt 110880

cttgtgtagt ttctcacagt gcaaaatttg ctgattgtag ccactgttgt aagcaatgaa 110940
catgtttttc accaccttat atttgctgta agttgtcagt gatagttaaa tgttaatcaa 111000
attcaaattc ggatcacgta gggcttttct ttttttgttt tctttttcta tttatatatt 111060
tatttattta ttttgagacg gagtctcact ccgtcaccag gctggagtgc aatggtgtga 111120
tctgggctca ctgcaatctc cacctcccgg gttcaagtga ttcccctggc tcagtctccc 111180
gagtagctgg gactatagga gaaccaccac gcccggctaa ctttttgtat tttagtagag 111240
atggggtttc accatgttgg ccaggatgct atagatctcc tgacctcacc gatcatgtag 111300
gacttcaatt gtcgaacaaa cgaaccttta atagcagtta caccattagg atgacctgat 111360
ccaacatcga ggtcgtaaac cctattgtcg atttggactc tagaatagga ttgtgctgtc 111420
atccctagtg tagcttgttc ccacttgatg aagttattgg atcagtgaac aatagcccac 111480
ttaaactagt acagtcttag tttaagatgg tgatgtgtat gtacttccat cagagggcac 111540
ataatacagt aaatcctcac ttaacttcat caatagtttc tggaaactgt gacttgaagc 111600
aaaacaacat ataacaaaac cagttttacc attggctaat tgatataagc aagaattaag 111660
tcctatggca aatttctgga cacaaaaaca ccatcaaact cctaaataaa gataaatcac 111720
ttctgacatt aaacattgaa attaatgtga gctatatata cgtttaagaa agattaatac 111780
aaacaagtca aataacttac ctaattattt cggtggaggc cgcaggtggt tggagcctat 111840
cctggcagct cagggagcaa tatgggaacc caccccggac aggacgctgt tccattactg 111900
cagggtgctc ttgtacacac ccactcaccc aggctggaac catgcagaca cacacactca 111960
cctaacctac acatctgtgt acatccttca aagttcagcc aaataacata taaacaaatc 112020
cagtaatatc catcagtctt agttccgtca taacaactcc tttttgatca tcaaacaaca 112080
aacagggtag gtctgccata tttacttgtc tggtccatat caaaattttc taacaaatta 112140
tattagaaaa tcaaatctct gtcagtttca aaatcatgga aaaaaatttg ccttatttcc 112200
cttatacttg gatatcctaa cagtaatcta aatattaatg agaaagttaa tgatgtcgtt 112260
tccttctccc tgttgtaaag aaggttttgc tgtcccgttt gatcactaag actaattgac 112320
actcagaaaa agcataggaa acttctcagc atcacaaaag ctctgtcatc tagagaagct 112380
aggacttgag ctcaagtcct gtgacatgga aggccttgtg cctagccatc ctgcagcaga 112440
ggcgtatcta ccaagaagtg aaacactacg aaaacagtat gtttactcca cattttaaag 112500
tgaggtagtt tggggtggtt catattttat ttaatttata tattatttgg atttttttta 112560
gtttataaaa agggcattgg caagggcaga atgatctgta agcttctctg cccacctacc 112620
ataagcatga tctttagtgt gaccttttct tactgttagc cattttctta tacttctgcg 112680
tccctgtcag tcacttccat gtgaagacat ggggaagctt ttttacatca gacatgttgt 112740
tgaaaatcag ccgcgttggc tgagggatta tttgatctct ttctccaagt ccctttaggc 112800

tcacattgcc tctctgttct ttgaattttc acttaccttt atcttcttat aattactttg 112860

ctgaaataaa tgcaaagcaa caaaaggtat ttagtgaaga ataccaacaa agccatgacc 112920

atttcaggct gagttttgta gtattctttg tctaggaaga gatacctaga aaaattttct 112980

gaccatgtat ttgattattt tccttcaata tgtatagtct cagtcttcaa atttcagaaa 113040

agaatttgtt tcttcattgt catttaaaat taatgtgtta aatatgtatg cttttacatt 113100

ataagtggtt ataaaagtta aacacttaga aaaaaagtca aaataacata catactatcc 113160

aacaaaataa ctttcatatt ttattgtgtt ttcttccaaa ctttttacct ttgcgtctga 113220

attctgtgta ggttgtatct ataatataga caacacttta tagcctgcta aatattatac 113280

cataaatagg tagttgttac ataattctca ggtaatagta atacaggtct ttatcataat 113340

ctactgagta gttgaatgat aatttttttt aagacaaggt ctccctctgt cacccaggct 113400

agaatgcagt ggcatgcaca tggctcactg tagcctctac ctcccaggct caagtgatcc 113460

tcctgcctca gcctcccaag tggctgggac tgtaggcatg tgccaccatg cccagctatt 113520

tatttgtatt tttagtagag atggggtttc attgtaacag cccaggctgg tcttgaactc 113580

ctggactcaa atgatccacc tgcctcagcc tcccaaagtg ctgaaatcac aggagtgaac 113640

cactgcaccc agcaataatt ttttaactct tcattattca ttgaacattt agttaacaat 113700

tctaaaaatt ttgtttcctg ctgtcattga tcttgtgaaa aatatctttg gactatagct 113760

gtggattatt tcctaaatag taaattactt gagcaaaaag tttacatact ttgagggttg 113820

ataacccatg ttgccgcaat gtttccccgg aggcattgtg gagtttagaa tgccagtagt 113880

aatattaagg tgtgccattt tcaagatccg tggccaacat ccctatatgt aagatttttc 113940

caaaacatgg ttctgatttt taaaagtgaa aaatgctact tcatcatgtt ctttttgtgc 114000

ttcttacttt aaatattaga atgaagaagg agccccacag gaaggaattc tggaagatat 114060

gcctgtggat cctgacaatg aggcttatga aatgccttct gaggtaggag tccaagctga 114120

atctttctaa caagacagta ccaaaaacct gtcattgtca catttctctt tcattagtgc 114180

ttagtgagaa tcatttgctc tctacatgct cattacgtgg acaacttgca agttaagaat 114240

agtttttaca tttttaaagg gtccttaaaa aaaaagagga ggaggaagat gaagaagagg 114300

aagaaaggat gtaaaagaaa tcatatgtag tccacatagc ttaatatact tactacttga 114360

ccctttacag gaaaagttta ctaacccctg cattagagaa tatattttta gaaactttac 114420

attctaaaat aaatttctaa atggaaagtt agggaaatca atggaatgcc aaaggaaggt 114480

tattattttt tgccatacat gtccaatggg atgacgcata gtaaaataaa agttacccac 114540

acaagttata gaataaaaag ataaatgcat gatttgcgac aattgatata ttccagtata 114600

atgttttaaa caacacaata tgattgttaa ttttattttg attgaaaatg aaagtatctt 114660

taatagaaaa tgtatcaaaa gggaaattag aaaatactgt tagatgaata aaactggccc 114720
aagaagaaac agtaaatctg aatagatttg taacacagcg aatagattaa attagtaata 114780
aaaaaaaaaa cctacctgca aagaaaatcc caggccgaga tggcatcact ggtaaattct 114840
accaaacatt taaagaggaa ttaatactaa ttagttaaca ccaattaata tctcttacaa 114900
aacagaagag gagacatttc ccaactaatt ttgtgagacc aatattaccc tgataatcaa 114960
aaccaaatga agatatcaca agaaaagaaa ctatataatg gctccattaa aaattgagtt 115020
caagtatgtt gtagtttggt tatgtattat tcctcacggc attattaaaa ggcatgtcga 115080
ggatgggcac agcagttcac acctgtaatc ccgcactttg tgagccaaag tggccaggtt 115140
acttgaggcc aggagttgga gaccagtctg gccaacatgg tgaaacccca tctctactaa 115200
aaatacaaaa attagccggg catggtggta cacgcctatg gttccagcta cttgggaggc 115260
tgaggcatga gagtcacttg aacccaggag gcagaggttg cagtgagctg agatggcacc 115320
cctgcactcc aatcttggta acagagcaag actgtctcac acagacacac gaaaggcata 115380
ttgataataa ttcaacttat agaaattgag attaaattgt ttgtttgcct aataagaatt 115440
tccaatattt tggggtcttt tatgcaagac acagtactaa acacaatgga aaactataga 115500
gtaattgaca ttaccaggac ataaggagtt tacagtctgg taggtttgat gaaaaaaaat 115560
agaaattcat tcattcattt cttcattatg attcctttaa caaacataat tgattgtctt 115620
cgatgtacca ggcatcacag gagcaaaaat atataagaca tactaaaaag taaaacattt 115680
taaagatctg tttcaatcaa tcaggagaag ttttattgag gaggtaatgt tgatctgggt 115740
gggaaaaggt aagagatata gtaggtcaaa acaaacagag gacattctgg cacaagggaa 115800
tatcagaagc aaaggcatgt atgtctgagc atgcaaatgg atatgtctga gaacagtgaa 115860
taattatgac tcaagcttag gaacaaggaa aatggtgata gattgaattt gcagctatgg 115920
gtcaaagaca agttatagag tattaggata atcttgtcat ttcagcttgt attctattca 115980
gaaaacaact tgagttattg aagttatgct tatttgtttg tttttaagca gaatcctgat 116040
attattagag ttgctcttta ggaggaataa tctgatccct ttaattaaat ccattaatat 116100
ttgtgttgtg gatgctatcc agatactgta tggagagctt gaggtttgaa atacaagtaa 116160
taattgaagc catagatgaa gacgaaattt tcaactggga gagtgaaagt agggaaaatg 116220
tatcttgcct tcaaacatct taatttcctt ctgagaatta gagcatctta gtctggaaaa 116280
ggctttatag acagcttgat tttgttctca cattttacag gtgaagaaac tgagaaccag 116340
acagtccaac ttatttgtcc taccaaacta ggtatatgat cattaaatgg tgcatccgga 116400
tcagaaccta gatattttaa ctctgactac tactgtaatt cacttttata tcagacaaga 116460
aagacacaac tattaaaaat aagataatat ttgctgcaga atatttgcaa aaacattgat 116520
tgtaaatttt agtgtaagtg gggagccatt tcctatctca ttggctgtca gtgctgatgc 116580

gtaattgaaa cttatactaa cagtgtgtgc tgtctttttg atttttctaa tattaggaag 116640
ggtatcaaga ctacgaacct gaagcctaag aaatatcttt gctcccagtt tcttgagatc 116700
tgctgacaga tgttccatcc tgtacaagtg ctcagttcca atgtgcccag tcatgacatt 116760
tctcaaagtt tttacagtgt atctcgaagt cttccatcag cagtgattga agtatctgta 116820
cctgcccccca ctcagcattt cggtgcttcc ctttcactga agtgaataca tggtagcagg 116880
gtctttgtgt gctgtggatt ttgtggcttc aatctacgat gttaaaacaa attaaaaaca 116940
cctaagtgac taccacttat ttctaaatcc tcactatttt tttgttgctg ttgttcagaa 117000
gttgttagtg atttgctatc atatattata agatttttag gtgtctttta atgatactgt 117060
ctaagaataa tgacgtattg tgaaatttgt taatatatat aatacttaaa aatatgtgag 117120
catgaaacta tgcacctata aatactaaat atgaaatttt accattttgc gatgtgtttt 117180
attcacttgt gtttgtatat aaatggtgag aattaaaata aaacgttatc tcattgcaaa 117240
aatattttat ttttatccca tctcacttta ataataaaaa tcatgcttat aagcaacatg 117300
aattaagaac tgacacaaag gacaaaaata taaagttatt aatagccatt tgaagaagga 117360
ggaattttag aagaggtaga gaaaatggaa cattaaccct acactcggaa ttccctgaag 117420
caacactgcc agaagtgtgt tttggtatgc actggttcct taagtggctg tgattaatta 117480
ttgaaagtgg ggtgttgaag accccaacta ctattgtaga gtggtctatt tctcccttca 117540
atcctgtcaa tgtttgcttt acgtattttg gggaactgtt gtttgatgtg tatgtgttta 117600
taattgttat acatttttaa ttgagccttt tattaacata tattgttatt tttgtctcga 117660
aataattttt tagttaaaat ctattttgtc tgatattggt gtgaatgctg tacctttctg 117720
acaataaata atattcgacc atgaataaaa aaaaaaaaaa agtgggttcc cgggaactaa 117780
gcagtgtaga agatgatttt gactacaccc tccttagaga gccataagac acattagcac 117840
atattagcac attcaaggct ctgagagaat gtggttaact ttgtttaact cagcattcct 117900
cacttttttt ttttaatcat cagaaattct ctctctctct ctctcttttt ctctcgctct 117960
cttttttttt ttttttttac aggaaatgcc tttaaacatc gttggaacta ccagagtcac 118020
cttaaaggag atcaattctc tagactgata aaaatttcat ggcctccttt aaatgttgcc 118080
aaatatatga attctaggat ttttccttag gaaaggtttt tctctttcag ggaagatcta 118140
ttaactcccc atgggtgctg aaaataaact tgatggtgaa aaactctgta taaattaatt 118200
taaaaattat ttggtttctc tttttaatta ttctggggca tagtcatttc taaaagtcac 118260
tagtagaaag tataatttca agacagaata ttctagacat gctagcagtt tatatgtatt 118320
catgagtaat gtgatatata ttgggcgctg gtgaggaagg aaggaggaat gagtgactat 118380
aaggatggtt accatagaaa cttccttttt tacctaattg aagagagact actacagagt 118440

gctaagctgc atgtgtcatc ttacactaga gagaaatggt aagtttcttg ttttatttaa 118500
gttatgttta agcaaggaaa ggatttgtta ttgaacagta tatttcagga aggttagaaa 118560
gtggcggtta ggatatattt taaatctacc taaagcagca tattttaaaa atttaaaagt 118620
attggtatta aattaagaaa tagaggacag aactagactg atagcagtga cctagaacaa 118680
tttgagatta ggaaagttgt gaccatgaat ttaaggattt atgtggatac aaattctcct 118740
ttaaagtgtt tcttccctta atatttatct gacggtaatt tttgagcagt gaattacttt 118800
atatatctta atagtttatt tgggaccaaa cacttaaaca aaaagttctt taagtcatat 118860
aagccttttc aggaagcttg tctcatattc actcccgaga cattcacctg ccaagtggcc 118920
tgaggatcaa tccagtccta ggtttatttt gcagacttac attctcccaa gttattcagc 118980
ctcatatgac tccacggtcg gctttaccaa aacagttcag agtgcacttt ggcacacaat 119040
tgggaacaga acaatctaat gtgtggtttg gtattccaag tggggtcttt ttcagaatct 119100
ctgcactagt gtgagatgca aacatgtttc ctcatctttc tggcttatcc agtatgtagc 119160
tatttgtgac ataataaata tatacatata tgaaaatatg tatttggttt ctgcctccag 119220
ttcttacaaa gagctcctaa aacccttgta atttcctgag tagtaggggt gctagggtca 119280
tcttttgttc taatatttgg tctttgactc tgctttctga cagagctcct tagtccctgg 119340
gtgagagtag catcttctct tctaatgaag tgactcttgc tgggttcctg gatgggggct 119400
ggtcaccaga aaggtcaagc catgataaga agcttgaagc ttttggcccc attcacatct 119460
tctggggacg ggagagaaga ggagctggag attgagttaa taagcaacaa tgcttccatg 119520
atgaagactc cataaaaatc cctaaaagac aggattcaga gtgctttgaa ataggtgaac 119580
atgcagaggt gctgggaatt gtggtgtgtc cagagaaggc atgcaagctc cccacgcctc 119640
ccccatacct ttccctgtgc atctcttcca tctggctgtt cctgagttgt atccttttat 119700
aacaaactgg taatctagta agcaaactgt tttcctgaag tctgtgaatc acactagcaa 119760
attatcaaac ctgaggagag ggccgtggag accttggatt tgtagacaag tcaaacagaa 119820
gctatgagta acatgaggac tcattgcttg tgattgtcat cttcagtggg aaggggaaaa 119880
atcttgtaaa actgagtcct taacctgtgg gtcaatgcta actccaggta gatagtgtcc 119940
gatttgaatt acgggacacc cagttggtag ccacaaagaa tgggagaatt gcttggtgta 120000
gaaaacacac cccacacaca catgtggtgt cagaaatgaa ccggaaatat tgtgttccgg 120060
aaatattgag tgttgtgagt gagtgtatag aaagaaaaac agcgtttcct tttcactact 120120
agattaaaac aaacacactc atgcattcac acatctcaaa gacaactatt aattctcaaa 120180
gacagtgctg tctaaatcca tactgaggaa gaaaacacat tttcttttca aatctgtaaa 120240
cctgacagac tgcctctgtc cacacactaa tggaactctg tgtttcatct gaaatgtgtt 120300
catcccactt tgttctttct gtcttgggca gggcaagagt gcaacagggc tgacattttc 120360

atatgagctc tgtccctgtt attggctata ctttagacaa attattatgt gtcaaatata 120420

gatgtaagtg atttatcaat attaagtcat ttaattctca aaacaacctt aataggttcc 120480

attatgattc taattttaca cataagccaa aggaggcacc cacaggctag ataactttcc 120540

cacggccaca cagctagtaa gcggcagagc caagaggccc aacattacag caccacagtc 120600

tgtgctctca gccccttggc cacatagtgt cagagtgagg acacacagct atttaagaaa 120660

acttccagaa gtctaggaaa tggggtgata gccccacttt tctaggtata ataattagat 120720

atttgttttt cttcaggtac ctaaagaaaa tttactagag tttgagcctt tagtaagttt 120780

tgctagtaca tctgtttttc ttcaggtgcc tgaagacaaa catatacaca cacacacaca 120840

cacaaacaca cacaaaatgt gtatctatat atatgtgtac acatatctct catctctata 120900

tatatgtctc tgtatatcta tatatctata aacatatcta tatctataga tacatataga 120960

gagatttctt tttttttttt tttgagatgg agtcttgctc ttgccaccta ggctggagtg 121020

caatggcaca atctcagttc actgcaacct ccgcctccca ggttcaagcg attctcctgc 121080

ctcagcctct cgagtaggtg ggattacagg aacacaccac cttagcccga ctaatttttg 121140

tatttttagt agagacaggg ttcaccacgt tggccaggct ggtctcaaac tcctgacc 121198

<210> 2
<211> 3211
<212> RNA
<213> Homo sapiens

<400> 2
gccauucgac gacagguuag cggguuugcc ucccacuccc ccagccucgc gucgccggcu 60

cacagcggcc uccucugggg acaguccccc ccgggugccg ccuccgcccu uccugugcgc 120

uccuuuuccu ucuucuuucc uauuaaauau uauuugggaa uuguuuaaau uuuuuuuuua 180

aaaaaagaga gaggcgggga ggagucggag uuguggagaa gcagagggac ucaguguggu 240

guaaaggaau ucauuagcca uggauguauu caugaaagga cuuucaaagg ccaaggaggg 300

aguuguggcu gcugcugaga aaaccaaaca gggugug gca gaagcagcag gaaagacaaa 360

agaggguguu cucuauguag gcuccaaaac caaggaggga guggugcaug guguggcaac 420

aguggcugag aagaccaaag agcaagugac aaauguugga ggagcagugg ugacgggugu 480

gacagcagua gcccagaaga caguggaggg agcagggagc auugcagcag ccacuggcuu 540

ugucaaaaag gaccaguugg gcaagaauga agaaggagcc ccacaggaag gaauucugga 600

agauaugccu guggauccug acaaugaggc uuaugaaaug ccuucugagg aaggguauca 660

agacuacgaa ccugaagccu aagaaauauc uuugcuccca guuucuugag aucugcugac 720

agauguucca uccuguacaa gugcucaguu ccaaugugcc cagucaugac auuucucaaa 780

guuuuuacag uguaucucga agucuuccau cagcagugau ugaaguaucu guaccugccc 840

ccacucagca uuucggugcu ucccuuucac ugaagugaau acaugguagc agggucuuug 900
ugugcugugg auuuuguggc uucaaucuac gauguuaaaa caaauuaaaa acaccuaagu 960
gacuaccacu uauuucuaaa ucccucacuau uuuuuguug cuguuguuca gaaguuguua 1020
gugauuugcu aucauauauu auaagauuuu uaggugucuu uuaaugauac ugucuaagaa 1080
uaaugacgua uugugaaauu uguuaauaua uauaauacuu aaaaauaugu gagcaugaaa 1140
cuaugcaccu auaaauacua aauaugaaau uuuaccauuu ugcgaugugu uuuauucacu 1200
uguguuugua uauaaauggu gagaauuaaa auaaaacguu aucucauugc aaaaauauuu 1260
uauuuuuauc ccaucucacu uuaauaauaa aaaucaugcu uauaagcaac augaauuaag 1320
aacugacaca aaggacaaaa auauaaaguu auuaauagcc auuugaagaa ggaggaauuu 1380
uagaagaggu agagaaaaug gaacauuaac ccuacacucg gaauucccug aagcaacacu 1440
gccagaagug uguuuuggua ugcacugguu ccuuaagugg cugugauuaa uuauugaaag 1500
uggggguguug aagaccccaa cuacuauugu agaguggucu auuucucccu ucaauccugu 1560
caauguuugc uuuacguauu uuggggaacu guuguuugau guguaugugu uuauaauugu 1620
uauacauuuu uaauugagcc uuuuauuaac auauauuguu auuuuugucu cgaaauaauu 1680
uuuuaguuaa aaucuauuuu gucugauauu ggugugaaug cuguaccuuu cugacaauaa 1740
auaauauucg accaugaaua aaaaaaaaaa aaaagugggu ucccgggaac uaagcagugu 1800
agaagaugau uuugacuaca cccuccuuag agagccauaa gacacauuag cacauauuag 1860
cacauucaag gcucugagag aauugugguua acuuuguuua acucagcauu ccucacuuuu 1920
uuuuuuuaau caucagaaau ucucucucuc ucucucucuu uuucucucgc ucucuuuuuu 1980
uuuuuuuuuu uacaggaaau gccuuuaaac aucguuggaa cuaccagagu caccuuaaag 2040
gagaucaauu cucuagacug auaaaaauuu cauggccucc uuuaaauguu gccaaauaua 2100
ugaauucuag gauuuuuccu uaggaaaggu uuuucucuuu cagggaagau cuauuaacuc 2160
cccaugggug cugaaaauaa acuugauggu gaaaaacucu guauaaauua auuuaaaaau 2220
uauuugguuu cucuuuuuaa uuauucuggg gcauagucau uucuaaaagu cacuaguaga 2280
aaguauaauu ucaagacaga auauucuaga caugcuagca guuuauaugu auucaugagu 2340
aaugugauau auauugggcg cuggugagga aggaaggagg aaugagugac uauaaggaug 2400
guuaccauag aaacuucccuu uuuuaccuaa uugaagagag acuacuacag agugcuaagc 2460
ugcaugugucc aucuuacacu agagagaaau gguaaguuuc uuguuuuauu uaaguuaugu 2520
uuaagcaagg aaaggauuug uuauugaaca guauauuuca ggaagguuag aaaguggcgg 2580
uuaggauaua uuuuaaaucu accuaaagca gcauauuuua aaaauuuaaa aguauuggua 2640
uuaaauuaag aaauagagga cagaacuaga cugauagcag ugaccuagaa caauuugaga 2700

uuaggaaagu ugugaccaug aauuuaagga uuuaugugga uacaaauucu ccuuuaaagu 2760

guuucuuccc uuaauauuua ucugacggua auuuuugagc agugaauuac uuuauauauc 2820

uuaauaguuu auuugggacc aaacacuuaa acaaaaaguu cuuuaaguca uauaagccuu 2880

uucaggaagc uugucucaua uucacucccg agacauucac cugccaagug gccugaggau 2940

caauccaguc cuagguuuau uuugcagacu uacauucucc caaguuauuc agccucauau 3000

gacuccacgg ucggcuuuac caaaacaguu cagagugcac uuuggcacac aauugggaac 3060

agaacaaucu aauguguggu uugguauucc aaguggggc uuuucagaa ucucugcacu 3120

agugugagau gcaaacaugu uuccucaucu uucuggcuua uccaguaugu agcuauuugu 3180

gacauaauaa auauauacau auaugaaaau a 3211

<210> 3
<211> 3022
<212> RNA
<213> Homo sapiens

<400> 3
auucuggugu gauccaggaa cagcugucuu ccagcucuga aagagugugg uguaaaggaa 60

uucauuagcc auggauguau ucaugaaagg acuuucaaag gccaaggagg gaguuguggc 120

ugcugcugag aaaaccaaac agggugugge agaagcagca ggaaagacaa aagagggugu 180

ucucuaugua ggcuccaaaa ccaaggaggg agugguggcau ggugugcaa caguggcuga 240

gaagaccaaa gagcaaguga caaauguugg aggagcagug gugacgggug ugacagcagu 300

agcccagaag acaguggagg gagcagggag cauugcagca gccacuggcu uugucaaaaa 360

ggaccaguug ggcaagaaug aagaaggagc cccacaggaa ggaauucugg aagauaugcc 420

uguggauccu gacaaugagg cuuaugaaau gccuucugag gaaggguauc aagacuacga 480

accugaagcc uaagaaauau cuuugcuccc aguuucuuga gaucugcuga cagauguucc 540

auccuguaca agugcucagu uccaaugugc ccagucauga cauuucucaa aguuuuuaca 600

guguaucucg aagucuucca ucagcaguga uugaaguauc uguaccugcc cccacucagc 660

auuucggugc uucccuuuca cugaagugaa uacaugguag cagggucuuu gugugcugug 720

gauuuugugg cuucaaucua cgauguuaaa acaaauuaaa aacaccuaag ugacuaccac 780

uuauuucuaa auccucacua uuuuuuuguu gcuguuguuc agaaguuguu agugauuugc 840

uaucauauau uauaagauuu uuagguguuu uuuaaugaua cugucuaaga auaaugacgu 900

auugugaaau uuguuaauau auauaauacu uaaaaauaug ugagcaugaa acuaugcacc 960

uauaaauacu aaauaugaaa uuuuaccauu uugcgaugug uuuuauucac uuguguuugu 1020

auauaaaugg ugagaauuaa aauaaaacgu uaucucauug caaaaauauu uuauuuuuau 1080

cccaucucac uuuaauaaua aaaaucaugc uuauaagcaa caugaauuaa gaacugacac 1140

aaaggacaaa aauauaaagu uauuaauagc cauuugaaga aggaggaauu uuagaagagg 1200
uagagaaaau ggaacauuaa cccuacacuc ggaauucccu gaagcaacac ugccagaagu 1260
guguuuuggu augcacuggu uccuuaagug gcugugauua auuauugaaa gugggguguu 1320
gaagacccca acuacuauug uagaguggucuauuucuccc uucaauccug ucaauguuug 1380
cuuuacguau uuuggggaac uguuguuuga uguguaugug uuuauaauug uuauacauuu 1440
uuaauugagc cuuuuauuaa cauauauugu uauuuuuguc ucgaaauaau uuuuuaguua 1500
aaaucuauuu ugucugauau uggugugaau gcuguaccuu ucugacaaua aauaauauuc 1560
gaccaugaau aaaaaaaaaa aaaaaguggg uucccgggaa cuaagcagug uagaagauga 1620
uuuugacuac acccuccuua gagagccaua agacacauua gcacauauua gcacauucaa 1680
ggcucugaga gaaugugguu aacuuuguuu aacucagcau uccucacuuu uuuuuuuuaa 1740
ucaucagaaa uucucucucu cucucucucu uuuucucucg cucucuuuuu uuuuuuuuuu 1800
uuacaggaaa ugccuuuaaa caucguugga acuaccagag ucaccuuaaa ggagaucaau 1860
ucucuagacu gauaaaaauu ucauggccuc cuuuaaaugu ugccaaauau augaauucua 1920
ggauuuuucc uuaggaaagg uuuuucucuu ucagggaaga ucuauuaacu ccccaugggu 1980
gcugaaaaua aacuugaugg ugaaaaacuc uguauaaauu aauuuaaaaa uuauuugguu 2040
ucucuuuuua auuauucugg ggcauaguca uuucuaaaag ucacuaguag aaaguauaau 2100
uucaagacag aauauucuag acaugcuagc aguuuauaug uauucaugag uaaugugaua 2160
uauauugggc gcuggugagg aaggaaggag gaaugaguga cuauaaggau gguuaccaua 2220
gaaacuuccu uuuuuaccua auugaagaga gacuacuaca gagugcuaag cugcaugugu 2280
caucuuacac uagagagaaa ugguaaguuu cuuguuuuau uuaaguuaug uuuaagcaag 2340
gaaaggauuu guuauugaac aguauauuuc aggaagguua gaaaguggcg guuaggauau 2400
auuuuaaauc uaccuaaagc agcauauuuu aaaaauuuaa aaguauuggu auuaaauuaa 2460
gaaauagagg acagaacuag acugauagca gugaccuaga acaauuugag auuaggaaag 2520
uugugaccau gaauuuaagg auuuaugugg auacaaauuc uccuuuaaag uguuucuucc 2580
cuuaauauuu aucugacggu aauuuuugag cagugaauua cuuuauauau cuuaauaguu 2640
uauuugggac caaacacuua aacaaaaagu ucuuuaaguc auauaagccu uuucaggaag 2700
cuugucucau auucacuccc gagacauuca ccugccaagu ggccugagga ucaauccagu 2760
ccuagguuua uuuugcagac uuacauucuc ccaaguuauu cagccucaua ugacuccacg 2820
gucggcuuua ccaaaacagu ucagagugca cuuuggcaca caauugggaa cagaacaauc 2880
uaaugugugg uuugguauuc caaguggggu cuuuuucaga aucucugcac uagugugaga 2940
ugcaaacaug uuuccucauc uuucuggcuu auccaguaug uagcuauuug ugacauaaua 3000
aauauauaca uauaugaaaa ua 3022

<210> 4
<211> 3215
<212> RNA
<213> Homo sapiens

<400> 4
aggagaagga gaaggaggag gacuaggagg aggaggacgg cgacgaccag aaggggccca 60

agagaggggg cgagcgaccg agcgccgcga cgcggaagug aggugcgugc gggcugcagc 120

gcagaccccg gcccggcccc uccgagagcg uccugggcgc ucccucacgc cuugccuuca 180

agccuucugc cuuuccaccc ucgugagcgg agaacuggga guggccauuc gacgacagug 240

ugguguaaag gaauucauua gccauggaug uauucaugaa aggacuuuca aaggccaagg 300

agggaguugu ggcugcugcu gagaaaacca aacagggugu ggcagaagca gcaggaaaga 360

caaaagaggg uguucucuau guaggcucca aaaccaagga gggaguggug cauggugugg 420

caacaguggc ugagaagacc aaagagcaag ugacaaaugu uggaggagca guggugacgg 480

gugugacagc aguagcccag aagacagugg agggagcagg gagcauugca gcagccacug 540

gcuuugucaa aaaggaccag uugggcaaga augaagaagg agccccacag gaaggaauuc 600

uggaagauau gccuguggau ccugacaaug aggcuuauga aaugccuucu gaggaagggu 660

aucaagacua cgaaccugaa gccuaagaaa uaucuuugcu cccaguuucu ugagaucugc 720

ugacagaugu uccauccugu acaagugcuc aguuccaaug ugcccaguca ugacauuucu 780

caaaguuuuu acaguguauc ucgaagucuu ccaucagcag ugauugaagu aucuguaccu 840

gcccccacuc agcauuucgg ugcuucccuu ucacugaagu gaauacaugg uagcaggguc 900

uuugugugcu guggauuuug uggcuucaau cuacgauguu aaaacaaauu aaaaacaccu 960

aagugacuac cacuuauuuc uaaauccuca cuauuuuuuu guugcuguug uucagaaguu 1020

guuagugauu ugcuaucaua uauuauaaga uuuuuaggug ucuuuuaaug auacugucua 1080

agaauaauga cguauuguga aauuuguuaa uauauauaau acuuaaaaau augugagcau 1140

gaaacuaugc accuauaaau acuaaauaug aaauuuuacc auuuugcgau guguuuuauu 1200

cacuuguguu uguauauaaa uggugagaau uaaaauaaaa cguuaucuca uugcaaaaau 1260

auuuuauuuu uaucccaucu cacuuuaaua auaaaaauca ugcuuauaag caacaugaau 1320

uaagaacuga cacaaaggac aaaaauauaa aguuauuaau agccauuuga agaaggagga 1380

auuuuagaag agguagagaa aauggaacau uaacccuaca cucggaauuc ccugaagcaa 1440

cacugccaga aguguguuuu gguaugcacu gguuccuuaa guggcuguga uuaauuauug 1500

aaagugggggu guugaagacc ccaacuacua uuguagagug gucuauuucu cccuucaauc 1560

cugucaaugu uugcuuuacg uauuuugggg aacuguuguu ugauguguau guguuuauaa 1620

uuguuauaca uuuuuaauug agccuuuuau uaacauauau uguuauuuuu gucucgaaau 1680

aauuuuuuag uuaaaaucua uuuugucuga uauuggugug aaugcuguac cuuucugaca 1740

auaaauaaua uucgaccaug aauaaaaaaa aaaaaaaagu ggguucccgg gaacuaagca 1800

guguagaaga ugauuuugac uacacccucc uuagagagcc auaagacaca uuagcacaua 1860

uuagcacauu caaggcucug agagaaugug guuaacuuug uuuaacucag cauuccucac 1920

uuuuuuuuuu uaaucaucag aaauucucuc ucucucucuc ucuuuuucuc ucgcucucuu 1980

uuuuuuuuuu uuuuuacagg aaaugccuuu aaacaucguu ggaacuacca gagucaccuu 2040

aaaggagauc aauucucuag acugauaaaa auuucauggc cuccuuuaaa uguugccaaa 2100

uauaugaauu cuaggauuuu uccuuaggaa agguuuuucu cuuucaggga agaucuauua 2160

acuccccaug ggugcugaaa auaaacuuga uggugaaaaa cucuguauaa auuaauuuaa 2220

aaauuauuug guuucucuuu uuaauuauuc uggggcauag ucauuucuaa aagucacuag 2280

uagaaaguau aauuucaaga cagaauauuc uagacaugcu agcaguuuau auguauucau 2340

gaguaaugug auauauauug ggcgcuggug aggaaggaag gaggaaugag ugacuauaag 2400

gaugguuacc auagaaacuu ccuuuuuuac cuaauugaag agagacuacu acagagugcu 2460

aagcugcaug ugucaucuua cacuagagag aaaugguaag uuucuuguuu uauuuaaguu 2520

auguuuaagc aaggaaagga uuuguuauug aacaguauau uucaggaagg uuagaaagug 2580

gcgguuagga uauauuuuaa aucuaccuaa agcagcauau uuuaaaaauu uaaaaguauu 2640

gguauuaaau uaagaaauag aggacagaac uagacugaua gcagugaccu agaacaauuu 2700

gagauuagga aaguugugac caugaauuua aggauuuaug uggauacaaa uucuccuuua 2760

aaguguuucu ucccuuaaua uuuaucugac gguaauuuuu gagcagugaa uuacuuuaua 2820

uaucuuaaua guuuauuugg gaccaaacac uuaaacaaaa aguucuuuaa gucauauaag 2880

ccuuuucagg aagcuugucu cauauucacu cccgagacau ucaccugcca aguggccuga 2940

ggaucaaucc aguccuaggu uuauuuugca gacuuacauu cucccaaguu auucagccuc 3000

auaugacucc acggucggcu uuaccaaaac aguucagagu gcacuuuggc acacaauugg 3060

gaacagaaca aucuaaugug ugguuuggua uuccaagugg ggucuuuuuc agaaucucug 3120

cacuagugug agaugcaaac auguuuccuc aucuuucugg cuuauccagu auguagcuau 3180

uugugacaua auaaauauau acauauauga aaaua 3215

<210> 5
<211> 2895
<212> RNA
<213> Homo sapiens

<400> 5
agugugugu aaaggaauuc auuagccaug gauguauuca ugaaaggacu uucaaaggcc 60

aaggagggag uuguggcugc ugcugagaaa accaaacagg guguggcaga agcagcagga 120

aagacaaaag agggguguucu cuauguaggc uccaaaacca aggagggagu ggugcauggu 180
guggcaacag uggcugagaa gaccaaagag caagugacaa auguuggagg agcaguggug 240
acggguguga cagcaguagc ccagaagaca guggagggag cagggagcau ugcagcagcc 300
acuggcuuug ucaaaaagga ccaguugggc aaggaagggu aucaagacua cgaaccugaa 360
gccuaagaaa uaucuuugcu cccaguuucu ugagaucugc ugacagaugu uccauccugu 420
acaagugcuc aguuccaaug ugcccaguca ugacauuucu caaaguuuuu acaguguauc 480
ucgaagucuu ccaucagcag ugauugaagu aucuguaccu gcccccacuc agcauuucgg 540
ugcuucccuu ucacugaagu gaauacaugg uagcaggguc uuugugugcu guggauuuug 600
uggcuucaau cuacgauguu aaaacaaauu aaaaacaccu aagugacuac cacuuauuuc 660
uaaauccuca cuauuuuuuu guugcuguug uucagaaguu guuagugauu ugcuaucaua 720
uauuauaaga uuuuuaggug ucuuuuaaug auacugucua agaauaauga cguauuguga 780
aauuuguuaa uauauauaau acuuaaaaau augugagcau gaaacuaugc accuauaaau 840
acuaaauaug aaauuuuacc auuuugcgau guguuuuauu cacuuguguu uguauauaaa 900
uggugagaau uaaaauaaaa cguuaucuca uugcaaaaau auuuuauuuu uaucccaucu 960
cacuuuaaua auaaaaauca ugcuuauaag caacaugaau uaagaacuga cacaaaggac 1020
aaaaauauaa aguuauuaau agccauuuga agaaggagga auuuuagaag agguagagaa 1080
aauggaacau uaacccuaca cucggaauuc ccugaagcaa cacugccaga aguguguuuu 1140
gguaugcacu gguuccuuaa guggcuguga uuaauuauug aaaguggggu guugaagacc 1200
ccaacuacua uuguagagug gucuauuucu cccuucaauc cugucaaugu uugcuuuacg 1260
uauuuugggg aacuguuguu ugauguguau guguuuauaa uuguuauaca uuuuuaauug 1320
agccuuuuau uaacauauau uguuauuuuu gucucgaaau aauuuuuuag uuaaaaucua 1380
uuuugucuga uauuggugug aaugcuguac cuuucugaca auaaauaaua uucgaccaug 1440
aauaaaaaaa aaaaaaaagu ggguucccgg gaacuaagca guguagaaga ugauuuugac 1500
uacacccucc uuagagagcc auaagacaca uuagcacaua uuagcacauu caaggcucug 1560
agagaaugug guuaacuuug uuuaacucag cauuccucac uuuuuuuuuu uaaucaucag 1620
aaauucucuc ucucucucuc ucuuuuucuc ucgcucucuu uuuuuuuuuu uuuuuacagg 1680
aaaugccuuu aaacaucguu ggaacuacca gagucaccuu aaaggagauc aauucucuag 1740
acugauaaaa auuucauggc cucccuuaaa uguugccaaa uauaugaauu cuaggauuuu 1800
uccuuaggaa agguuuuucu cuuucaggga agaucuauua acuccccaug ggugcugaaa 1860
auaaacuuga uggugaaaaa cucuguauaa auuaauuuaa aaauuauuug guuucucuuu 1920
uuaauuauuc uggggcauag ucauuucuaa aagucacuag uagaaaguau aauuucaaga 1980

cagaauauuc uagacaugcu agcaguuuau auguauucau gaguaaugug auauauauug 2040

ggcgcuggug aggaaggaag gaggaaugag ugacuauaag gaugguuacc auagaaacuu 2100

ccuuuuuuac cuaauugaag agagacuacu acagagugcu aagcugcaug ugucaucuua 2160

cacuagagag aaaugguaag uuucuuguuu uauuuaaguu auguuuaagc aaggaaagga 2220

uuuguuauug aacaguauau uucaggaagg uuagaaagug gcgguuagga uauauuuuaa 2280

aucuaccuaa agcagcauau uuuaaaaauu uaaaaguauu gguauuaaau uaagaaauag 2340

aggacagaac uagacugaua gcagugaccu agaacaauuu gagauuagga aaguugugac 2400

caugaauuua aggauuuaug uggauacaaa uucuccuuua aaguguuucu ucccuuaaua 2460

uuuaucugac gguaauuuuu gagcagugaa uuacuuuaua uaucuuaaua guuuauuugg 2520

gaccaaacac uuaaacaaaa aguucuuuaa gucauauaag ccuuuucagg aagcuugucu 2580

cauauucacu cccgagacau ucaccugcca aguggccuga ggaucaaucc aguccuaggu 2640

uuauuuugca gacuuacauu cucccaaguu auucagccuc auaugacucc acggucggcu 2700

uuaccaaaac aguucagagu gcacuuuggc acacaauugg gaacagaaca aucuaaugug 2760

ugguuuggua uuccaagugg ggucuuuuuc agaaucucug cacuagugug agaugcaaac 2820

auguuuccuc aucuuucugg cuuauccagu auguagcuau uugugacaua auaaauauau 2880

acauauauga aaaua 2895

<210> 6
<211> 21
<212> DNA
<213> Artificial Sequence

<220>
<223> Specific primer for transcript SNCAtv2

<400> 6
agtcggagtt gtggagaagc a 21

<210> 7
<211> 20
<212> DNA
<213> Artificial Sequence

<220>
<223> Specific primer for transcript SNCAtv3

<400> 7
atccaggaac agctgtcttc 20

<210> 8
<211> 19
<212> DNA
<213> Artificial Sequence

<220>

<223> Generic primer for SNCA transcripts

<400> 8
accactgctc ctccaacat 19

<210> 9
<211> 1980
<212> DNA
<213> Homo sapiens

<400> 9
ggaggcagag gttgcggtga gccaagatgg cgccattgca ctccagcctg ggtggcaaga 60

gtgaactctg tctcaaaaaa aaataaataa ataaaataaa acattgtatt ataataaggg 120

taaaatctat gtggaatgat aattaaaaag ttatccttat gcattaataa atacagtatg 180

acctttgtat ttgcgggtta cacatccatg gatttaacca accacgagtc aaaaacattt 240

gataaataaa tacatctata ctaaacatat acagaccttt ttttcttatc attattcctt 300

aaacaatata atataataaa catttacaca gcactttcat tatattaggt atgaattatc 360

tagggatgtg catggcttat gtgaaaatgt tatgctattt tacattaggg acttgagtat 420

gagaggattt tggtatccct gagaggtcct agaaccaact ccccacaaat actaaggatg 480

actgtagtca tatataattt tcatcagaaa taattatctg ttatataact atatatgtaa 540

atgagaccat taaatgaaat ttgtgttatt ttaaagtggg tcaagaaaag agcataatat 600

ctacatatcc tcttgaaaac taaagaaaat atatgtaatg taattataca taatacaata 660

tataaactca ttttccattt tatatgttat acgttatata aattatatat acattatttg 720

cattttcaca taatctgtaa agattttatt aaaatctctt ttcaggcaaa gcgagctaat 780

aacaaataat aaagaataaa taaagaaaat aatgctgtac tgtgtagtta gagaaaatgg 840

cttttgtatt cgaaattatt tttcagttaa tgaaacagat aaaaattttg attaatacaa 900

cttattccat attttacagg aaatattgat gaagcagaat gggagtggaa agcaggattc 960

catcgctgga acaattacat gatggactgg aaaaatcaat ttaacgatta cactagcaag 1020

aaagaaagtt gtgtgggtct ctaattaata gatttaccct ttatagaaca tattttcctt 1080

tagatcaagg caaaaatatc aggagctttt ttacacacct actaaaaaag ttattatgta 1140

gctgaaacaa aaatgccaga aggataatat tgattcctca catctttaac ttagtatttt 1200

acctagcatt tcaaaaccca aatggctaga acgtgtttaa ttaaatttca caatataaag 1260

ttctacagtt aattatgtgc atattaaaac aatggcctgg ttcaatttct ttctttcctt 1320

aataaattta agtttttccc cccaaaatta tcagtgctct gcttttagtc acgtgtattt 1380

tcattaccac tcgtaaaaag gtatcttttt taaatgaatt aaatattgaa acactgtaca 1440

ccatagttta caatattatg tttcctaatt aaaataagaa ttgaatgtca atatgagata 1500

ttaaaataag cacagaaaat cattggtctc tttgtttttt atataaaggc aagaaagaat 1560

cttaaaatga gaggctcagc cactttaaaa caccttataa tagaaacaga atgctgctac 1620

caagaaacaa aatataaaat aatgtaatta tttatgtaat gccttcaaat tatattcttc 1680

atttgactct tgctctccct cacctcctct atcctcaaat caccacaaac atgtacatat 1740

gtgaattacc tttccgtaat tcaaaaattt ttaaaaagtg ggtgcataga agtctaaagt 1800

taggttgtgt accatctggg acagggcaac ccttgcctgg acccagaaat attaacatat 1860

taataggaaa attgttcaat tccagaagag aaatggttgt actttctgta gacaaactgt 1920

cttatttttg caattaaaaa aattacagag ctgcatttat tagcatgctt ggcaagggag 1980

<210> 10
<211> 5040
<212> DNA
<213> Homo sapiens

<400> 10
tccattttta tgttactatt tccaggaaaa atttaaaata gccttcatat acaataaaag 60

caagttagat aaattatatt gtacttatac aaataattaa tacataggca tataaatgaa 120

gttaaaaaaa tattcgatag cattattttg gaaaaactta agataggtta tatgaaagta 180

ggctaccaaa gactatgtat gatgtatttc caaattagct tgaagcaaaa ggtacagttg 240

tgcatgagaa taagcaatta aaaaaaagaa aaagcatata tatatatatg gtagcacaaa 300

aatgtcctta gtatccaagc tccaggactt tgcctcttag tgtgtagttt gtgaactccc 360

tctatcagct tcacagagga cagtagtata acgctgtcct caagtctact gaaaccaaag 420

cccagggatg tggcctagaa atctggatgt ttaaatagca ctttgatgaa tttgataatg 480

tgttactttg caatttttct gagagtgaga ctccaaagat agtcaagggt ttgggaagag 540

taaatttaag ccaattcagt aaattaggtg ataaacaaag aagcttgctg tttttctctc 600

tttttttatg ttttaaaaca atgagtttct cctttttttgc tcaagccctg aacaatttag 660

ttcagaattt gttttcttac tttgattctg ctggctttgc caattccatg aaaatccact 720

tggcaccaca tttccttaga agctcttgct ttttcttgat tttctttctg ttcttagagt 780

ctaatattct agtgtctcac caaattgttg ttgtctcttt ccaaaactcc tttaaccaaa 840

ctatctgtat cttcatgtaa aaggcaccac agacctccca tcactcactc tcccttaacc 900

ccaaaatatc tatgtgttat cccttacaga tgtcacttcc tgcaaggcct tccatattag 960

ttgctacaac atcactaaag aattttatta cctgtggctc tggacctact tctaatatct 1020

ctggagatat ccaccttcaa acacctgcca catccagcta tgagggtcta gtacctgggc 1080

tccagagaca tgttcaggtt agttctgatt ataagaacat ttctcttttt tgccagaatt 1140

gttggaaagt tatctctatt caacagtctg tgtaggattt cagaagattg aatatacata 1200

taaatatata tacatacaca catgtatata tacacacaca catatatgta tatgtacttt 1260

gtccatgtcg tatttggtta ttccaattct gggaaaataa attaagaaaa taattaaaag 1320

atgcactcaa atatcgatat acaaaatgcc tattgtaata atacataaaa aaagtcagaa 1380

gctaacctcg gtatctgtat gatttggagc aagttactta aactctttgt ggctatattt 1440

tctcaaatgt aaaataaggt taataatagt gcttatttta aaggtagtca tgagttttaa 1500

atgaattcat gtattcaaag tggatagaat agtgctttca aataatattt attacctaaa 1560

attattatta tcatcatata agtaacaaga gatacaatga accataatat gcccatatga 1620

taatatatca cacagtaatc aaaatatttt aataggaaat taatatcata agataaaaag 1680

caagaagaat gatgataagc catttgacta ccactatatt tatctatgta actataacca 1740

catacacata ttttaaaaaa ttgattggaa tatacaaaat ggtcttttca ttctctgtga 1800

tatgtaaaat gtagaaatat acttttctgg gctggatgca gtagctcaca cctataatcc 1860

cagcactttg ggaggccgag gttggtggag cacctgaggt caggaattcg agaccagcct 1920

ggccaacata gtgaaatcct gtgtctacta aaaatacaaa aattagccag atttggtggt 1980

gggtgcctgt aatcccagct actcaggagg ctgaggcagg aaaattgctt gaacccagga 2040

ggcagaggtt gcagtgagcc gagatcctgc cactgcactc cagcctgggc gacagagtga 2100

gactccatct caggaaaaaa aaaaaaaaat ctatctatct atctatctat ctatctatct 2160

atctatctat ctatctatct atacttatat atacttttct ttgttatcca tattcatttc 2220

agagaatgca gtgctattgt aaaaaaaatg aataaaatta atataagttt agagcacttt 2280

tttggcatca gtatgcctaa ggaaaactaa tgtataatac aggattaatg tttttcacac 2340

catctcaaat cttactgaat gctgctatct agaaaacagt ggtaactgag ttacaatggg 2400

accttaaaag caaaaaacca ctgtgactgg ctaactgaaa gtgatcatag agccaaaacc 2460

ataatgattg tcctgcccaa gcttcagcat atatggttgc aaaatgtgcg cagtgttaat 2520

gccgtgtcaa gtgtcttgtg gtatttcatc agtgtcgctt acaaggcata ctcaacattt 2580

aacagcagga tcattaacca tccatgctgt gatgtaatgg aatagtgctt ccctactaac 2640

cctgaaacaa gctcactgtg tgtgacctac atccttccgc tggatgagtg tgagaaaatg 2700

atgacaccac agaagcaaag cagctgctgt aagttgagcc acaaggaaga aactctcaag 2760

acaggaagaa taaatgcttt agaggtgcac taaaataatg aatgaactaa tgtggcatag 2820

ctgtgaaatc atgcgaggaa aagaactaca gggacagtaa cagaaggtgg ttctttgaag 2880

taaagacatt gagaagaaga aaaattgcca acttgtgtaa tcagttgatg ttgctagtta 2940

gccagtttaa acatgcgtta aagataaaga tctgcttgaa accaattatt ttattgtttg 3000

gcttcctgtt taaagatgaa aatgaaatat gtgtatatat gtgacatatt ttacctagca 3060

gtgagtagat gcaggaagca tgtaaaatgc tttgctgaat tcactaacgt cacaaaatat 3120

tccttaataa ttgccgcgtt tgataggctg accttatgct agcacaggaa acaatgcagt 3180
ggacttgggt tggagagaag aattttgaca tttggatttt taaaaatcat gtatattgaa 3240
cccatgaact ctcatgtgga acatcagatt tactatattt taaacagaaa tgccatcact 3300
ataaaaacca ttgcaaattt gtcaatatac ttccatttaa aaagcctttt cctgctttta 3360
ttgatttatt gattaatttt tttttgagac aggatctcac tctgtcaccc acgatgaagt 3420
gcagtggcac aatcgtggct tgcagcctcg acctcctagg ctcaggtgat actcttacct 3480
cagctacctc ccaagtaact gggaatgtag gcttgcacca ccatgtctga cccattttct 3540
tgcagaaatt gagttttgct atgttgtcca gacttgtctt gaactcccag actgaagcaa 3600
tccgcccact taggcctcct aaaatgttgg gattacagtc atgaaccact gtgctccacc 3660
tcctttttct ccttttcaga tagattatgt attctgtacc ttttgtgtaa ctaacttgtt 3720
attacataaa ctatattcta aatacattat aaattattgt gatcatgata tttatcattg 3780
aattaacact gcctaagtag caattttcac agaaaagata attttaaaag accaaaatgg 3840
ttattctctt ggaagcagtt gacataaaat aacagaataa tacagattaa aattctgttt 3900
tccaacagct actagtggta agaaatatag taagcttctg atttgtagaa accttagctt 3960
tctcatctga tgaggtggtt aataatatta actctcatat ttgctgaaat aatataaaat 4020
atttaatata aattgtcaat tataatgact ggcataaggt agaattacaa caaatgctaa 4080
ttatatttgc ttgtttgaaa atttaacact ctattaaaag gcaaaaaagt gctcacctgc 4140
aaatattaat acatgccttc tgtttagtcc atagaaagcc cacaagtaag agattcaaaa 4200
gtgaaagcta tcacttacag aagattaaga atatattgca ttgagaagat agggattaag 4260
gagtttatca aaggtttttt aaaaaattat tttcctgaac atcatttcaa gaaagataag 4320
cgccaaagac ataaatatat tctccagagc atgtgcactg caagttgaca aatgtgtctc 4380
ttttatagag tttggtgaag gttattcatt ttatttttcc tgtcttgacc agaaaattgt 4440
ggcttctcat catgtatgac tgcctgcagg gtcttctgaa taggaaatta ccaatgctga 4500
aatttgtatg acttaaataa gtgttgaagt gtgggggttg aaaggagata catatcagag 4560
acatccattc agttaataga actaagtttt tgatttgcat agcttatatc tagagcattg 4620
tgtttcttat tttctatatt actatcttct ctaactatat aattagcaca gattgaagct 4680
ataatggatt tcaataatag cactacttta gaatgatgta tacgtcttta aatcttaaga 4740
caagcatagg aaatatgtat attcttaaaa aaaggaaaat ttttttaaag tttagagtta 4800
aaagaaacat gcattttctg ttacatttca gattatttta gttaaagaat cttctatgct 4860
tttttcttc attcttagaa gactacctgc aattgtaaag catgaatcct gtaatacttc 4920
tgtaaatgaa tcactattgc atttatacca ttagcctctg aacagatttc aagttgctgc 4980
tgccaactct cgcgagcttt gtcagtaaca gttgattgtt acattcagta acactgaatg 5040